# EUROPEAN PATENT SPECIFICATION

(11) **EP 3 728 592 B1**
(45) Date of publication and mention of the grant of the patent: **29.05.2024**
(21) Application number: 18833230.8
(22) Date of filing: 21.12.2018
(51) Int. Cl.: C12N 15/11, C07F 9/165, C07H 19/10, C07H 19/20, C07H 21/00

(54) **OLIGONUCLEOTIDES COMPRISING A PHOSPHORODITHIOATE INTERNUCLEOSIDE LINKAGE**
OLIGONUKLEOTIDE MIT EINER PHOSPHORDITHIOAT-INTERNUKLEOSIDVERKNÜPFUNG
OLIGONUCLÉOTIDES COMPRENANT UNE LIAISON INTERNUCLÉOSIDIQUE PHOSPHORODITHIOATE

(30) Priority: 22.12.2017 WO PCT/CN2017/118040; 03.10.2018 EP 18198479
(43) Date of publication of application: 28.10.2020
(60) Divisional application: 22161952.1
(73) Proprietor: Roche Innovation Center Copenhagen A/S, 2970 Hørsholm (DK)
(72) Inventor: BLEICHER, Konrad, 4070 Basel (CH); HANSEN, Henrik, Frydenlund, 2970 Hørsholm (DK); KOCH, Troels, 2970 Hørsholm (DK); WORM, Jesper, 2970 Hørsholm (DK); SCHAEUBLIN, Adrian, 4070 Basel (CH); FUNDER, Erik, 2970 Hørsholm (DK); DUSCHMALÉ, Joerg, 4070 Basel (CH); JOENSON, Lars, 2970 Hørsholm (DK); LI, Meiling, 4070 Basel (CH); DUSCHMALÉ, Martina, Brigitte, 4070 Basel (CH); WU, Yong, Hubei, 430075 (CN); SHU, Xi, Hubei,430075 (CN)
(74) Representative: Pomeranc, Didier
(86) International application number: PCT/EP2018/086460
(87) International publication number: WO 2019/122279

(56) References cited:
- WO-A1-90/12022
- WO-A1-90/12022
- WO-A1-2014/076195
- WO-A1-2016/079183
- US-A- 5 218 088
- US-A- 5 218 088
- US-A- 5 684 148
- US-A- 5 684 148
- US-A1- 2015 322 431
- US-A1- 2015 322 431
- US-A1- 2017 319 614
- US-A1- 2017 319 614
- PIOTTO M E ET AL: "Synthesis, NMR and structure of oligonucleotide phosphorodithioates", TETRAHEDRON, ELSEVIER SIENCE PUBLISHERS, AMSTERDAM, NL, vol. 47, no. 14-15, 1991, pages 2449-2461, XP028086758, ISSN: 0040-4020, DOI: 10.1016/S0040-4020(01)81780-4 [retrieved on 1991-01-01]
- WIESLER W T ET AL: "SYNTHESIS OF PHOSPHORODITHIOATE DNA VIA SULFUR-LINKED BASE-LABILE PROTECTING GROUPS", THE JOURNAL OF ORGANIC CHEMISTRY, AMERICAN CHEMICAL SOCIETY, vol. 61, no. 13, 28 June 1996 (1996-06-28) , pages 4272-4281, XP002920045, ISSN: 0022-3263, DOI: 10.1021/JO960274Y
- XIANBIN YANG ET AL: "Gene Silencing Activity of siRNA Molecules Containing Phosphorodithioate Substitutions", ACS CHEMICAL BIOLOGY, vol. 7, no. 7, 18 April 2012 (2012-04-18), pages 1214-1220, XP055567178, ISSN: 1554-8929, DOI: 10.1021/cb300078e
- Xianbin Yang: "Solid-Phase Synthesis of Oligodeoxynucleotide Analogs Containing Phosphorodithioate Linkages : Synthesis of Oligodeoxynucleotide Analogs Containing PS2 Linkages" In: "Current Protocols in Nucleic Acid Chemistry", 1 September 2016 (2016-09-01), John Wiley & Sons, Inc., Hoboken, NJ, USA, XP055556956, ISBN: 978-0-471-14270-6 vol. 6614, pages 4.71.1-4.71.14, DOI: 10.1002/cpnc.13, the whole document
- SHERRY Y. WU ET AL: "2'-OMe-phosphorodithioate-modified siRNAs show increased loading into the RISC complex and enhanced anti-tumour activity", NATURE COMMUNICATIONS, vol. 5, no. 1, 12 March 2014 (2014-03-12), XP055566602, DOI: 10.1038/ncomms4459
- PIOTTO M E ET AL: "Synthesis, NMR and structure of oligonucleotide phosphorodithioates", TETRAHEDRON, ELSEVIER SIENCE PUBLISHERS, AMSTERDAM, NL, vol. 47, no. 14-15, 1991, pages 2449-2461, XP028086758, ISSN: 0040-4020, DOI: 10.1016/S0040-4020(01)81780-4 [retrieved on 1991-01-01]
- WIESLER W T ET AL: "SYNTHESIS OF PHOSPHORODITHIOATE DNA VIA SULFUR-LINKED BASE-LABILE PROTECTING GROUPS", THE JOURNAL OF ORGANIC CHEMISTRY, AMERICAN CHEMICAL SOCIETY, vol. 61, no. 13, 28 June 1996 (1996-06-28) , pages 4272-4281, XP002920045, ISSN: 0022-3263, DOI: 10.1021/JO960274Y
- XIANBIN YANG ET AL: "Gene Silencing Activity of siRNA Molecules Containing Phosphorodithioate Substitutions", ACS CHEMICAL BIOLOGY, vol. 7, no. 7, 18 April 2012 (2012-04-18), pages 1214-1220, XP055567178, ISSN: 1554-8929, DOI: 10.1021/cb300078e

## Description

### BACKGROUND

The use of synthetic oligonucleotides as therapeutic agents has witnessed remarkable progress over recent decades leading to the development of molecules acting by diverse mechanisms including RNase H activating gapmers, splice switching oligonucleotides, microRNA inhibitors, siRNA or aptamers (S. T. Crooke, Antisense drug technology: principles, strategies, and applications, 2nd ed. ed., Boca Raton, FL: CRC Press, 2008). However, oligonucleotides are inherently unstable towards nucleolytic degradation in biological systems. Furthermore, they show a highly unfavorable pharmacokinetic behavior. In order to improve on these drawbacks a wide variety of chemical modifications have been investigated in recent decades. Arguably one of the most successful modification is the introduction of phosphorothioate linkages, where one of the non-bridging phosphate oxygen atoms is replaced with a sulfur atom (F. Eckstein, Antisense and Nucleic Acid Drug Development 2009, 10, 117-121.). Such phosphorothioate oligodeoxynucleotides show an increased protein binding as well as a distinctly higher stability to nucleolytic degradation and thus a substantially higher half-live in plasma, tissues and cells than their unmodified phosphodiester analogues. These crucial features have allowed for the development of the first generation of oligonucleotide therapeutics as well as opened the door for their further improvement through later generation modifications such as Locked Nucleic Acids (LNAs). Replacement of a phosphodiester linkage with a phosphorothioate, however, creates a chiral center at the phosphorous atom. As a consequence, all approved phosphorothioate oligonucleotide therapeutics are used as mixtures of a huge amount of diastereoisomeric compounds, which all potentially have different (and possibly opposing) physiochemical and pharmacological properties.

While the stereospecific synthesis of single stereochemically defined phosphorothioate oligonucleotides is now possible (N. Oka, M. Yamamoto, T. Sato, T. Wada, J. Am. Chem. Soc. 2008, 130, 16031-16037) it remains a challenge to identify the stereoisomer with optimal properties within the huge number of possible diastereoisomers. In this context, the reduction of the diastereoisomeric complexity by the use of non-chiral thiophosphate linkages is of great interest. For example, the symmetrical non-bridging dithioate modification (see e.g. W. T. Wiesler, M. H. Caruthers, J. Org. Chem. 1996, 61, 4272-4281), where both non-bridging oxygen atoms within the phosphate linkage are replaced by sulfur has been applied to immunostimulatory oligonucleotides (A. M. Krieg, S. Matson, E. Fisher, Antisense Nucleic Acid Drug Dev. 1996, 6, 133-139) , siRNA (e.g. X. Yang, M. Sierant, M. Janicka, L. Peczek, C. Martinez, T. Hassell, N. Li, X. Li, T. Wang, B. Nawrot, ACS Chem. Biol. 2012, 7, 1214-1220) and aptamers (e.g. X. Yang, S. Fennewald, B. A. Luxon, J. Aronson, N. K. Herzog, D. G. Gorenstein, Bioorg. Med. Chem. Lett. 1999, 9, 3357-3362). Interestingly, attempts to make use of this non-chiral modification in the context of antisense oligonucleotides have met with limited success to date (see e.g. M. K. Ghosh, K. Ghosh, O. Dahl, J. S. Cohen, Nucleic Acids Res. 1993, 21, 5761-5766. and J. P. Vaughn, J. Stekler, S. Demirdji, J. K. Mills, M. H. Caruthers, J. D. Iglehart, J. R. Marks, Nucleic Acids Res. 1996, 24, 4558-4564).

To our surprise we have now found that non-bridging phosphorodithioates can be introduced into oligonucleotide, in particular to oligonucleotide gapmers or mixmers in general and LNA-DNA-LNA gapmers or LNA/DNA mixmers in particular. The modification is well tolerated and the resulting molecules show great potential for therapeutic applications, while every non-bridging phosphorodithioate modification reduces the size of the overall library of possible diastereoisomers by 50%. When the modification is placed in the LNA flanks of gapmers, the resulting oligonucleotides turn out to be generally more potent than the corresponding all-phosphorothioate parent. In general, the modification is additionally well tolerated within the gap region and even more surprisingly can lead to an improved potency as well, when positioned appropriately.

We have thus surprisingly found that the invention provides oligonucleotides with improved physiochemical and pharmacological properties, including, for example, improved potency. In some aspects, the oligonucleotide of the invention retains the activity or effiacy, and may be as potent or is more potent, than the identical compound where the phosphodithioate linkages of formula (IA or IBIB) are replaced with the conventional stereorandom phosphorothioate linkages (phosphorothioate reference compound). Every introduction of the non-bridging phosphorodithioate modification removes one of the chiral centers at phosphorous and thereby reduces the diastereoisomeric complexity of the compound by 50%. Additionally, whenever a dithioate modification is introduced, the oligonucleotide appears to be taken up dramatically better into cells, in particular into hepatocytes, muscle cells, heart cells for example.

The introduction of non-bridging dithioate modifications into the LNA flanks of gapmers appears to be particularly beneficial, leading to molecules demonstrating a higher target reduction and a substantially better uptake behavior, higher stability and good safetly profile.

The chemical synthesis of non-bridging phosphorodithioate linkages in oligonucleotides is best achieved by solid phase oligonucleotide synthesis techniques using appropriate thiophosphoramidite building blocks. The successful application of such thiophosphoramidites has been described for regular DNA (X. Yang, Curr Protoc Nucleic Acid Chem 2016, 66, 4.71.71-74.71.14.) as well as RNA (X. Yang, Curr Protoc Nucleic Acid Chem 2017, 70, 4.77.71-74.77.13.) and the required building blocks are available from commercial sources. Interestingly, the more challenging synthesis of the corresponding LNA thiophosphoramidites has not been reported. Within this application, we also report the successful synthesis of all four LNA thiophosphoramidites and their incorporation into oligonucleotides.

US 2017/319614 describes antisense-oligonucleotides having at least two LNA nucleotides and their use as inhibitors of TGF-R signalling.

### STATEMENT OF THE INVENTION

The invention relates to an oligonucleotide comprising at least one phosphorodithioate internucleoside linkage of formula (I) 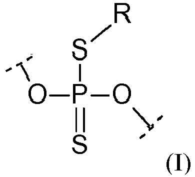 wherein one of the two oxygen atoms is linked to the 3'carbon atom of an adjacent nucleoside (A¹) and the other one is linked to the 5'carbon atom of another adjacent nucleoside (A²), wherein at least one of the two nucleosides (A¹) and (A²) is a LNA nucleoside and wherein R is hydrogen or a phosphate protecting group; wherein the oligonucleotide comprises between 1 and 5 internucleoside linkages of formula (I) and wherein the further internucleoside linkages are all phosphorothioate internucleoside linkages.

A gapmer oligonucleotide may comprise a phosphorodithioate internucleoside linkage of formula (I). Also described here is a process for the manufacture of an oligonucleotide according to the invention and to a LNA nucleoside monomer useful in particular in the manufacture of on oligonucleotide according to the invention.

In an aspect, the invention provides an oligonucleotide comprising at least one phosphorodithioate internucleoside linkage of formula (IA) or (IB) 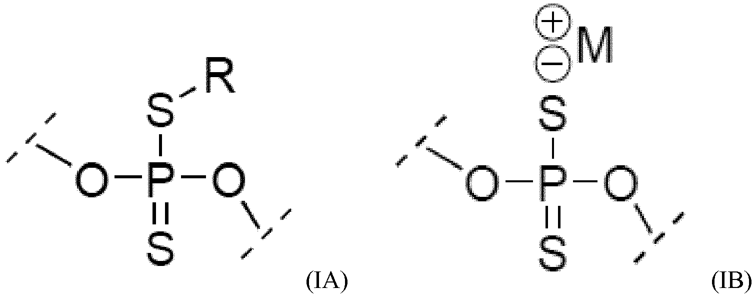 wherein one of the two oxygen atoms is linked to the 3'carbon atom of an adjacent nucleoside (A¹) and the other one is linked to the 5'carbon atom of another adjacent nucleoside (A²), wherein in (IA) R is hydrogen or a phosphate protecting group, and in (IB) M+ is a cation, such as a metal cation, such as an alkali metal cation, such as a Na+ or K+ cation; or M+ is an ammonium cation, wherein at least one of the two nucleosides (A¹) and (A²) is a LNA nucleoside, and wherein the oligonucleotide comprises between 1 and 5 internucleoside linkages of formula (IA) or (IB) and wherein the further internucleoside linkages are all phosphorothioate internucleoside linkages.

Alternatively stated M is a metal, such as an alkali metal, such as Na or K; or M is NH₄.

The oligonucleotide may be an antisense oligonucleotide comprising a phosphorodithioate internucleoside linkage, of formula IA or IB as described herein. The oligonucleotide of the invention is preferably a single stranded antisense oligonucleotide, which comprises one or more 2'sugar modificied nucleosides, such as one or more LNA nucleosides or one or more 2' MOE nucleosides. The antisense oligonucleotide of the invention is capable of modulating the expression of a target nucleic acid, such as a target pre-mRNA, or target microRNA in a cell which is expressing the target RNA - in vivo or in vitro. In the single stranded antisense oligonucleotide further internucleoside linkages are phosphorothioate internucleoside linkages. The single stranded antisense oligonucleotide may, for example may be in the form of a gapmer oligonucleotide, a mixmer oligonucleotide or a totalmer oligonucleotide. The single stranded antisense oligonucleotide mixmer may be for use in modulating a splicing event in a target pre-mRNA. The single stranded antisense oligonucleotide mixmer may be for use in inhibiting the expression of a target microRNA.

The invention further refers to the use of the oligonucleotide of the invention, such as the single stranded antisense oligonucleotide as a therapeutic.

The oligonucleotide may be a mixmer oligonucleotide comprising a phosphorodithioate internucleoside linkage of formula (IA or IB).

The oligonucleotide may be a totalmer oligonucleotide comprising a phosphorodithioate internucleoside linkage of formula (IA or IB).

Also described herein is a process for the manufacture of an oligonucleotide according to the invention and to a LNA nucleoside monomer useful in particular in the manufacture of on oligonucleotide according to the invention.

Also described herein is a process for the manufacture of an oligonucleotide according to the invention and to a MOE nucleoside monomer useful in particular in the manufacture of on oligonucleotide according to the invention.

Novel MOE and LNA monomers described herein may be used in the manufacture of on oligonucleotide according to the invention.

During oligonucleotide synthesis, the use of a protective R group is often used. After oligonucleotide synthesis, the protecting group is typically exchanged for either a hydrogen atom or cation like an alkali metal or an ammonium cation, such as when the oligonucleotide is in the form of a salt. The salt typically contains a cation, such as a metal cation, e.g. sodium or potassium cation or an ammonium cation. With regards antisense oligonucleotides, preferably R is hydrogen, or the the antisense oligonucleotide is in the form of a salt (as shown in IB).

The phosphorodithioate internucleoside linkage of formula (IB) may, for example, be selected from the group consisting of: 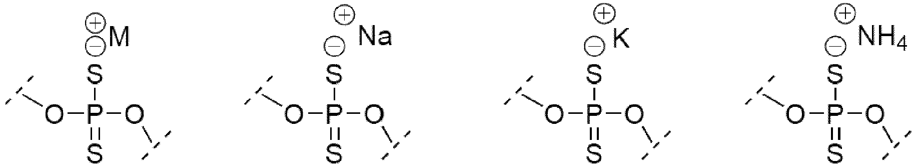 wherein M+ is a is a cation, such as a metal cation, such as an alkali metal cation, such as a Na+ or K+ cation; or M+ is an ammonium cation. The oligonucleotide of the invention may therefore be in the form of an oligonucleotide salt, an alkali metal salt, such as a sodium salt, a potassium salt or an ammonium salt.

Alternatively represented, the oligonucleotide of the invention may comprise a phosphorodithioate internucleoside linkage of formula IA' or IB'

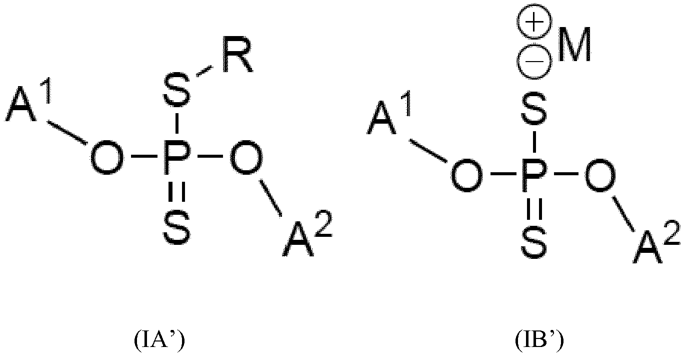

An oligonucleotide of the invention may be a gapmer oligonucleotide comprising a phosphorodithioate internucleoside linkage of formula (I), for formula IA or IB, or formula IA' or formula IB'.

An oligonucleotide of the invention may be a mixmer oligonucleotide comprising a phosphorodithioate internucleoside linkage of formula (I), for formula IA or IB, or formula IA' or formula IB'.

An oligonucleotide of the invention may be a totalmer oligonucleotide comprising a phosphorodithioate internucleoside linkage of formula (I), for formula IA or IB, or formula IA' or formula IB'.

In the oligonucleotide of the invention at least one of the two nucleosides (A¹) and (A²) is a LNA nucleoside.

In preferred embodiments of the oligonucleotide of the invention one of the two nucleosides (A¹) and (A²) is a 2'-O-MOE nucleoside.

In preferred embodiments of the oligonucleotide of the invention, the oligonucleotide is a single stranded antisense oligonucleotide, at least one of the two nucleosides (A¹) and (A²) is a LNA nucleoside.

In preferred embodiments of the oligonucleotide of the invention the oligonucleotide is a single stranded antisense oligonucleotide, and one of the two nucleosides (A¹) and (A²) is a 2'-O-MOE nucleoside.

The oligonucleotide may be an antisense oligonucleotide, for inhibition of a target RNA in a cell, wherein the antisense gapmer oligonucleotide comprises at least one phosphorodithioate internucleoside linkage of formula (IA) or (IB) 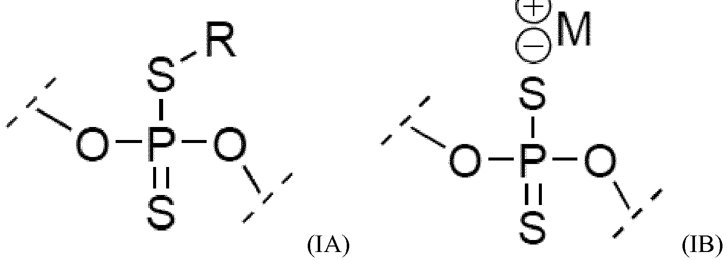 wherein in (IA) R is hydrogen or a phosphate protecting group, and in (IB) M+ is a cation, such as a metal cation, such as an alkali metal cation, such as a Na+ or K+ cation; or M+ is an ammonium cation, wherein the antisense oligonucleotide is or comprises an antisense gapmer oligonucleotide (referred to herein as a gapmer or a gapmer ligonucleotide),

The antisense oligonucleotide of the invention may therefore comprise or consist of a gapmer.

The oligonucleotide of the invention may be an antisense oligonucleotide comprising at least one phosphorodithioate internucleoside linkage formula IA or IB 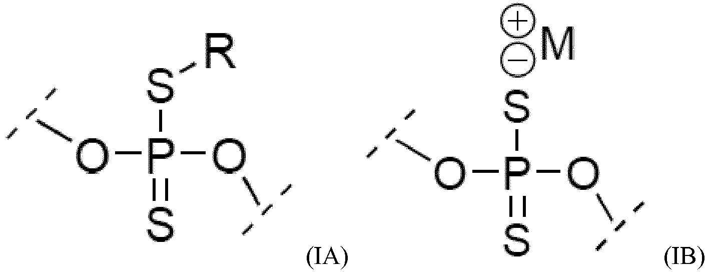 wherein one of the two oxygen atoms is linked to the 3'carbon atom of an adjacent nucleoside (A¹) and the other one is linked to the 5'carbon atom of another adjacent nucleoside (A²), wherein at least one of the two nucleosides (A¹) and (A²) is a LNA nucleoside and and wherein in (IA) R is hydrogen or a phosphate protecting group, and in (IB) M+ is a cation, such as a metal cation, such as an alkali metal cation, such as a Na+ or K+ cation; or M+ is an ammonium cation, wherein A² is the 3' terminal nucleoside of the oligonucleotide; wherein the oligonucleotide comprises between 1 and 5 internucleoside linkages of formula (IA) or (IB) and wherein the further internucleoside linkages are all phosphorothioate internucleoside linkages.

The oligonucleotide of the invention may be an antisense oligonucleotide comprising at least one phosphorodithioate internucleoside linkage of formula (IA or IB) 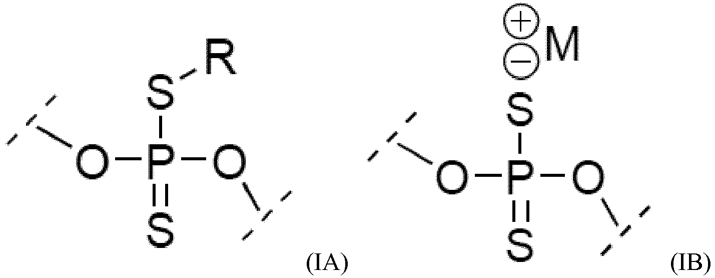 wherein one of the two oxygen atoms is linked to the 3'carbon atom of an adjacent nucleoside (A¹) and the other one is linked to the 5'carbon atom of another adjacent nucleoside (A²), wherein at least one of the two nucleosides (A¹) and (A²) is a LNA nucleoside and and wherein in (IA) R is hydrogen or a phosphate protecting group, and in (IB) M+ is a cation, such as a metal cation, such as an alkali metal cation, such as a Na+ or K+ cation; or M+ is an ammonium cation, wherein A¹ is the 5' terminal nucleoside of the oligonucleotide; wherein the oligonucleotide comprises between 1 and 5 internucleoside linkages of formula (IA) or (IB) and wherein the further internucleoside linkages are all phosphorothioate internucleoside linkages.

The oligonucleotide of the disclosure may be an antisense oligonucleotide comprising at least one phosphorodithioate internucleoside linkage of formula (IA or IB) 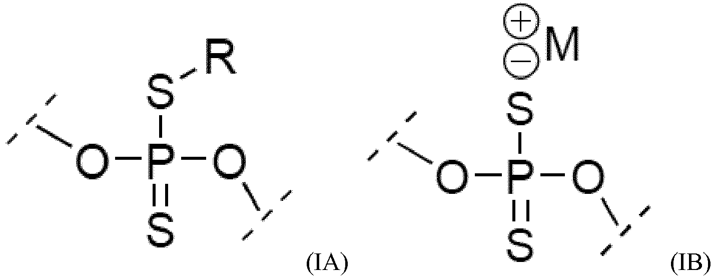 wherein one of the two oxygen atoms is linked to the 3'carbon atom of an adjacent nucleoside (A¹) and the other one is linked to the 5'carbon atom of another adjacent nucleoside (A²), wherein one of the two nucleosides (A¹) and (A²) is a 2-O-MOE nucleoside and wherein in (IA) R is hydrogen or a phosphate protecting group, and in (IB) M+ is a cation, such as a metal cation, such as an alkali metal cation, such as a Na+ or K+ cation; or M+ is an ammonium cation, wherein A² is the 3' terminal nucleoside of the oligonucleotide.

The oligonucleotide of the disclosure may be an antisense oligonucleotide comprising at least one phosphorodithioate internucleoside linkage of formula (IA or IB) 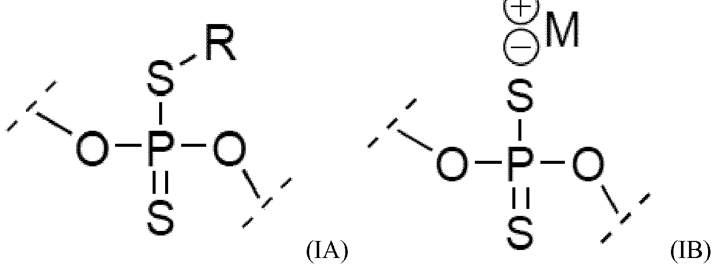 wherein one of the two oxygen atoms is linked to the 3'carbon atom of an adjacent nucleoside (A¹) and the other one is linked to the 5'carbon atom of another adjacent nucleoside (A²), wherein one of the two nucleosides (A¹) and (A²) is a 2-O-MOE nucleoside and wherein in (IA) R is hydrogen or a phosphate protecting group, and in (IB) M+ is a cation, such as a metal cation, such as an alkali metal cation, such as a Na+ or K+ cation; or M+ is an ammonium cation, wherein A¹ is the 5' terminal nucleoside of the oligonucleotide.

The oligonucleotide of the disclosure may be an antisense oligonucleotide comprising at least one phosphorodithioate internucleoside linkage of formula (IA or IB) 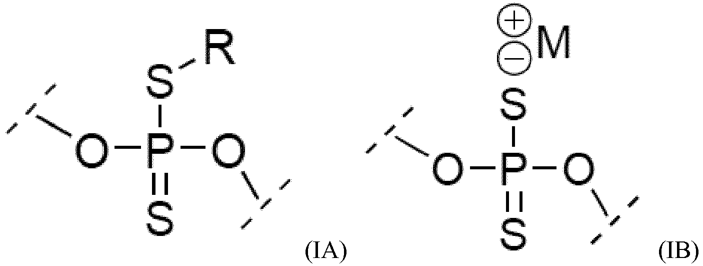 wherein one of the two oxygen atoms is linked to the 3'carbon atom of an adjacent nucleoside (A¹) and the other one is linked to the 5'carbon atom of another adjacent nucleoside (A²), wherein at least one of the two nucleosides (A¹) and (A²) is a 2' sugar modified nucleoside and and wherein in (IA) R is hydrogen or a phosphate protecting group, and in (IB) M+ is a cation, such as a metal cation, such as an alkali metal cation, such as a Na+ or K+ cation; or M+ is an ammonium cation, and wherein A² is the 3' terminal nucleoside of the oligonucleotide.

The oligonucleotide of the disclosure may be an antisense oligonucleotide comprising at least one phosphorodithioate internucleoside linkage of formula (IA or IB) 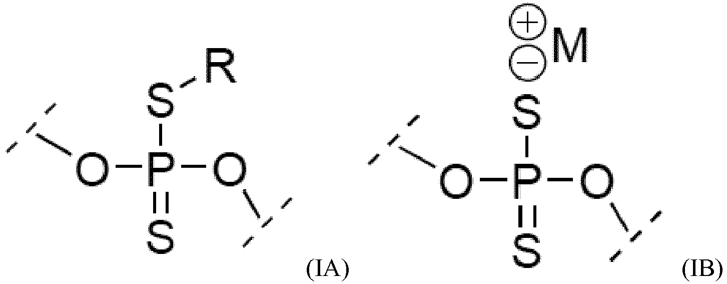 wherein one of the two oxygen atoms is linked to the 3'carbon atom of an adjacent nucleoside (A¹) and the other one is linked to the 5'carbon atom of another adjacent nucleoside (A²), wherein at least one of the two nucleosides (A¹) and (A²) is a 2' sugar modified nucleoside and wherein in (IA) R is hydrogen or a phosphate protecting group, and in (IB) M+ is a cation, such as a metal cation, such as an alkali metal cation, such as a Na+ or K+ cation; or M+ is an ammonium cation, and wherein A¹ is the 5' terminal nucleoside of the oligonucleotide.

A̅ 2' sugar modidied nucleoside may be independently selected from the group consisting of 2' sugar modified nucleoside selected from the group consisting of 2'-alkoxy-RNA, 2'-alkoxyalkoxy-RNA, 2'-amino-DNA, 2'-fluoro-RNA, 2'-fluoro-ANA and an LNA nucleoside.

The oligonucleotide of the disclosure may be a single stranded antisense oligonucleotide comprising at least one phosphorodithioate internucleoside linkage of formula (IA) or (IB) 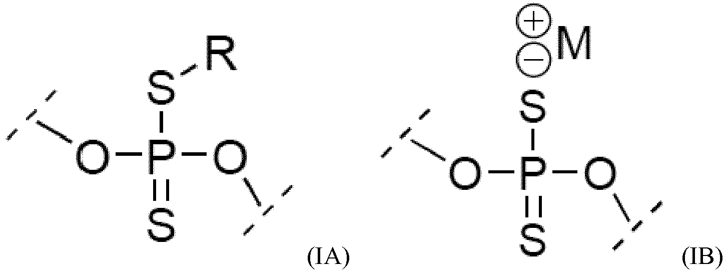 wherein one of the two oxygen atoms is linked to the 3'carbon atom of an adjacent nucleoside (A¹) and the other one is linked to the 5'carbon atom of another adjacent nucleoside (A²), and wherein in (IA) R is hydrogen or a phosphate protecting group, and in (IB) M+ is a cation, such as a metal cation, such as an alkali metal cation, such as a Na+ or K+ cation; or M+ is an ammonium cation, and wherein the single stranded oligonucleotide further comprises at least one stereo defined phosphorothioate internucleoside linkage, (Sp, S) or (Rp, R) 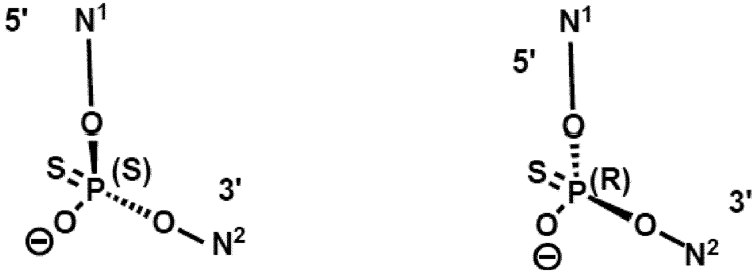 wherein N¹ and N² are nucleosides.

The oligonucleotide of the disclosure may be a single stranded antisense oligonucleotide, for modulation of a RNA target in a cell, wherein the antisense oligonucleotide comprises or consists of a contiguous nucleotide sequence of 10 - 30 nucleotides in length, wherein the contiguous nucleotide sequence comprises one or more 2'sugar modified nucleosides, and wherein at least one of the internucleoside linkages present between the nucleosides of the contiguous nucleotide sequence is a phosphorodithioate linkage of formula IA or IB 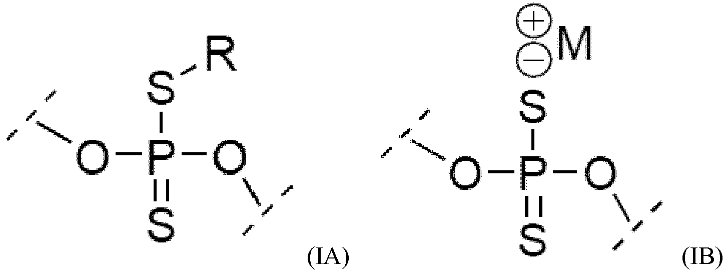 wherein one of the two oxygen atoms is linked to the 3'carbon atom of an adjacent nucleoside (A1) and the other one is linked to the 5'carbon atom of another adjacent nucleoside (A2) and wherein R is hydrogen or a phosphate protecting group.

The oligonucleotide of the disclosure may be a single stranded antisense oligonucleotide, for modulation of a RNA target in a cell, wherein the antisense oligonucleotide comprises or consists of a contiguous nucleotide sequence of 10 - 30 nucleotides in length, wherein the contiguous nucleotide sequence comprises one or more 2'sugar modified nucleosides, and wherein at least one of the internucleoside linkages present between the nucleosides of the contiguous nucleotide sequence is a phosphorodithioate linkage of formula IA or IB 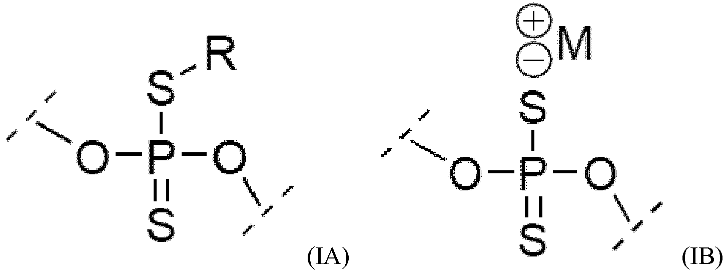 wherein one of the two oxygen atoms is linked to the 3'carbon atom of an adjacent nucleoside (A1) and the other one is linked to the 5'carbon atom of another adjacent nucleoside (A2); and wherein in (IA) R is hydrogen or a phosphate protecting group, and in (IB) M+ is a cation, such as a metal cation, such as an alkali metal cation, such as a Na+ or K+ cation; or M+ is an ammonium cation, and wherein the single stranded antisense oligonucleotide is for use in modulating the splicing of a pre-mRNA target RNA.

The oligonucleotide of the disclosure may be a single stranded antisense oligonucleotide, for modulation of a RNA target in a cell, wherein the antisense oligonucleotide comprises or consists of a contiguous nucleotide sequence of 10 - 30 nucleotides in length, wherein the contiguous nucleotide sequence comprises one or more 2'sugar modified nucleosides, and wherein at least one of the internucleoside linkages present between the nucleosides of the contiguous nucleotide sequence is a phosphorodithioate linkage of formula IA or IB 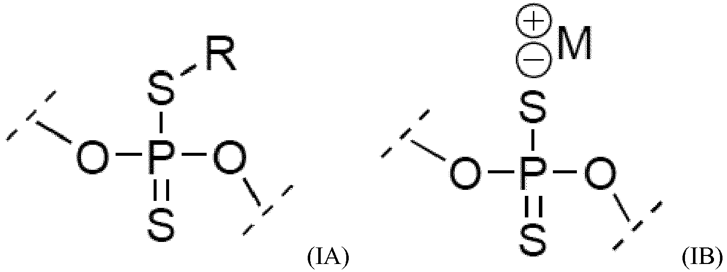 wherein one of the two oxygen atoms is linked to the 3'carbon atom of an adjacent nucleoside (A¹) and the other one is linked to the 5'carbon atom of another adjacent nucleoside (A²); and wherein in (IA) R is hydrogen or a phosphate protecting group, and in (IB) M+ is a cation, such as a metal cation, such as an alkali metal cation, such as a Na+ or K+ cation; or M+ is an ammonium cation, and wherein the single stranded antisense oligonucleotide is for use in inhibiting the expression of a long-non coding RNA. See WO 2012/065143 for examples of lncRNAs which may be targeted by the compounds of the invention.

The oligonucleotide of the disclosure may be a single stranded antisense oligonucleotide, for modulation of a RNA target in a cell, wherein the antisense oligonucleotide comprises or consists of a contiguous nucleotide sequence of 10 - 30 nucleotides in length, wherein the contiguous nucleotide sequence comprises one or more 2'sugar modified nucleosides, and wherein at least one of the internucleoside linkages present between the nucleosides of the contiguous nucleotide sequence is a phosphorodithioate linkage of formula IA or IB 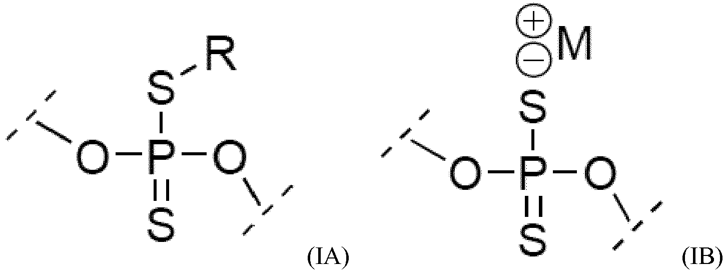 wherein one of the two oxygen atoms is linked to the 3'carbon atom of an adjacent nucleoside (A¹) and the other one is linked to the 5'carbon atom of another adjacent nucleoside (A²); and wherein in (IA) R is hydrogen or a phosphate protecting group, and in (IB) M+ is a cation, such as a metal cation, such as an alkali metal cation, such as a Na+ or K+ cation; or M+ is an ammonium cation, and wherein the single stranded antisense oligonucleotide is for use in inhibiting the expression of a human mRNA or pre-mRNA target.

The oligonucleotide of the disclosure may be a single stranded antisense oligonucleotide, for modulation of a RNA target in a cell, wherein the antisense oligonucleotide comprises or consists of a contiguous nucleotide sequence of 10 - 30 nucleotides in length, wherein the contiguous nucleotide sequence comprises one or more 2'sugar modified nucleosides, and wherein at least one of the internucleoside linkages present between the nucleosides of the contiguous nucleotide sequence is a phosphorodithioate linkage of formula IA or IB 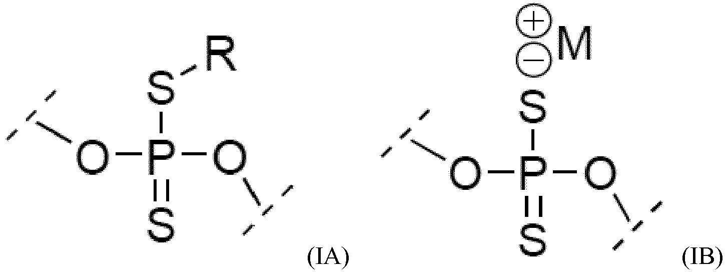 wherein one of the two oxygen atoms is linked to the 3'carbon atom of an adjacent nucleoside (A1) and the other one is linked to the 5'carbon atom of another adjacent nucleoside (A2); and wherein in (IA) R is hydrogen or a phosphate protecting group, and in (IB) M+ is a cation, such as a metal cation, such as an alkali metal cation, such as a Na+ or K+ cation; or M+ is an ammonium cation, and wherein the single stranded antisense oligonucleotide is for use in inhibiting the expression of a viral RNA target. Suitable the viral RNA target may be HCV or HBV for example.

The oligonucleotide of the disclosure may be a single stranded antisense oligonucleotide, for modulation of a RNA target in a cell, wherein the antisense oligonucleotide comprises or consists of a contiguous nucleotide sequence of 7 - 30 nucleotides in length, wherein the contiguous nucleotide sequence comprises one or more 2'sugar modified nucleosides, and wherein at least one of the internucleoside linkages present between the nucleosides of the contiguous nucleotide sequence is a phosphorodithioate linkage of formula IA or IB 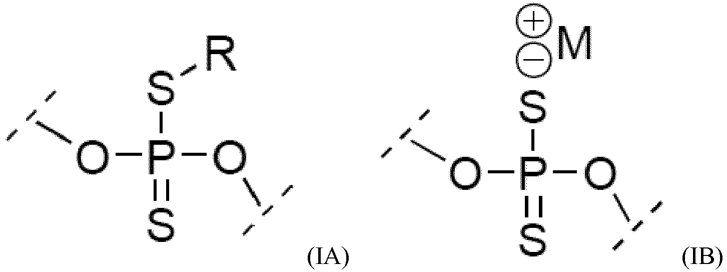 wherein one of the two oxygen atoms is linked to the 3'carbon atom of an adjacent nucleoside (A¹) and the other one is linked to the 5'carbon atom of another adjacent nucleoside (A²); and wherein in (IA) R is hydrogen or a phosphate protecting group, and in (IB) M+ is a cation, such as a metal cation, such as an alkali metal cation, such as a Na+ or K+ cation; or M+ is an ammonium cation, and wherein the single stranded antisense oligonucleotide is for use in inhibiting the expression of a microRNA.

For targeting a RNA target, e.g. a pre-mRNA target, an mRNA target, a viral RNA target, a microRNA or a long non coding RNA target, the oligonucleotide of the invention is suitably capable of inhibiting the expression of the target RNA. This is achieved by the complementarity between tha antisense oligonucleotide and the target RNA. Inhibition of the RNA target may be achieved by reducing the level of the RNA target or by blocking the function of the RNA target. RNA inhibition of an RNA target may suitably be achieved via recruitment of a cellular RNAse such as RNaseH, e.g. via the use of a gapmer, or may be achieved via a non nuclease mediated mechanism, such as a steric blocking mechanism (such as for microRNA inhibition, for splice modulating of pre-mRNAs, or for blocking the interaction between a long non coding RNA and chromatin).

Also described herein is a process for the manufacture of an oligonucleotide according to the invention and to a LNA or MOE nucleoside monomer useful in particular in the manufacture of an oligonucleotide according to the invention.

The invention provides for a pharmaceutically acceptable salt of an oligonucleotide according to the invention, or a conjugate thereof, in particular a sodium or a potassium salt or an ammonium salt.

The invention provides for a conjugate comprising an oligonucleotide, or a pharmaceutically acceptable salt, thereof, and at least one conjugate moiety covalently attached to said oligonucleotide or said pharmaceutically acceptable salt, optionally via a linker moiety.

The invention provides for a pharmaceutical composition comprising an oligonucleotide, pharmaceutically acceptable salt or conjugate according to the invention and a therapeutically inert carrier.

The invention provides for an oligonucleotide, a pharmaceutically acceptable salt or a conjugate according to any the invention for use as a therapeutically active substance.

The oligonucleotide may be used in a method for the modulation of a target RNA in a cell which is expressing said RNA, said method comprising the step of administering an effective amount of the oligonucleotide, pharmaceutically acceptable salt, conjugate or composition according to the invention to the cell, wherein the oligonucleotide is complementary to the target RNA.

The oligonucleotide may be used in a method of modulation of a splicing of a target pre-RNA in a cell which is expressing said target pre-mRNA, said method comprising the step of administering an effective amount of the oligonucleotide, pharmaceutically acceptable salt, conjugate or composition according to the invention to the cell, wherein the oligonucleotide is complementary to the target RNA and is capable of modulating a splicing event in the pre-mRNA.

The oligonucleotide, pharmaceutical salt, conjugate, or composition of the invention may be used for inhibition of a pre-mRNA, an mRNA, or a long-non coding RNA in a cell, such as in a human cell.

The above methods or uses may be an in vitro method or an in vivo method.

The oligonucleotide, pharmaceutical salt, conjugate, or composition of the invention may be used in the manufacture of a medicament.

A phosphorodithioate internucleoside linkage of formula IA or IB, may be for use for enhancing the in vitro or in vivo stability of a single stranded phosphorothioate antisense oligonucleotide.

A phosphorodithioate internucleoside linkage of formula IA or IB, may be for use for enhancing the in vitro or in vivo duration of action a single stranded phosphorothioate antisense oligonucleotide.

A phosphorodithioate internucleoside linkage of formula IA or IB, may be for use for enhancing cellular uptake or tissue distribution of a single stranded phosphorothioate antisense oligonucleotide.

A phosphorodithioate internucleoside linkage of formula IA or IB, may be for use for enhancing uptake of a single stranded phosphorothioate antisense oligonucleotide into a tissue selected from the group consisting of skeletal muscle, heart, epithelial cells, including retinal epithelial cells (e.g. for Htra1 targeting compounds), livre, kidney, or spleen.

For in vivo use a single stranded phosphorothioate antisense oligonucleotide may be a therapeutic oligonucleotide.

### FIGURES

Figures 1-4 show the target mRNA levels in primary rat hepatocytes after 24 and 74 hours of administration of oligonucleotides according to the invention.
Figure 1 shows the target mRNA levels in primary rat hepatocytes after 24 and 74 hours of administration of oligonucleotide gapmers having a single phosphorodithioate internucleoside linkage according the invention in the gap.
Figure 2 shows the target mRNA levels in primary rat hepatocytes after 24 and 74 hours of administration of oligonucleotide gapmers having multiple phosphorodithioate internucleoside linkages according the invention in the gap.
Figure 3 shows the target mRNA levels in primary rat hepatocytes after 24 and 74 hours of administration of oligonucleotide gapmers having multiple phosphorodithioate internucleoside linkages according the invention in the gap.
Figure 4 shows the target mRNA levels in primary rat hepatocytes after 24 and 74 hours of administration of oligonucleotide gapmers having phosphorodithioate internucleoside linkages according the invention in the flanks.
Figure 5 shows the thermal melting (Tm) of oligonuicleotides containing a phophorodithioate internucleoside linkage according to the invention hybridized to RNA and DNA.
Figure 6 shows the stability of oligonucleotides containing a phosphorodithioate internucleoside linkage according to the invention in rat serum.
Figure 7: Exploring achiral phosphodithioate in the gap and flank regions of gapmers - residual mRNA levels after treatment of primary rat hepatocytes.
Figure 8: Exploring positional dependency and optimization of achiral phosphodithioate in the gap regions of gapmers - residual mRNA levels after treatment of primary rat hepatocytes.
Figures 9A-9B: Exploring achiral phosphodithioate in the gap regions of gapmers - effect on cellular uptake.
Figures 10A-10B: Introduction of achiral phosphorodithioate in the flank regions of gapmers provides increased potency, with a correlation between phosphorothioate load with increased potency (4 linkages > 3 linkages >2 linkages >1 linkage>no phosphorodithioate linkages in the flanks).
Figure 11: IC50 values in difference cell types.
Figure 12: In vitro rat serum stability of 3' end protected LNA oligonucleotides.
Figure 13: In vivo evaluation of gapmers containing achiral phosphorodithioate linkages in the flanks and the gap regions - Target inhibition.
Figure 14A: In vivo evaluation of gapmers containing achiral phosphorodithioate linkages in the flanks and the gap regions - Tissue uptake.
Figure 14B: In vivo evaluation of gapmers containing achiral phosphorodithioate linkages in the flanks and the gap regions - Liver/kidney ratio.
Figures 15A and 15B: In vivo evaluation of gapmers containing achiral phosphorodithioate linkages in the flanks and the gap regions - metabolite analysis.
Figure 16: The prolonged duration of action with antisense oligonucleotides comprising achiral phosphorodithioate internucleoside linkages can be further enhanced by combination with stereodefined phosphorothioate internucleoside linkages.
Figure 17A: In vitro EC50 determination of achiral phosphorodithioate gapmers targeting MALAT-1.
Figure 17B: In vivo potency of achiral phosphorodithioate gapmers targeting MALAT-1.
Figure 17C: In vivo study of achiral phosphorodithioate gapmers targeting MALAT-1 - tissue content
Figure 18A: In vitro study of achiral monophosphorothioate modified gapmer oligonucleotides targeting ApoB. Activity data.
Figure 18B: In vitro study of achiral monophosphorothioate modified gapmer oligonucleotides targeting ApoB. Cellular content data.
Figure 19A: In vitro study of chiral phosphorodithioate modified gapmer oligonucleotides targeting ApoB. Activity data.
Figure 19B: In vitro study of chiral phosphorodithioate modified gapmer oligonucleotides targeting ApoB. Cellular content data.
Figure 20: Effects of achiral phosphorodithioates (P2S) internucleoside linkages present in splice-switching oligonucleotide targeting the 3' splice site of TNFRSF1B. Human Colo 205 cells was seeded in a 96 well plate and subjected to 5 µM (A) and 25 µM (B) of oligo, respectively. The percentage of exon 7 skipping was analyzed by droplet digital PCR using probes targeting the exon 6-8 junction and compared to the total amount of TNFRSF1B by the assay targeting exon 2-3. SSO#26 is the parent oligo, and SSO#27 is a negative control not targeting TNFRSF1B.
Figure 21: Stability assay using S1 nuclease. Dithioate containing oligos were incubated with S1 nuclease for 30 and 120 minutes, respectively. The oligos were visualized on a 15% TBE-Urea gel. As marker of the migration of intact oligos (SSO#14) was included without being subjected to S1 nuclease.

### DEFINITIONS

In the present description the term "alkyl", alone or in combination, signifies a straight-chain or branched-chain alkyl group with 1 to 8 carbon atoms, particularly a straight or branched-chain alkyl group with 1 to 6 carbon atoms and more particularly a straight or branched-chain alkyl group with 1 to 4 carbon atoms. Examples of straight-chain and branched-chain Ci-Cs alkyl groups are methyl, ethyl, propyl, isopropyl, butyl, isobutyl, tert.-butyl, the isomeric pentyls, the isomeric hexyls, the isomeric heptyls and the isomeric octyls, particularly methyl, ethyl, propyl, butyl and pentyl. Particular examples of alkyl are methyl, ethyl and propyl.

The term "cycloalkyl", alone or in combination, signifies a cycloalkyl ring with 3 to 8 carbon atoms and particularly a cycloalkyl ring with 3 to 6 carbon atoms. Examples of cycloalkyl are cyclopropyl, cyclobutyl, cyclopentyl, cyclohexyl, cycloheptyl and cyclooctyl, more particularly cyclopropyl and cyclobutyl. A particular example of "cycloalkyl" is cyclopropyl.

The term "alkoxy", alone or in combination, signifies a group of the formula alkyl-O- in which the term "alkyl" has the previously given significance, such as methoxy, ethoxy, n-propoxy, isopropoxy, n-butoxy, isobutoxy, sec.butoxy and tert.butoxy. Particular "alkoxy" are methoxy and ethoxy. Methoxyethoxy is a particular example of "alkoxyalkoxy".

The term "oxy", alone or in combination, signifies the -O- group.

The term "alkenyl", alone or in combination, signifies a straight-chain or branched hydrocarbon residue comprising an olefinic bond and up to 8, preferably up to 6, particularly preferred up to 4 carbon atoms. Examples of alkenyl groups are ethenyl, 1-propenyl, 2-propenyl, isopropenyl, 1-butenyl, 2-butenyl, 3-butenyl and isobutenyl.

The term "alkynyl", alone or in combination, signifies a straight-chain or branched hydrocarbon residue comprising a triple bond and up to 8, particularly 2 carbon atoms.

The terms "halogen" or "halo", alone or in combination, signifies fluorine, chlorine, bromine or iodine and particularly fluorine, chlorine or bromine, more particularly fluorine. The term "halo", in combination with another group, denotes the substitution of said group with at least one halogen, particularly substituted with one to five halogens, particularly one to four halogens, i.e. one, two, three or four halogens.

The term "haloalkyl", alone or in combination, denotes an alkyl group substituted with at least one halogen, particularly substituted with one to five halogens, particularly one to three halogens. Examples of haloalkyl include monofluoro-, difluoro- or trifluoromethyl, -ethyl or -propyl, for example 3,3,3-trifluoropropyl, 2-fluoroethyl, 2,2,2-trifluoroethyl, fluoromethyl or trifluoromethyl. Fluoromethyl, difluoromethyl and trifluoromethyl are particular "haloalkyl".

The term "halocycloalkyl", alone or in combination, denotes a cycloalkyl group as defined above substituted with at least one halogen, particularly substituted with one to five halogens, particularly one to three halogens. Particular example of "halocycloalkyl" are halocyclopropyl, in particular fluorocyclopropyl, difluorocyclopropyl and trifluorocyclopropyl.

The terms "hydroxyl" and "hydroxy", alone or in combination, signify the -OH group.

The terms "thiohydroxyl" and "thiohydroxy", alone or in combination, signify the - SH group.

The term "carbonyl", alone or in combination, signifies the -C(O)- group.

The term "carboxy" or "carboxyl", alone or in combination, signifies the -COOH group.

The term "amino", alone or in combination, signifies the primary amino group (-NH₂), the secondary amino group (-NH-), or the tertiary amino group (-N-).

The term "alkylamino", alone or in combination, signifies an amino group as defined above substituted with one or two alkyl groups as defined above.

The term "sulfonyl", alone or in combination, means the -SO₂ group.

The term "sulfinyl", alone or in combination, signifies the -SO- group.

The term "sulfanyl", alone or in combination, signifies the -S- group.

The term "cyano", alone or in combination, signifies the -CN group.

The term "azido", alone or in combination, signifies the -N₃ group.

The term "nitro", alone or in combination, signifies the NO₂ group.

The term "formyl", alone or in combination, signifies the -C(O)H group.

The term "carbamoyl", alone or in combination, signifies the -C(O)NH₂ group.

The term "cabamido", alone or in combination, signifies the -NH-C(O)-NH₂ group.

The term "aryl", alone or in combination, denotes a monovalent aromatic carbocyclic mono- or bicyclic ring system comprising 6 to 10 carbon ring atoms, optionally substituted with 1 to 3 substituents independently selected from halogen, hydroxyl, alkyl, alkenyl, alkynyl, alkoxy, alkoxyalkyl, alkenyloxy, carboxyl, alkoxycarbonyl, alkylcarbonyl and formyl. Examples of aryl include phenyl and naphthyl, in particular phenyl.

The term "heteroaryl", alone or in combination, denotes a monovalent aromatic heterocyclic mono- or bicyclic ring system of 5 to 12 ring atoms, comprising 1, 2, 3 or 4 heteroatoms selected from N, O and S, the remaining ring atoms being carbon, optionally substituted with 1 to 3 substituents independently selected from halogen, hydroxyl, alkyl, alkenyl, alkynyl, alkoxy, alkoxyalkyl, alkenyloxy, carboxyl, alkoxycarbonyl, alkylcarbonyl and formyl. Examples of heteroaryl include pyrrolyl, furanyl, thienyl, imidazolyl, oxazolyl, thiazolyl, triazolyl, oxadiazolyl, thiadiazolyl, tetrazolyl, pyridinyl, pyrazinyl, pyrazolyl, pyridazinyl, pyrimidinyl, triazinyl, azepinyl, diazepinyl, isoxazolyl, benzofuranyl, isothiazolyl, benzothienyl, indolyl, isoindolyl, isobenzofuranyl, benzimidazolyl, benzoxazolyl, benzoisoxazolyl, benzothiazolyl, benzoisothiazolyl, benzooxadiazolyl, benzothiadiazolyl, benzotriazolyl, purinyl, quinolinyl, isoquinolinyl, quinazolinyl, quinoxalinyl, carbazolyl or acridinyl.

The term "heterocyclyl", alone or in combination, signifies a monovalent saturated or partly unsaturated mono- or bicyclic ring system of 4 to 12, in particular 4 to 9 ring atoms, comprising 1, 2, 3 or 4 ring heteroatoms selected from N, O and S, the remaining ring atoms being carbon, optionally substituted with 1 to 3 substituents independently selected from halogen, hydroxyl, alkyl, alkenyl, alkynyl, alkoxy, alkoxyalkyl, alkenyloxy, carboxyl, alkoxycarbonyl, alkylcarbonyl and formyl. Examples for monocyclic saturated heterocyclyl are azetidinyl, pyrrolidinyl, tetrahydrofuranyl, tetrahydro-thienyl, pyrazolidinyl, imidazolidinyl, oxazolidinyl, isoxazolidinyl, thiazolidinyl, piperidinyl, tetrahydropyranyl, tetrahydrothiopyranyl, piperazinyl, morpholinyl, thiomorpholinyl, 1,1-dioxo-thiomorpholin-4-yl, azepanyl, diazepanyl, homopiperazinyl, or oxazepanyl. Examples for bicyclic saturated heterocycloalkyl are 8-aza-bicyclo[3.2.1]octyl, quinuclidinyl, 8-oxa-3-aza-bicyclo[3.2.1]octyl, 9-aza-bicyclo[3.3.1]nonyl, 3-oxa-9-aza-bicyclo[3.3.1]nonyl, or 3-thia-9-aza-bicyclo[3.3.1]nonyl. Examples for partly unsaturated heterocycloalkyl are dihydrofuryl, imidazolinyl, dihydro-oxazolyl, tetrahydro-pyridinyl or dihydropyranyl.

The term "pharmaceutically acceptable salts" refers to those salts which retain the biological effectiveness and properties of the free bases or free acids, which are not biologically or otherwise undesirable. The salts are formed with inorganic acids such as hydrochloric acid, hydrobromic acid, sulfuric acid, nitric acid, phosphoric acid, particularly hydrochloric acid, and organic acids such as acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid, N-acetylcystein. In addition, these salts may be prepared form addition of an inorganic base or an organic base to the free acid. Salts derived from an inorganic base include, but are not limited to, the sodium, potassium, lithium, ammonium, calcium, magnesium salts. Salts derived from organic bases include, but are not limited to salts of primary, secondary, and tertiary amines, substituted amines including naturally occurring substituted amines, cyclic amines and basic ion exchange resins, such as isopropylamine, trimethylamine, diethylamine, triethylamine, tripropylamine, ethanolamine, lysine, arginine, N-ethylpiperidine, piperidine, polyamine resins. The oligonucleotide of the invention can also be present in the form of zwitterions. Particularly preferred pharmaceutically acceptable salts of the invention are the sodium, lithium, potassium and trialkylammonium salts.

The term "protecting group", alone or in combination, signifies a group which selectively blocks a reactive site in a multifunctional compound such that a chemical reaction can be carried out selectively at another unprotected reactive site. Protecting groups can be removed. Exemplary protecting groups are amino-protecting groups, carboxy-protecting groups or hydroxy-protecting groups.

"Phosphate protecting group" is a protecting group of the phosphate group. Examples of phosphate protecting group are 2-cyanoethyl and methyl. A particular example of phosphate protecting group is 2-cyanoethyl.

"Hydroxyl protecting group" is a protecting group of the hydroxyl group and is also used to protect thiol groups. Examples of hydroxyl protecting groups are acetyl (Ac), benzoyl (Bz), benzyl (Bn), β-methoxyethoxymethyl ether (MEM), dimethoxytrityl (or bis-(4-methoxyphenyl)phenylmethyl) (DMT), trimethoxytrityl (or tris-(4-methoxyphenyl)phenylmethyl) (TMT), methoxymethyl ether (MOM), methoxytrityl [(4-methoxyphenyl)diphenylmethyl (MMT), p-methoxybenzyl ether (PMB), methylthiomethyl ether, pivaloyl (Piv), tetrahydropyranyl (THP), tetrahydrofuran (THF), trityl or triphenylmethyl (Tr), silyl ether (for example trimethylsilyl (TMS), tert-butyldimethylsilyl (TBDMS), tri-iso-propylsilyloxymethyl (TOM) and triisopropylsilyl (TIPS) ethers), methyl ethers and ethoxyethyl ethers (EE). Particular examples of hydroxyl protecting group are DMT and TMT, in particular DMT.

"Thiohydroxyl protecting group" is a protecting group of the thiohydroxyl group. Examples of thiohydroxyl protecting groups are those of the "hydroxyl protecting group".

If one of the starting materials or compounds of the invention contain one or more functional groups which are not stable or are reactive under the reaction conditions of one or more reaction steps, appropriate protecting groups (as described e.g. in "Protective Groups in Organic Chemistry" by T. W. Greene and P. G. M. Wuts, 3rd Ed., 1999, Wiley, New York) can be introduced before the critical step applying methods well known in the art. Such protecting groups can be removed at a later stage of the synthesis using standard methods described in the literature. Examples of protecting groups are tert-butoxycarbonyl (Boc), 9-fluorenylmethyl carbamate (Fmoc), 2-trimethylsilylethyl carbamate (Teoc), carbobenzyloxy (Cbz) and p-methoxybenzyloxycarbonyl (Moz).

The compounds described herein can contain several asymmetric centers and can be present in the form of optically pure enantiomers, mixtures of enantiomers such as, for example, racemates, mixtures of diastereoisomers, diastereoisomeric racemates or mixtures of diastereoisomeric racemates.

### Oligonucleotide

The term "oligonucleotide" as used herein is defined as it is generally understood by the skilled person as a molecule comprising two or more covalently linked nucleosides. Such covalently bound nucleosides may also be referred to as nucleic acid molecules or oligomers. Oligonucleotides are commonly made in the laboratory by solid-phase chemical synthesis followed by purification. When referring to a sequence of the oligonucleotide, reference is made to the sequence or order of nucleobase moieties, or modifications thereof, of the covalently linked nucleotides or nucleosides. The oligonucleotide of the invention is man-made, and is chemically synthesized, and is typically purified or isolated. The oligonucleotide of the invention may comprise one or more modified nucleosides or nucleotides.

### Antisense oligonucleotides

The term "Antisense oligonucleotide" as used herein is defined as oligonucleotides capable of modulating expression of a target gene by hybridizing to a target nucleic acid, in particular to a contiguous sequence on a target nucleic acid. The antisense oligonucleotides are not essentially double stranded and are therefore not siRNAs or shRNAs. Preferably, the antisense oligonucleotides of the present invention are single stranded. It is understood that single stranded oligonucleotides of the present invention can form hairpins or intermolecular duplex structures (duplex between two molecules of the same oligonucleotide), as long as the degree of intra or inter self complementarity is less than 50% across of the full length of the oligonucleotide.

### Modulation of expression

The term "modulation of expression" as used herein is to be understood as an overall term for an oligonucleotide's ability to alter the expression of or alter the level of the target nucleic acid. Modulation of expression may be determined by comparison to expression or level of the target nucleic acid prior to administration of the oligonucleotide, or modulation of expression may be determined by reference to a control experiment where the oligonucleotide of the invention is not administered. It is generally understood that the control is an individual or target cell treated with a saline composition or an individual or target cell treated with a non-targeting oligonucleotide (mock).

One type of modulation is the ability of an oligonucleotide's ability to inhibit, down-regulate, reduce, suppress, remove, stop, block, prevent, lessen, lower, avoid or terminate expression of the target nucleic acid e.g. by degradation of the target nucleic acid (e.g. via RNaseH1 mediated degradation) or blockage of transcription. Another type of modulation is an oligonucleotide's ability to restore, increase or enhance expression of the target RNA, e.g. modulating the splicing event on a target pre-mRNA, or via blockage of inhibitory mechanisms such as microRNA repression of an mRNA.

### Contiguous Nucleotide Sequence

The term "contiguous nucleotide sequence" refers to the region of the oligonucleotide which is complementary to, such as fully complementary to, the target nucleic acid. The term is used interchangeably herein with the term "contiguous nucleobase sequence" and the term "oligonucleotide motif sequence". In some embodiments all the nucleotides of the oligonucleotide constitute the contiguous nucleotide sequence. In some embodiments the oligonucleotide comprises the contiguous nucleotide sequence, such as a F-G-F' gapmer region, and may optionally comprise further nucleotide(s), for example a nucleotide linker region which may be used to attach a functional group to the contiguous nucleotide sequence, e.g. region D or D'. The nucleotide linker region may or may not be complementary to the target nucleic acid. The antisense oligonucleotide mixmer referred to herein may comprise or may consist of the contiguous nucleotide sequence.

### Nucleotides

Nucleotides are the building blocks of oligonucleotides and polynucleotides, and for the purposes of the present invention include both naturally occurring and non-naturally occurring nucleotides. In nature, nucleotides, such as DNA and RNA nucleotides comprise a ribose sugar moiety, a nucleobase moiety and one or more phosphate groups (which is absent in nucleosides). Nucleosides and nucleotides may also interchangeably be referred to as "units" or "monomers".

### Modified nucleoside

The term "modified nucleoside" or "nucleoside modification" as used herein refers to nucleosides modified as compared to the equivalent DNA or RNA nucleoside by the introduction of one or more modifications of the sugar moiety or the (nucleo)base moiety. In a preferred embodiment the modified nucleoside comprises a modified sugar moiety. The term modified nucleoside may also be used herein interchangeably with the term "nucleoside analogue" or modified "units" or modified "monomers". Nucleosides with an unmodified DNA or RNA sugar moiety are termed DNA or RNA nucleosides herein. Nucleosides with modifications in the base region of the DNA or RNA nucleoside are still generally termed DNA or RNA if they allow Watson Crick base pairing.

### Modified internucleoside linkage

The term "modified internucleoside linkage" is defined as generally understood by the skilled person as linkages other than phosphodiester (PO) linkages, that covalently couples two nucleosides together. The oligonucleotides of the invention may therefore comprise modified internucleoside linkages. In some embodiments, the modified internucleoside linkage increases the nuclease resistance of the oligonucleotide compared to a phosphodiester linkage. For naturally occurring oligonucleotides, the internucleoside linkage includes phosphate groups creating a phosphodiester bond between adjacent nucleosides. Modified internucleoside linkages are particularly useful in stabilizing oligonucleotides for *in vivo* use, and may serve to protect against nuclease cleavage at regions of DNA or RNA nucleosides in the oligonucleotide of the invention, for example within the gap region of a gapmer oligonucleotide, as well as in regions of modified nucleosides, such as region F and F'.

In an embodiment, the oligonucleotide comprises one or more internucleoside linkages modified from the natural phosphodiester, such one or more modified internucleoside linkages that is for example more resistant to nuclease attack. Nuclease resistance may be determined by incubating the oligonucleotide in blood serum or by using a nuclease resistance assay (e.g. snake venom phosphodiesterase (SVPD)), both are well known in the art. Internucleoside linkages which are capable of enhancing the nuclease resistance of an oligonucleotide are referred to as nuclease resistant internucleoside linkages. In some embodiments at least 50% of the internucleoside linkages in the oligonucleotide, or contiguous nucleotide sequence thereof, are modified, such as at least 60%, such as at least 70%, such as at least 80 or such as at least 90% of the internucleoside linkages in the oligonucleotide, or contiguous nucleotide sequence thereof, are nuclease resistant internucleoside linkages. In some embodiments all of the internucleoside linkages of the oligonucleotide, or contiguous nucleotide sequence thereof, are nuclease resistant internucleoside linkages. It will be recognized that, in some embodiments the nucleosides which link the oligonucleotide of the invention to a non-nucleotide functional group, such as a conjugate, may be phosphodiester.

A preferred modified internucleoside linkage for use in the oligonucleotide of the invention is phosphorothioate.

Phosphorothioate internucleoside linkages are particularly useful due to nuclease resistance, beneficial pharmacokinetics and ease of manufacture. In some embodiments at least 50% of the internucleoside linkages in the oligonucleotide, or contiguous nucleotide sequence thereof, are phosphorothioate, such as at least 60%, such as at least 70%, such as at least 80% or such as at least 90% of the internucleoside linkages in the oligonucleotide, or contiguous nucleotide sequence thereof, are phosphorothioate. In the oligonucleotides of the invention, other than the phosphorodithioate internucleoside linkages, all of the internucleoside linkages of the oligonucleotide, or contiguous nucleotide sequence thereof, are phosphorothioate.

Nuclease resistant linkages, such as phosphorothioate linkages, are particularly useful in oligonucleotide regions capable of recruiting nuclease when forming a duplex with the target nucleic acid, such as region G for gapmers. Phosphorothioate linkages may, however, also be useful in non-nuclease recruiting regions and/or affinity enhancing regions such as regions F and F' for gapmers. Gapmer oligonucleotides may, in some embodiments comprise one or more phosphodiester linkages in region F or F', or both region F and F', which the internucleoside linkage in region G may be fully phosphorothioate.

Advantageously, all the internucleoside linkages in the contiguous nucleotide sequence of the oligonucleotide, or all the internucleoside linkages of the oligonucleotide, are phosphorothioate linkages.

It is recognized that, as disclosed in EP 2 742 135, antisense oligonucleotides may comprise other internucleoside linkages (other than phosphodiester and phosphorothioate), for example alkyl phosphonate/methyl phosphonate internucleosides, which according to EP 2 742 135 may for example be tolerated in an otherwise DNA phosphorothioate the gap region.

### Stereorandom Phosphorothioate Linkages

Phosphorothioate linkages are internucleoside phosphate linkages where one of the non-bridging oxygens has been substituted with a sulfur. The substitution of one of the non-bridging oxygens with a sulfur introduces a chiral center, and as such within a single phosphorothioate oligonucleotide, each phosphorothioate internucleoside linkage will be either in the S (Sp) or R (Rp) stereoisoforms. Such internucleoside linkages are referred to as "chiral internucleoside linkages". By comparison, phosphodiester internucleoside linkages are non-chiral as they have two non-terminal oxygen atoms.

The designation of the chirality of a stereocenter is determined by standard Cahn-Ingold-Prelog rules (CIP priority rules) first published in Cahn, R.S.; Ingold, C.K.; Prelog, V. (1966) "Specification of Molecular Chirality" Angewandte Chemie International Edition 5 (4): 385-415. doi:10.1002/anie. 196603851.

During standard oligonucleotide synthesis the stereoselectivity of the coupling and the following sulfurization is not controlled. For this reason, the stereochemistry of each phosphorothioate internucleoside linkages is randomly Sp or Rp, and as such a phosphorothioate oligonucleotide produced by traditional oligonucleotide synthesis actually can exist in as many as 2^{X} different phosphorothioate diastereoisomers, where X is the number of phosphorothioate internucleoside linkages. Such oligonucleotides are referred to as stereorandom phosphorothioate oligonucleotides herein, and do not contain any stereodefined internucleoside linkages. Stereorandom phosphorothioate oligonucleotides are therefore mixtures of individual diastereoisomers originating from the non-stereodefined synthesis. In this context the mixture is defined as up to 2^{X} different phosphorothioate diastereoisomers.

### Stereodefined Internucleoside Linkages

A stereodefined internucleoside linkage is a chiral internucleoside linkage having a diastereoisomeric excess for one of its two diastereomeric forms, Rp or Sp.

It should be recognized that stereoselective oligonucleotide synthesis methods used in the art typically provide at least about 90% or at least about 95% diastereoselectivity at each chiral internucleoside linkage, and as such up to about 10%, such as about 5% of oligonucleotide molecules may have the alternative diastereoisomeric form.

In some embodiments the diastereoisomeric ratio of each stereo defined chiral internucleoside linkage is at least about 90:10. In some embodiments the diastereoisomeric ratio of each chiral internucleoside linkage is at least about 95:5.

The stereodefined phosphorothioate linkage is a particular example of stereo defined internucleoside linkage.

### Stereodefinedphosphorothioate linkage

A stereodefined phosphorothioate linkage is a phosphorothioate linkage having a diastereomeric excess for one of its two diastereosiomeric forms, Rp or Sp.

The Rp and Sp configurations of the phosphorothioate internucleoside linkages are presented below

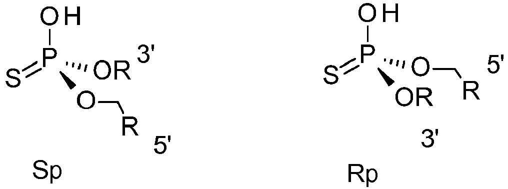

Where the 3' R group represents the 3' position of the adjacent nucleoside (a 5' nucleoside), and the 5' R group represents the 5' position of the adjacent nucleoside (a 3' nucleoside).

Rp internucleoside linkages may also be represented as srP, and Sp internucleoside linkages may be represented as ssP herein.

In a particular embodiment, the diastereomeric ratio of each stereo defined phosphorothioate linkage is at least about 90:10 or at least 95:5.

In some embodiments the diastereomeric ratio of each stereodefined phosphorothioate linkage is at least about 97:3. In some embodiments the diastereomeric ratio of each stereodefined phosphorothioate linkage is at least about 98:2. In some embodiments the diastereomeric ratio of each stereodefined phosphorothioate linkage is at least about 99:1.

In some embodiments a stereodefined internucleoside linkage is in the same diastereomeric form (Rp or Sp) in at least 97%, such as at least 98%, such as at least 99%, or (essentially) all of the oligonucleotide molecules present in a population of the oligonucleotide molecule.

Diastereomeric purity can be measured in a model system only having an achiral backbone (*i.e*. phosphodiesters). It is possible to measure the diastereomeric purity of each monomer by e.g. coupling a monomer having a stereodefine internucleoside linkage to the following model-system "5' t-po-t-po-t-po 3 "'. The result of this will then give : 5' DMTr-t-srp-t-po-t-po-t-po 3' or 5' DMTr-t-ssp-t-po-t-po-t-po 3' which can be separated using HPLC. The diastereomeric purity is determined by integrating the UV signal from the two possible diastereoisomers and giving a ratio of these e.g. 98:2, 99:1 or >99:1.

It will be understood that the diastereomeric purity of a specific single diastereoisomer (a single stereo defined oligonucleotide molecule) will be a function of the coupling selectivity for the defined stereocenter at each internucleoside position, and the number of stereo defined internucleoside linkages to be introduced. By way of example, if the coupling selectivity at each position is 97%, the resulting purity of the stereodefined oligonucleotide with 15 stereodefined internucleoside linkages will be 0.97¹⁵, *i.e.* 63% of the desired diastereoisomer as compared to 37% of the other diastereoisomers. The purity of the defined diastereoisomer may after synthesis be improved by purification, for example by HPLC, such as ion exchange chromatography or reverse phase chromatography.

In some embodiments, a stereodefined oligonucleotide refers to a population of an oligonucleotide wherein at least about 40%, such as at least about 50% of the population is of the desired diastereoisomer.

Alternatively stated, in some embodiments, a stereo defined oligonucleotide refers to a population of oligonucleotides wherein at least about 40%, such as at least about 50%, of the population consists of the desired (specific) stereodefined internucleoside linkage motifs (also termed stereodefined motif).

For stereo defined oligonucleotides which comprise both stereorandom and stereodefined internucleoside chiral centers, the purity of the stereodefined oligonucleotide is determined with reference to the % of the population of the oligonucleotide which retains the desired stereo defined internucleoside linkage motif(s), the stereorandom linkages being disregarded in the calculation.

### Nucleobase

The term nucleobase includes the purine (e.g. adenine and guanine) and pyrimidine (e.g. uracil, thymine and cytosine) moieties present in nucleosides and nucleotides which form hydrogen bonds in nucleic acid hybridization. In the context of the present invention the term nucleobase also encompasses modified nucleobases which may differ from naturally occurring nucleobases, but are functional during nucleic acid hybridization. In this context "nucleobase" refers to both naturally occurring nucleobases such as adenine, guanine, cytosine, thymidine, uracil, xanthine and hypoxanthine, as well as non-naturally occurring variants. Such variants are for example described in Hirao et al (2012) Accounts of Chemical Research vol 45 page 2055 and Bergstrom (2009) Current Protocols in Nucleic Acid Chemistry Suppl. 37 1.4.1.

In some embodiments the nucleobase moiety is modified by changing the purine or pyrimidine into a modified purine or pyrimidine, such as substituted purine or substituted pyrimidine, such as a nucleobase selected from isocytosine, pseudoisocytosine, 5-methyl cytosine, 5-thiozolo-cytosine, 5-propynyl-cytosine, 5-propynyl-uracil, 5-bromouracil 5-thiazolo-uracil, 2-thio-uracil, 2'thio-thymine, inosine, diaminopurine, 6-aminopurine, 2-aminopurine, 2,6-diaminopurine and 2-chloro-6-aminopurine.

The nucleobase moieties may be indicated by the letter code for each corresponding nucleobase, e.g. A, T, G, C or U, wherein each letter may optionally include modified nucleobases of equivalent function. For example, in the exemplified oligonucleotides, the nucleobase moieties are selected from A, T, G, C, and 5-methyl cytosine. Optionally, for LNA gapmers, 5-methyl cytosine LNA nucleosides may be used.

### Modified oligonucleotide

The term modified oligonucleotide describes an oligonucleotide comprising one or more sugar-modified nucleosides and/or modified internucleoside linkages. The term chimeric" oligonucleotide is a term that has been used in the literature to describe oligonucleotides with modified nucleosides.

### Stereodefined oligonucleotide

A stereodefined oligonucleotide is an oligonucleotide wherein at least one of the internucleoside linkages is a stereodefined internucleoside linkage.

A stereodefined phosphorothioate oligonucleotide is an oligonucleotide wherein at least one of the internucleoside linkages is a stereodefined phosphorothioate internucleoside linkage.

### Complementarity

The term "complementarity" describes the capacity for Watson-Crick base-pairing of nucleosides/nucleotides. Watson-Crick base pairs are guanine (G)-cytosine (C) and adenine (A) - thymine (T)/uracil (U). It will be understood that oligonucleotides may comprise nucleosides with modified nucleobases, for example 5-methyl cytosine is often used in place of cytosine, and as such the term complementarity encompasses Watson Crick base-paring between non-modified and modified nucleobases (see for example Hirao et al (2012) Accounts of Chemical Research vol 45 page 2055 and Bergstrom (2009) Current Protocols in Nucleic Acid Chemistry Suppl. 37 1.4.1).

The term "% complementary" as used herein, refers to the proportion of nucleotides in a contiguous nucleotide sequence in a nucleic acid molecule (e.g. oligonucleotide) which, at a given position, are complementary to (*i.e.* form Watson Crick base pairs with) a contiguous nucleotide sequence, at a given position of a separate nucleic acid molecule (e.g. the target nucleic acid). The percentage is calculated by counting the number of aligned bases that form pairs between the two sequences (when aligned with the target sequence 5'-3' and the oligonucleotide sequence from 3'-5'), dividing by the total number of nucleotides in the oligonucleotide and multiplying by 100. In such a comparison a nucleobase/nucleotide which does not align (form a base pair) is termed a mismatch. Preferably, insertions and deletions are not allowed in the calculation of % complementarity of a contiguous nucleotide sequence.

The term "fully complementary", refers to 100% complementarity.

### Identity

The term "Identity" as used herein, refers to the number of nucleotides in percent of a contiguous nucleotide sequence in a nucleic acid molecule (e.g. oligonucleotide) which, at a given position, are identical to (*i.e.* in their ability to form Watson Crick base pairs with the complementary nucleoside) a contiguous nucleotide sequence, at a given position of a separate nucleic acid molecule (e.g. the target nucleic acid). The percentage is calculated by counting the number of aligned bases that are identical between the two sequences dividing by the total number of nucleotides in the oligonucleotide and multiplying by 100. Percent Identity = (Matches x 100)/Length of aligned region. Preferably, insertions and deletions are not allowed in the calculation of % complementarity of a contiguous nucleotide sequence.

### Hybridization

The term "hybridizing" or "hybridizes" as used herein is to be understood as two nucleic acid strands (e.g. an oligonucleotide and a target nucleic acid) forming hydrogen bonds between base pairs on opposite strands thereby forming a duplex. The affinity of the binding between two nucleic acid strands is the strength of the hybridization. It is often described in terms of the melting temperature (Tₘ) defined as the temperature at which half of the oligonucleotides are duplexed with the target nucleic acid. At physiological conditions Tₘ is not strictly proportional to the affinity (Mergny and Lacroix, 2003,Oligonucleotides 13:515-537). The standard state Gibbs free energy ΔG° is a more accurate representation of binding affinity and is related to the dissociation constant (K_{d}) of the reaction by ΔG°=-RTln(K_{d}), where R is the gas constant and T is the absolute temperature. Therefore, a very low ΔG° of the reaction between an oligonucleotide and the target nucleic acid reflects a strong hybridization between the oligonucleotide and target nucleic acid. ΔG° is the energy associated with a reaction where aqueous concentrations are 1M, the pH is 7, and the temperature is 37°C. The hybridization of oligonucleotides to a target nucleic acid is a spontaneous reaction and for spontaneous reactions ΔG° is less than zero. ΔG° can be measured experimentally, for example, by use of the isothermal titration calorimetry (ITC) method as described in Hansen et al., 1965, Chem. Comm. 36-38 and Holdgate et al., 2005, Drug Discov Today*.* The skilled person will know that commercial equipment is available for ΔG° measurements. ΔG° can also be estimated numerically by using the nearest neighbor model as described by SantaLucia, 1998, Proc Natl Acad Sci USA. 95: 1460-1465 using appropriately derived thermodynamic parameters described by Sugimoto et al., 1995, Biochemistry 34:11211-11216 and McTigue et al., 2004, Biochemistry 43:5388-5405. In order to have the possibility of modulating its intended nucleic acid target by hybridization, oligonucleotides of the present invention hybridize to a target nucleic acid with estimated ΔG° values below -10 kcal for oligonucleotides that are 10-30 nucleotides in length. In some embodiments the degree or strength of hybridization is measured by the standard state Gibbs free energy ΔG°. The oligonucleotides may hybridize to a target nucleic acid with estimated ΔG° values below the range of -10 kcal, such as below -15 kcal, such as below -20 kcal and such as below -25 kcal for oligonucleotides that are 8-30 nucleotides in length. In some embodiments the oligonucleotides hybridize to a target nucleic acid with an estimated ΔG° value of -10 to -60 kcal, such as -12 to -40, such as from -15 to -30 kcal or-16 to -27 kcal such as -18 to -25 kcal.

### Sugar modifications

The oligomer of the invention comprises one or more nucleosides which have a modified sugar moiety, *i.e.* a modification of the sugar moiety when compared to the ribose sugar moiety found in DNA and RNA.

Numerous nucleosides with modification of the ribose sugar moiety have been made, primarily with the aim of improving certain properties of oligonucleotides, such as affinity and/or nuclease resistance.

Such modifications include those where the ribose ring structure is modified, *e.g.* by replacement with a hexose ring (HNA), or a bicyclic ring, which typically have a biradical bridge between the C2 and C4 carbons on the ribose ring (LNA), or an unlinked ribose ring which typically lacks a bond between the C2 and C3 carbons (e.g. UNA). Other sugar modified nucleosides include, for example, bicyclohexose nucleic acids (WO 2011/017521) or tricyclic nucleic acids (WO 2013/154798). Modified nucleosides also include nucleosides where the sugar moiety is replaced with a non-sugar moiety, for example in the case of peptide nucleic acids (PNA), or morpholino nucleic acids.

Sugar modifications also include modifications made via altering the substituent groups on the ribose ring to groups other than hydrogen, or the 2'-OH group naturally found in DNA and RNA nucleosides. Substituents may, for example be introduced at the 2', 3', 4' or 5' positions.

### 2' sugar modified nucleosides.

A 2' sugar modified nucleoside is a nucleoside which has a substituent other than H or -OH at the 2' position (2' substituted nucleoside) or comprises a 2' linked biradical capable of forming a bridge between the 2' carbon and a second carbon in the ribose ring, such as LNA (2' - 4' biradical bridged) nucleosides.

Indeed, much focus has been spent on developing 2' substituted nucleosides, and numerous 2' substituted nucleosides have been found to have beneficial properties when incorporated into oligonucleotides. For example, the 2' modified sugar may provide enhanced binding affinity and/or increased nuclease resistance to the oligonucleotide. Examples of 2' substituted modified nucleosides are 2'-O-alkyl-RNA, 2'-O-methyl-RNA, 2'-alkoxy-RNA, 2'-O-methoxyethyl-RNA (MOE), 2'-amino-DNA, 2'-fluoro-RNA and 2'-F-ANA nucleoside. Further examples can be found in e.g. Freier & Altmann; Nucl. Acid Res., 1997, 25, 4429-4443 and Uhlmann; Curr. Opinion in Drug Development, 2000, 3(2), 293-213 and Deleavey and Damha, Chemistry and Biology 2012, 19, 937. Below are illustrations of some 2' substituted modified nucleosides.

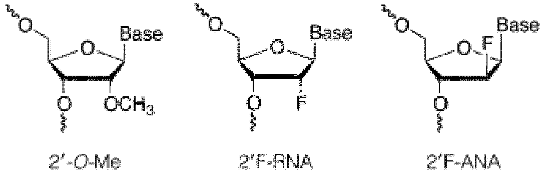

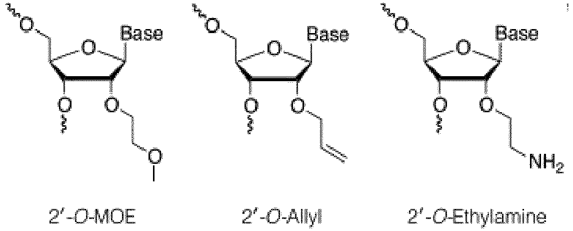

In relation to the present invention 2' substituted does not include 2' bridged molecules like LNA.

### Locked Nucleic Acid Nucleosides (LNA nucleosides)

A "LNA nucleoside" is a 2'-modified nucleoside which comprises a biradical linking the C2' and C4' of the ribose sugar ring of said nucleoside (also referred to as a "2'- 4' bridge"), which restricts or locks the conformation of the ribose ring. These nucleosides are also termed bridged nucleic acid or bicyclic nucleic acid (BNA) in the literature. The locking of the conformation of the ribose is associated with an enhanced affinity of hybridization (duplex stabilization) when the LNA is incorporated into an oligonucleotide for a complementary RNA or DNA molecule. This can be routinely determined by measuring the melting temperature of the oligonucleotide/complement duplex.

Non limiting, exemplary LNA nucleosides are disclosed in WO 99/014226, WO 00/66604, WO 98/039352 , WO 2004/046160, WO 00/047599, WO 2007/134181, WO 2010/077578, WO 2010/036698, WO 2007/090071, WO 2009/006478, WO 2011/156202, WO 2008/154401, WO 2009/067647, WO 2008/150729, Morita et al., Bioorganic & Med.Chem. Lett. 12, 73-76, Seth et al. J. Org. Chem. 2010, Vol 75(5) pp. 1569-81 and Mitsuoka et al., Nucleic Acids Research 2009, 37(4), 1225-1238.

The 2'-4' bridge comprises 2 to 4 bridging atoms and is in particular of formula -X-Y-, X being linked to C4' and Y linked to C2',
wherein
X is oxygen, sulfur, -CR^{a}R^{b}-, -C(R^{a})=C(R^{b})-, -C(=CR^{a}R^{b})-, -C(R^{a})=N-, -Si(R^{a})₂-, - SO₂-, -NR^{a}-; -O-NR^{a}-, -NR^{a}-O-, -C(=J)-, Se, -O-NR^{a}-, -NR^{a}-CR^{a}R^{b}-, -N(R^{a})-O- or -O-CR^{a}R^{b}-;
Y is oxygen, sulfur, -(CR^{a}R^{b})n-, -CR^{a}R^{b}-O-CR^{a}R^{b}-, -C(R^{a})=C(R^{b})-, -C(R^{a})=N-, - Si(R^{a})₂-, -SO₂-, -NR^{a}-, -C(=J)-, Se, -O-NR^{a}-, -NR^{a}-CR^{a}R^{b}-, -N(R^{a})-O- or -O-CR^{a}R^{b}-;
with the proviso that -X-Y- is not -O-O-, Si(R^{a})₂-Si(R^{a})₂-, -SO₂-SO₂-, -C(R^{a})=C(R^{b})-C(R^{a})=C(R^{b}), -C(R^{a})=N-C(R^{a})=N-, -C(R^{a})=N-C(R^{a})=C(R^{b}) , -C(R^{a})=C(R^{b})-C(R^{a})=N- or -Se-Se-;
J is oxygen, sulfur, =CH₂ or =N(R^{a});
R^{a} and R^{b} are independently selected from hydrogen, halogen, hydroxyl, cyano, thiohydroxyl, alkyl, substituted alkyl, alkenyl, substituted alkenyl, alkynyl, substituted alkynyl, alkoxy, substituted alkoxy, alkoxyalkyl, alkenyloxy, carboxyl, alkoxycarbonyl, alkylcarbonyl, formyl, aryl, heterocyclyl, amino, alkylamino, carbamoyl, alkylaminocarbonyl, aminoalkylaminocarbonyl, alkylaminoalkylaminocarbonyl, alkylcarbonylamino, carbamido, alkanoyloxy, sulfonyl, alkylsulfonyloxy, nitro, azido, thiohydroxylsulfidealkylsulfanyl, aryloxycarbonyl, aryloxy, arylcarbonyl, heteroaryl, heteroaryloxycarbonyl, heteroaryloxy, heteroarylcarbonyl, -OC(=X^{a})R^{c}, -OC(=X^{a})NR^{c}R^{d} and - NR^{e}C(=X^{a})NR^{c}R^{d};
or two geminal R^{a} and R^{b} together form optionally substituted methylene;
or two geminal R^{a} and R^{b}, together with the carbon atom to which they are attached, form cycloalkyl or halocycloalkyl, with only one carbon atom of -X-Y-;
wherein substituted alkyl, substituted alkenyl, substituted alkynyl, substituted alkoxy and substituted methylene are alkyl, alkenyl, alkynyl and methylene substituted with 1 to 3 substituents independently selected from halogen, hydroxyl, alkyl, alkenyl, alkynyl, alkoxy, alkoxyalkyl, alkenyloxy, carboxyl, alkoxycarbonyl, alkylcarbonyl, formyl, heterocylyl, aryl and heteroaryl;
X^{a} is oxygen, sulfur or -NR^{c};
R^{c}, R^{d} and R^{e} are independently selected from hydrogen and alkyl; and
n is 1, 2 or 3.

In a further particular embodiment of the invention, X is oxygen, sulfur, -NR^{a}-, - CR^{a}R^{b}- or -C(=CR^{a}R^{b})-, particularly oxygen, sulfur, -NH-, -CH₂- or -C(=CH₂)-, more particularly oxygen.

In another particular embodiment of the invention, Y is -CR^{a}R^{b}-, -CR^{a}R^{b}-CR^{a}R^{b}- or - CR^{a}R^{b} CR^{a}R^{b} CR^{a}R^{b}-, particularly -CH₂-CHCH₃-, -CHCH₃-CH₂-, -CH₂-CH₂- or -CH₂-CH₂-CH₂-.

In a particular embodiment of the invention, -X-Y- is -O-(CR^{a}R^{b})ₙ-, -S-CR^{a}R^{b}-, - N(R^{a})CR^{a}R^{b}-, -CR^{a}R^{b}-CR^{a}R^{b}-, -O-CR^{a}R^{b}-O-CR^{a}R^{b}-, -CR^{a}R^{b}-O-CR^{a}R^{b}-, -C(=CR^{a}R^{b})-CR^{a}R^{b}-, -N(R^{a})CR^{a}R^{b}-, -O-N(R^{a})-CR^{a}R^{b}- or -N(R^{a})-O-CR^{a}R^{b}-.

In a particular embodiment of the invention, R^{a} and R^{b} are independently selected from the group consisting of hydrogen, halogen, hydroxyl, alkyl and alkoxyalkyl, in particular hydrogen, halogen, alkyl and alkoxyalkyl.

In another embodiment of the invention, R^{a} and R^{b} are independently selected from the group consisting of hydrogen, fluoro, hydroxyl, methyl and -CH₂-O-CH₃, in particular hydrogen, fluoro, methyl and -CH₂-O-CH₃.

Advantageously, one of R^{a} and R^{b} of -X-Y- is as defined above and the other ones are all hydrogen at the same time.

In a further particular embodiment of the invention, R^{a} is hydrogen or alkyl, in particular hydrogen or methyl.

In another particular embodiment of the invention, R^{b} is hydrogen or or alkyl, in particular hydrogen or methyl.

In a particular embodiment of the invention, one or both of R^{a} and R^{b} are hydrogen.

In a particular embodiment of the invention, only one of R^{a} and R^{b} is hydrogen.

In one particular embodiment of the invention, one of R^{a} and R^{b} is methyl and the other one is hydrogen.

In a particular embodiment of the invention, R^{a} and R^{b} are both methyl at the same time.

In a particular embodiment of the invention, -X-Y- is -O-CH₂-, -S-CH₂-, -S-CH(CH₃)-, -NH-CH₂-, -O-CH₂CH₂-, -O-CH(CH₂-O-CH₃)-, -O-CH(CH₂CH₃)-, -O-CH(CH₃)-, -O-CH₂₋O-CH₂-, -O-CH₂-O-CH₂-, -CH₂-O-CH₂-, -C(=CH₂)CH₂-, - C(=CH₂)CH(CH₃)-, -N(OCH₃)CH₂- or -N(CH₃)CH₂-;

In a particular embodiment of the invention, -X-Y- is -O-CR^{a}R^{b}- wherein R^{a} and R^{b} are independently selected from the group consisting of hydrogen, alkyl and alkoxyalkyl, in particular hydrogen, methyl and -CH₂-O-CH₃.

In a particular embodiment, -X-Y- is -O-CH₂- or -O-CH(CH₃)-, particularly -O-CH₂-

The 2'- 4' bridge may be positioned either below the plane of the ribose ring (beta-D- configuration), or above the plane of the ring (alpha-L- configuration), as illustrated in formula (A) and formula (B) respectively.

The LNA nucleoside according to the invention is in particular of formula (B 1) or (B2) 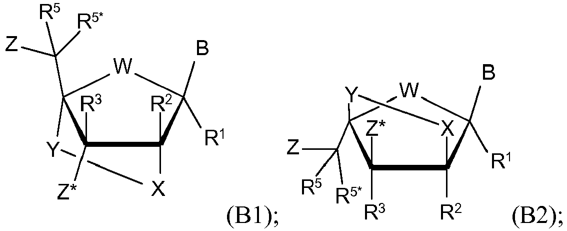 wherein
W is oxygen, sulfur, -N(R^{a})- or -CR^{a}R^{b}-, in particular oxygen;
B is a nucleobase or a modified nucleobase;
Z is an internucleoside linkage to an adjacent nucleoside or a 5'-terminal group;
Z* is an internucleoside linkage to an adjacent nucleoside or a 3'-terminal group;
R¹, R², R³, R⁵ and R⁵* are independently selected from hydrogen, halogen, alkyl, haloalkyl, alkenyl, alkynyl, hydroxy, alkoxy, alkoxyalkyl, azido, alkenyloxy, carboxyl, alkoxycarbonyl, alkylcarbonyl, formyl and aryl; and
X, Y, R^{a} and R^{b} are as defined above.

In a particuliar embodiment, in the definition of -X-Y-, R^{a} is hydrogen or alkyl, in particular hydrogen or methyl. In another particular embodiment, in the definition of -X-Y-, R^{b} is hydrogen or alkyl, in particular hydrogen or methyl. In a further particular embodiment, in the definition of -X-Y-, one or both of R^{a} and R^{b} are hydrogen. In a particular embodiment, in the definition of -X-Y-, only one of R^{a} and R^{b} is hydrogen. In one particular embodiment, in the definition of -X-Y-, one of R^{a} and R^{b} is methyl and the other one is hydrogen. In a particular embodiment, in the definition of -X-Y-, R^{a} and R^{b} are both methyl at the same time.

In a further particuliar embodiment, in the definition of X, R^{a} is hydrogen or alkyl, in particular hydrogen or methyl. In another particular embodiment, in the definition of X, R^{b} is hydrogen or alkyl, in particular hydrogen or methyl. In a particular embodiment, in the definition of X, one or both of R^{a} and R^{b} are hydrogen. In a particular embodiment, in the definition of X, only one of R^{a} and R^{b} is hydrogen. In one particular embodiment, in the definition of X, one of R^{a} and R^{b} is methyl and the other one is hydrogen. In a particular embodiment, in the definition of X, R^{a} and R^{b} are both methyl at the same time.

In a further particuliar embodiment, in the definition of Y, R^{a} is hydrogen or alkyl, in particular hydrogen or methyl. In another particular embodiment, in the definition of Y, R^{b} is hydrogen or alkyl, in particular hydrogen or methyl. In a particular embodiment, in the definition of Y, one or both of R^{a} and R^{b} are hydrogen. In a particular embodiment, in the definition of Y, only one of R^{a} and R^{b} is hydrogen. In one particular embodiment, in the definition of Y, one of R^{a} and R^{b} is methyl and the other one is hydrogen. In a particular embodiment, in the definition of Y, R^{a} and R^{b} are both methyl at the same time.

In a particular embodiment of the invention R¹, R², R³, R⁵ and R⁵* are independently selected from hydrogen and alkyl, in particular hydrogen and methyl.

In a further particular advantageous embodiment of the invention, R¹, R², R³, R⁵ and R⁵* are all hydrogen at the same time.

In another particular embodiment of the invention, R¹, R², R³, are all hydrogen at the same time, one of R⁵ and R⁵* is hydrogen and the other one is as defined above, in particular alkyl, more particularly methyl.

In a particular embodiment of the invention, R⁵ and R⁵* are independently selected from hydrogen, halogen, alkyl, alkoxyalkyl and azido, in particular from hydrogen, fluoro, methyl, methoxyethyl and azido. In particular advantageous embodiments of the invention, one of R⁵ and R⁵* is hydrogen and the other one is alkyl, in particular methyl, halogen, in particular fluoro, alkoxyalkyl, in particular methoxyethyl or azido; or R⁵ and R⁵* are both hydrogen or halogen at the same time, in particular both hydrogen of fluoro at the same time. In such particular embodiments, W can advantageously be oxygen, and -X-Y-advantageously -O-CH₂-.

In a particular embodiment of the invention, -X-Y- is -O-CH₂-, W is oxygen and R¹, R², R³, R⁵ and R⁵* are all hydrogen at the same time. Such LNA nucleosides are disclosed in WO 99/014226, WO 00/66604, WO 98/039352 and WO 2004/046160 which
include what are commonly known in the art as beta-D-oxy LNA and alpha-L-oxy LNA nucleosides.

In another particular embodiment of the invention, -X-Y- is -S-CH₂-, W is oxygen and R¹, R², R³, R⁵ and R⁵* are all hydrogen at the same time. Such thio LNA nucleosides are disclosed in WO 99/014226 and WO 2004/046160

In another particular embodiment of the invention, -X-Y- is -NH-CH₂-, W is oxygen and R¹, R², R³, R⁵ and R⁵* are all hydrogen at the same time. Such amino LNA nucleosides are disclosed in WO 99/014226 and WO 2004/046160

In another particular embodiment of the invention, -X-Y- is -O-CH₂CH₂- or - OCH₂CH₂CH₂-, W is oxygen, and R¹, R², R³, R⁵ and R⁵* are all hydrogen at the same time. Such LNA nucleosides are disclosed in WO 00/047599 and Morita et al., Bioorganic & Med.Chem. Lett. 12, 73-76, which include what are commonly known in the art as 2'-O-4'C-ethylene bridged nucleic acids (ENA).

In another particular embodiment of the invention, -X-Y- is -O-CH₂-, W is oxygen, R¹, R², R³ are all hydrogen at the same time, one of R⁵ and R⁵* is hydrogen and the other one is not hydrogen, such as alkyl, for example methyl. Such 5' substituted LNA nucleosides are disclosed in WO 2007/134181 .

In another particular embodiment of the invention, -X-Y- is -O-CR^{a}R^{b}-, wherein one or both of R^{a} and R^{b} are not hydrogen, in particular alkyl such as methyl, W is oxygen, R¹, R², R³ are all hydrogen at the same time, one of R⁵ and R⁵* is hydrogen and the other one is not hydrogen, in particular alkyl, for example methyl. Such bis modified LNA nucleosides are disclosed in WO 2010/077578 .

In another particular embodiment of the invention, -X-Y- is -O-CHR^{a}-, W is oxygen and R¹, R², R³, R⁵ and R⁵* are all hydrogen at the same time. Such 6'-substituted LNA nucleosides are disclosed in WO 2010/036698 and WO 2007/090071.

In such 6'-substituted LNA nucleosides, R^{a} is in particular C₁-C₆ alkyl, such as methyl.

In another particular embodiment of the invention, -X-Y- is -O-CH(CH₂-O-CH₃)-("2' O-methoxyethyl bicyclic nucleic acid", Seth et al. J. Org. Chem. 2010, Vol 75(5) pp. 1569-81).

In another particular embodiment of the invention, -X-Y- is -O-CH(CH₂CH₃)-;

In another particular embodiment of the invention, -X-Y- is -O-CH(CH₂-O-CH₃)-, W is oxygen and R¹, R², R³, R⁵ and R⁵* are all hydrogen at the same time. Such LNA nucleosides are also known in the art as cyclic MOEs (cMOE) and are disclosed in WO 2007/090071.

In another particular embodiment of the invention, -X-Y- is -O-CH(CH₃)- ("2'O-ethyl bicyclic nucleic acid", Seth at al., J. Org. Chem. 2010, Vol 75(5) pp. 1569-81).

In another particular embodiment of the invention, -X-Y- is -O-CH₂₋O-CH₂- (Seth et al., J. Org. Chem 2010 op. cit.)

In another particular embodiment of the invention, -X-Y- is -O-CH(CH₃)-, W is oxygen and R¹, R², R³, R⁵ and R⁵* are all hydrogen at the same time. Such 6'-methyl LNA nucleosides are also known in the art as cET nucleosides, and may be either (S)-cET or (R)-cET diastereoisomers, as disclosed in WO 2007/090071 (beta-D) and WO 2010/036698 (alpha-L) .

In another particular embodiment of the invention, -X-Y- is -O-CR^{a}R^{b}-, wherein neither R^{a} nor R^{b} is hydrogen, W is oxygen and R¹, R², R³, R⁵ and R⁵* are all hydrogen at the same time. In a particular embodiment, R^{a} and R^{b} are both alkyl at the same time, in particular both methyl at the same time. Such 6'-di-substituted LNA nucleosides are disclosed in WO 2009/006478 .

In another particualr embodiment of the invention, -X-Y- is -S-CHR^{a}-, W is oxygen and R¹, R², R³, R⁵ and R⁵* are all hydrogen at the same time. Such 6'-substituted thio LNA nucleosides are disclosed in WO 2011/156202 . In a particular embodiment of such 6'-substituted thio LNA, R^{a} is alkyl, in particular methyl.

In a particular embodiment of the invention, -X-Y- is -C(=CH₂)C(R^{a}R^{b})-, - C(=CHF)C(R^{a}R^{b})- or -C(=CE₂)C(R^{a}R^{b})-, W is oxygen and R¹, R², R³, R⁵ and R⁵* are all hydrogen at the same time. R^{a} and R^{b} are advantagesously independently selected from hydrogen, halogen, alkyl and alkoxyalkyl, in particular hydrogen, methyl, fluoro and methoxymethyl. R^{a} and R^{b} are in particular both hydrogen or methyl at the same time or one of R^{a} and R^{b} is hydrogen and the other one is methyl. Such vinyl carbo LNA nucleosides are disclosed in WO 2008/154401 and WO 2009/067647

In a particular embodiment of the invention, -X-Y- is -N(OR^{a})-CH₂-, W is oxygen and R¹, R², R³, R⁵ and R⁵* are all hydrogen at the same time. In a particular embodiment, R^{a} is alkyl such as methyl. Such LNA nucleosides are also known as N substituted LNAs and are disclosed in WO 2008/150729 .

In a particular embodiment of the invention, -X-Y- is -O-N(R^{a})-, -N(R^{a})-O-, -NR^{a}-CR^{a}R^{b}-CR^{a}R^{b}- or -NR^{a}-CR^{a}R^{b}-, W is oxygen and R¹, R², R³, R⁵ and R⁵* are all hydrogen at the same time. R^{a} and R^{b} are advantagesously independently selected from hydrogen, halogen, alkyl and alkoxyalkyl, in particular hydrogen, methyl, fluoro and methoxymethyl. In a particular embodiment, R^{a} is alkyl, such as methyl, R^{b} is hydrogen or methyl, in particular hydrogen. (Seth et al., J. Org. Chem 2010 op. cit.).

In a particular embodiment of the invention, -X-Y- is -O-N(CH₃)- (Seth et al., J. Org. Chem 2010 op. cit.).

In a particular embodiment of the invention, R⁵ and R⁵* are both hydrogen at the same time. In another particular embodiment of the invention, one of R⁵ and R⁵* is hydrogen and the other one is alkyl, such as methyl. In such embodiments, R¹, R² and R³ can be in particular hydrogen and -X-Y- can be in particular -O-CH₂- or -O-CHC(R^{a})₃-, such as -O-CH(CH₃)-.

In a particular embodiment of the invention, -X-Y- is -CR^{a}R^{b}-O-CR^{a}R^{b}-, such as - CH₂-O-CH₂-, W is oxygen and R¹, R², R³, R⁵ and R⁵* are all hydrogen at the same time. In such particular embodiments, R^{a} can be in particular alkyl such as methyl, R^{b} hydrogen or methyl, in particular hydrogen. Such LNA nucleosides are also known as conformationally restricted nucleotides (CRNs) and are disclosed in WO 2013/036868

In a particular embodiment of the invention, -X-Y- is -O-CR^{a}R^{b}-O-CR^{a}R^{b}-, such as - O-CH₂-O-CH₂-, W is oxygen and R¹, R², R³, R⁵ and R⁵* are all hydrogen at the same time. R^{a} and R^{b} are advantagesously independently selected from hydrogen, halogen, alkyl and alkoxyalkyl, in particular hydrogen, methyl, fluoro and methoxymethyl. In such a particular embodiment, R^{a} can be in particular alkyl such as methyl, R^{b} hydrogen or methyl, in particular hydrogen. Such LNA nucleosides are also known as COC nucleotides and are disclosed in Mitsuoka et al., Nucleic Acids Research 2009, 37(4), 1225-1238,

It will be recognized than, unless specified, the LNA nucleosides may be in the beta-D or alpha-L stereoisoform.

Particular examples of LNA nucleosides of the invention are presented in Scheme 1 (wherein B is as defined above).

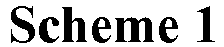

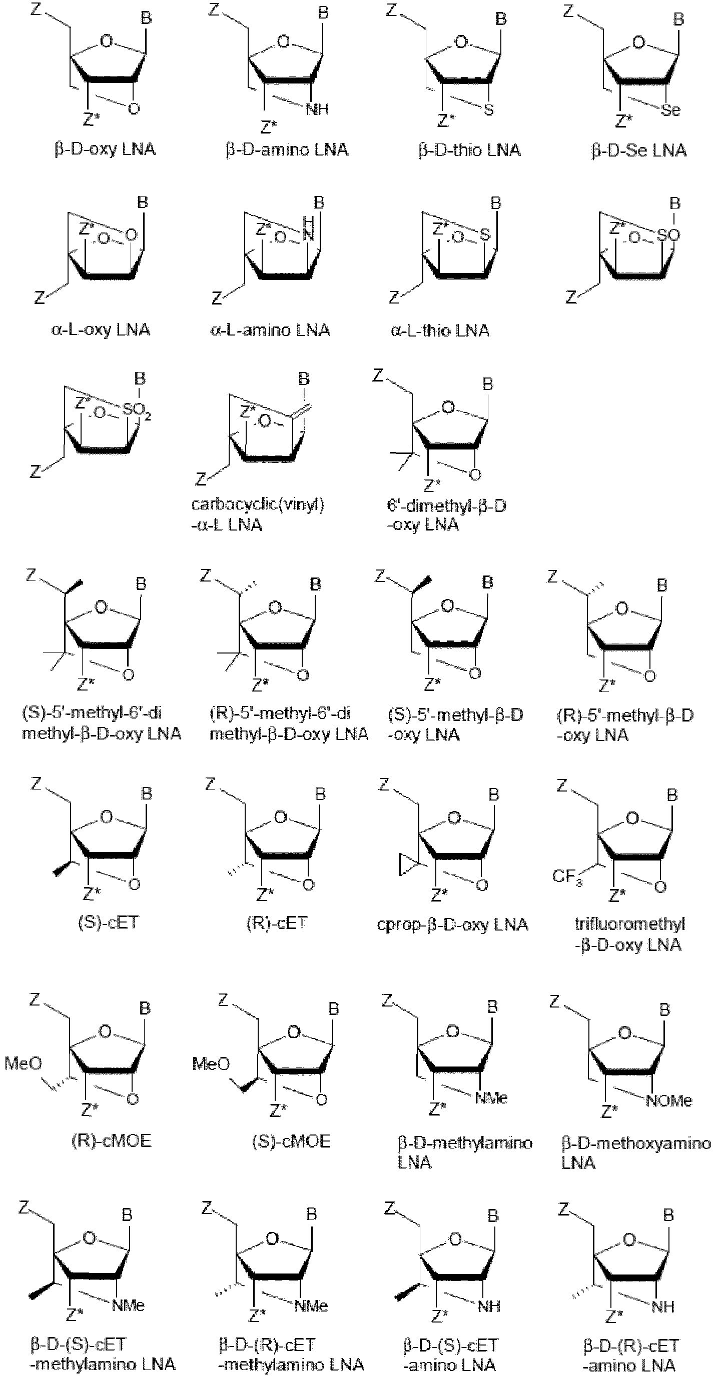

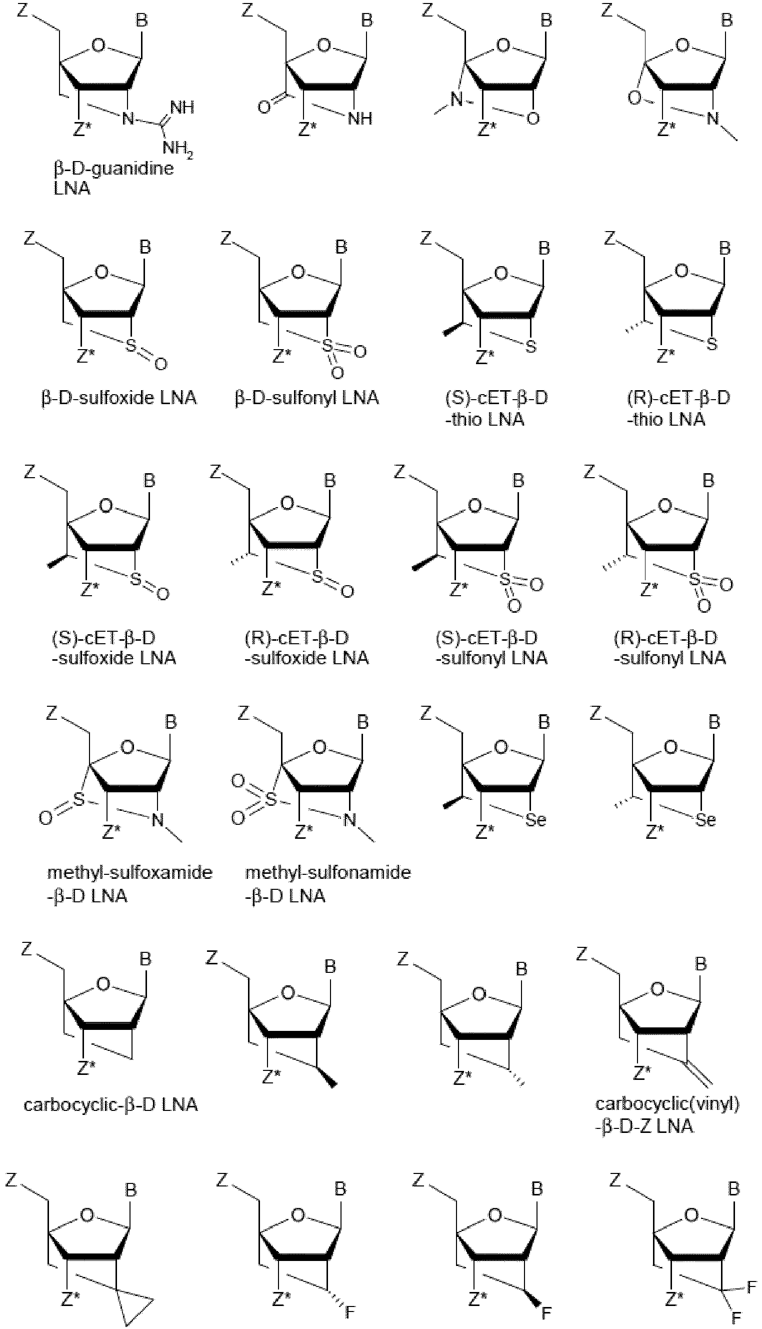

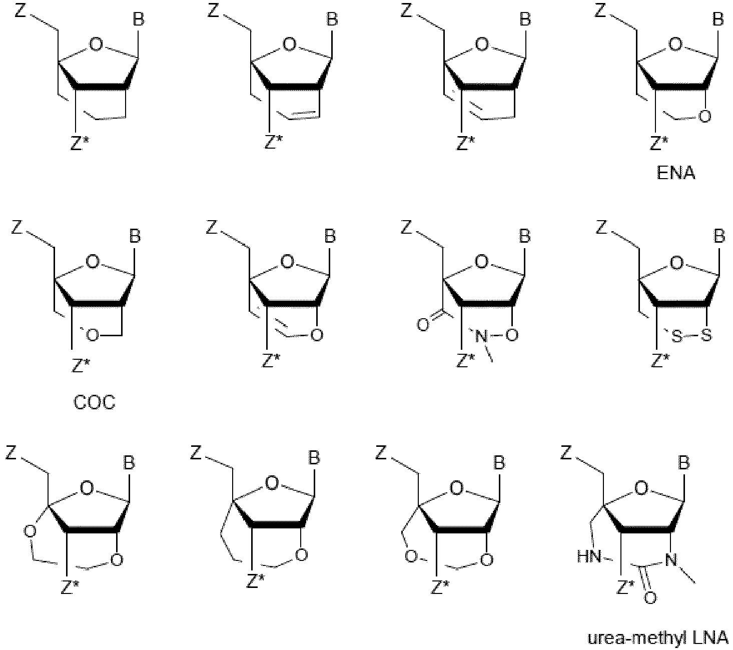

Particular LNA nucleosides are beta-D-oxy-LNA, 6'-methyl-beta-D-oxy LNA such as (S)-6'-methyl-beta-D-oxy-LNA ((S)-cET) and ENA.

### MOE nucleoside

The term "MOE" stands for "methoxy-ethyl" and refers by means of abbreviation to a nucleoside substituted in 2' position with a methoxy-ethoxy group as represented below.

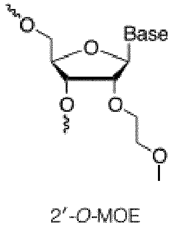

The above nucleoside can thus be named either "MOE" or "2'-O-MOE nucleoside".

### RNase H Activity and Recruitment

The RNase H activity of an antisense oligonucleotide refers to its ability to recruit RNase H when in a duplex with a complementary RNA molecule. WO01/23613 provides *in vitro* methods for determining RNaseH activity, which may be used to determine the ability to recruit RNaseH. Typically an oligonucleotide is deemed capable of recruiting RNase H if it, when provided with a complementary target nucleic acid sequence, has an initial rate, as measured in pmol/l/min, of at least 5%, such as at least 10% or more than 20% of the of the initial rate determined when using a oligonucleotide having the same base sequence as the modified oligonucleotide being tested, but containing only DNA monomers with phosphorothioate linkages between all monomers in the oligonucleotide, and using the methodology provided by Example 91 - 95 of WO01/23613.

For use in determining RHase H activity, recombinant human RNase H1 is available from Lubio Science GmbH, Lucerne, Switzerland.

### Gapmer

The antisense oligonucleotide of the invention, or contiguous nucleotide sequence thereof may be a gapmer. The antisense gapmers are commonly used to inhibit a target nucleic acid via RNase H mediated degradation. A gapmer oligonucleotide comprises at least three distinct structural regions a 5'-flank, a gap and a 3'-flank, F-G-F' in the '5 -> 3' orientation. The "gap" region (G) comprises a stretch of contiguous DNA nucleotides which enable the oligonucleotide to recruit RNase H. The gap region is flanked by a 5' flanking region (F) comprising one or more sugar modified nucleosides, advantageously high affinity sugar modified nucleosides, and by a 3' flanking region (F') comprising one or more sugar modified nucleosides, advantageously high affinity sugar modified nucleosides. The one or more sugar modified nucleosides in region F and F' enhance the affinity of the oligonucleotide for the target nucleic acid (*i.e.* are affinity enhancing sugar modified nucleosides). In some embodiments, the one or more sugar modified nucleosides in region F and F' are 2' sugar modified nucleosides, such as high affinity 2' sugar modifications, such as independently selected from LNA and 2'-MOE.

In a gapmer design, the 5' and 3' most nucleosides of the gap region are DNA nucleosides, and are positioned adjacent to a sugar modified nucleoside of the 5' (F) or 3' (F') region respectively. The flanks may further defined by having at least one sugar modified nucleoside at the end most distant from the gap region, i.e. at the 5' end of the 5' flank and at the 3' end of the 3' flank.

Regions F-G-F' form a contiguous nucleotide sequence. Antisense oligonucleotides of the invention, or the contiguous nucleotide sequence thereof, may comprise a gapmer region of formula F-G-F'.

The overall length of the gapmer design F-G-F' may be, for example 12 to 32 nucleosides, such as 13 to 24, such as 14 to 22 nucleosides, Such as from 14 to 17, such as 16 to 18 nucleosides.

By way of example, the gapmer oligonucleotide of the present invention can be represented by the following formulae:
F₁₋₈-G₅₋₁₆-F'₁₋₈, such as
F₁₋₈-G₇₋₁₆-F'₂₋₈
with the proviso that the overall length of the gapmer regions F-G-F' is at least 12, such as at least 14 nucleotides in length.

Regions F, G and F' are further defined below and can be incorporated into the F-G-F' formula.

### Gapmer - Region G

Region G (gap region) of the gapmer is a region of nucleosides which enables the oligonucleotide to recruit RNaseH, such as human RNase H1, typically DNA nucleosides. RNaseH is a cellular enzyme which recognizes the duplex between DNA and RNA, and enzymatically cleaves the RNA molecule. Suitable gapmers may have a gap region (G) of at least 5 or 6 contiguous DNA nucleosides, such as 5 - 16 contiguous DNA nucleosides, such as 6 - 15 contiguous DNA nucleosides, such as 7-14 contiguous DNA nucleosides, such as 8 - 12 contiguous DNA nucleotides, such as 8 - 12 contiguous DNA nucleotides in length. The gap region G may, in some embodiments consist of 6, 7, 8, 9, 10, 11, 12, 13, 14, 15 or 16 contiguous DNA nucleosides. Cytosine (C) DNA in the gap region may in some instances be methylated, such residues are either annotated as 5-methyl-cytosine (^{me}C or with an e instead of a c). Methylation of Cytosine DNA in the gap is advantageous if cg dinucleotides are present in the gap to reduce potential toxicity, the modification does not have significant impact on efficacy of the oligonucleotides..

In some embodiments the gap region G may consist of 6, 7, 8, 9, 10, 11, 12, 13, 14, 15 or 16 contiguous phosphorothioate linked DNA nucleosides. In some embodiments, all internucleoside linkages in the gap are phosphorothioate linkages.

Whilst traditional gapmers have a DNA gap region, there are numerous examples of modified nucleosides which allow for RNaseH recruitment when they are used within the gap region. Modified nucleosides which have been reported as being capable of recruiting RNaseH when included within a gap region include, for example, alpha-L-LNA, C4' alkylated DNA (as described in PCT/EP2009/050349 and Vester et al., Bioorg. Med. Chem. Lett. 18 (2008) 2296 - 2300 , arabinose derived nucleosides like ANA and 2'F-ANA (Mangos et al. 2003 J. AM. CHEM. SOC. 125, 654-661), UNA (unlocked nucleic acid) (as described in Fluiter et al., Mol. Biosyst., 2009, 10, 1039 . UNA is unlocked nucleic acid, typically where the bond between C2 and C3 of the ribose has been removed, forming an unlocked "sugar" residue. The modified nucleosides used in such gapmers may be nucleosides which adopt a 2' endo (DNA like) structure when introduced into the gap region, *i.e.* modifications which allow for RNaseH recruitment). In some embodiments the DNA Gap region (G) described herein may optionally contain 1 to 3 sugar modified nucleosides which adopt a 2' endo (DNA like) structure when introduced into the gap region.

### Region G - "Gap-breaker"

Alternatively, there are numerous reports of the insertion of a modified nucleoside which confers a 3' endo conformation into the gap region of gapmers, whilst retaining some RNaseH activity. Such gapmers with a gap region comprising one or more 3'endo modified nucleosides are referred to as "gap-breaker" or "gap-disrupted" gapmers, see for example WO2013/022984. Gap-breaker oligonucleotides retain sufficient region of DNA nucleosides within the gap region to allow for RNaseH recruitment. The ability of gapbreaker oligonucleotide design to recruit RNaseH is typically sequence or even compound specific - see Rukov et al. 2015 Nucl. Acids Res. Vol. 43 pp. 8476-8487, which discloses "gapbreaker" oligonucleotides which recruit RNaseH which in some instances provide a more specific cleavage of the target RNA. Modified nucleosides used within the gap region of gap-breaker oligonucleotides may for example be modified nucleosides which confer a 3'endo confirmation, such 2' -O-methyl (OMe) or 2'-O-MOE (MOE) nucleosides, or beta-D LNA nucleosides (the bridge between C2' and C4' of the ribose sugar ring of a nucleoside is in the beta conformation), such as beta-D-oxy LNA or ScET nucleosides.

As with gapmers containing region G described above, the gap region of gap-breaker or gap-disrupted gapmers, have a DNA nucleosides at the 5' end of the gap (adjacent to the 3' nucleoside of region F), and a DNA nucleoside at the 3' end of the gap (adjacent to the 5' nucleoside of region F'). Gapmers which comprise a disrupted gap typically retain a region of at least 3 or 4 contiguous DNA nucleosides at either the 5' end or 3' end of the gap region.

Exemplary designs for gap-breaker oligonucleotides include
F₁₋₈-[D₃₋₄-E1- D ₃₋₄]-F'₁₋₈
F₁₋₈- [D ₁₋₄-E₁- D ₃₋₄]-F'₁₋₈
F₁₋₈- [D ₃₋₄-E₁- D ₁₋₄]-F'₁₋₈
wherein region G is within the brackets [Dₙ-Eᵣ- Dₘ], D is a contiguous sequence of DNA nucleosides, E is a modified nucleoside (the gap-breaker or gap-disrupting nucleoside), and F and F' are the flanking regions as defined herein, and with the proviso that the overall length of the gapmer regions F-G-F' is at least 12, such as at least 14 nucleotides in length.

In some embodiments, region G of a gap disrupted gapmer comprises at least 6 DNA nucleosides, such as 6, 7, 8, 9, 10, 11, 12, 13, 14, 15 or 16 DNA nucleosides. As described above, the DNA nucleosides may be contiguous or may optionally be interspersed with one or more modified nucleosides, with the proviso that the gap region G is capable of mediating RNaseH recruitment.

### Gapmer - flanking regions, F and F'

Region F is positioned immediately adjacent to the 5' DNA nucleoside of region G. The 3' most nucleoside of region F is a sugar modified nucleoside, such as a high affinity sugar modified nucleoside, for example a 2' substituted nucleoside, such as a MOE nucleoside, or an LNA nucleoside.

Region F' is positioned immediately adjacent to the 3' DNA nucleoside of region G. The 5' most nucleoside of region F' is a sugar modified nucleoside, such as a high affinity sugar modified nucleoside, for example a 2' substituted nucleoside, such as a MOE nucleoside, or an LNA nucleoside.

Region F is 1-8 contiguous nucleotides in length, such as 2-6, such as 3-4 contiguous nucleotides in length. Advantageously the 5' most nucleoside of region F is a sugar modified nucleoside. In some embodiments the two 5' most nucleoside of region F are sugar modified nucleoside. In some embodiments the 5' most nucleoside of region F is an LNA nucleoside. In some embodiments the two 5' most nucleoside of region F are LNA nucleosides. In some embodiments the two 5' most nucleoside of region F are 2' substituted nucleoside nucleosides, such as two 3' MOE nucleosides. In some embodiments the 5' most nucleoside of region F is a 2' substituted nucleoside, such as a MOE nucleoside.

Region F' is 2-8 contiguous nucleotides in length, such as 3-6, such as 4-5 contiguous nucleotides in length. Advantageously, embodiments the 3' most nucleoside of region F' is a sugar modified nucleoside. In some embodiments the two 3' most nucleoside of region F' are sugar modified nucleoside. In some embodiments the two 3' most nucleoside of region F' are LNA nucleosides. In some embodiments the 3' most nucleoside of region F' is an LNA nucleoside. In some embodiments the two 3' most nucleoside of region F' are 2' substituted nucleoside nucleosides, such as two 3' MOE nucleosides. In some embodiments the 3' most nucleoside of region F' is a 2' substituted nucleoside, such as a MOE nucleoside.

It should be noted that when the length of region F or F' is one, it is advantageously an LNA nucleoside.

In some embodiments, region F and F' independently consists of or comprises a contiguous sequence of sugar modified nucleosides. In some embodiments, the sugar modified nucleosides of region F may be independently selected from 2'-O-alkyl-RNA units, 2'-O-methyl-RNA, 2'-amino-DNA units, 2'-fluoro-DNA units, 2'-alkoxy-RNA, MOE units, LNA units, arabino nucleic acid (ANA) units and 2'-fluoro-ANA units.

In some embodiments, region F and F' independently comprises both LNA and a 2' substituted modified nucleosides (mixed wing design).

In some embodiments, region F and F' consists of only one type of sugar modified nucleosides, such as only MOE or only beta-D-oxy LNA or only ScET. Such designs are also termed uniform flanks or uniform gapmer design.

In some embodiments, all the nucleosides of region F or F', or F and F' are LNA nucleosides, such as independently selected from beta-D-oxy LNA, ENA or ScET nucleosides. In some embodiments region F consists of 1-5, such as 2-4, such as 3-4 such as 1, 2, 3, 4 or 5 contiguous LNA nucleosides. In some embodiments, all the nucleosides of region F and F' are beta-D-oxy LNA nucleosides.

In some embodiments, all the nucleosides of region F or F', or F and F' are 2' substituted nucleosides, such as OMe or MOE nucleosides. In some embodiments region F consists of 1, 2, 3, 4, 5, 6, 7, or 8 contiguous OMe or MOE nucleosides. In some embodiments only one of the flanking regions can consist of 2' substituted nucleosides, such as OMe or MOE nucleosides. In some embodiments it is the 5' (F) flanking region that consists 2' substituted nucleosides, such as OMe or MOE nucleosides whereas the 3' (F') flanking region comprises at least one LNA nucleoside, such as beta-D-oxy LNA nucleosides or cET nucleosides. In some embodiments it is the 3' (F') flanking region that consists 2' substituted nucleosides, such as OMe or MOE nucleosides whereas the 5' (F) flanking region comprises at least one LNA nucleoside, such as beta-D-oxy LNA nucleosides or cET nucleosides.

In some embodiments, all the modified nucleosides of region F and F' are LNA nucleosides, such as independently selected from beta-D-oxy LNA, ENA or ScET nucleosides, wherein region For F', or F and F' may optionally comprise DNA nucleosides (an alternating flank, see definition of these for more details). In some embodiments, all the modified nucleosides of region F and F' are beta-D-oxy LNA nucleosides, wherein region F or F', or F and F' may optionally comprise DNA nucleosides (an alternating flank, see definition of these for more details).

In some embodiments the 5' most and the 3' most nucleosides of region F and F' are LNA nucleosides, such as beta-D-oxy LNA nucleosides or ScET nucleosides.

In some embodiments, the internucleoside linkage between region F and region G is a phosphorothioate internucleoside linkage. In some embodiments, the internucleoside linkage between region F' and region G is a phosphorothioate internucleoside linkage. In some embodiments, the internucleoside linkages between the nucleosides of region F or F', F and F' are phosphorothioate internucleoside linkages.

Further gapmer designs are disclosed in WO 2004/046160, WO 2007/146511 and WO 2008/113832 .

### LNA Gapmer

An LNA gapmer is a gapmer wherein either one or both of region F and F' comprises or consists of LNA nucleosides. A beta-D-oxy gapmer is a gapmer wherein either one or both of region F and F' comprises or consists of beta-D-oxy LNA nucleosides.

In some embodiments the LNA gapmer is of formula: [LNA]i-s-[region G] -[LNA]₁₋₅, wherein region G is as defined in the Gapmer region G definition.

### MOE Gapmers

A MOE gapmers is a gapmer wherein regions F and F' consist of MOE nucleosides. In some embodiments the MOE gapmer is of design [MOB]₁₋₈-[Region G]-[MOE] ₁₋₈, such as [MOE]₂₋₇-[Region G]₅₋₁₆-[MOE] ₂₋₇, such as [MOE]₃₋₆-[Region G]-[MOE] ₃₋₆, wherein region G is as defined in the Gapmer definition. MOE gapmers with a 5-10-5 design (MOE-DNA-MOE) have been widely used in the art.

### Mixed Wing Gapmer

A mixed wing gapmer is an LNA gapmer wherein one or both of region F and F' comprise a 2' substituted nucleoside, such as a 2' substituted nucleoside independently selected from the group consisting of 2'-O-alkyl-RNA units, 2'-O-methyl-RNA, 2'-amino-DNA units, 2'-fluoro-DNA units, 2'-alkoxy-RNA, MOE units, arabino nucleic acid (ANA) units and 2'-fluoro-ANA units, such as a MOE nucleosides. In some embodiments wherein at least one of region F and F', or both region F and F' comprise at least one LNA nucleoside, the remaining nucleosides of region F and F' are independently selected from the group consisting of MOE and LNA. In some embodiments wherein at least one of region F and F', or both region F and F' comprise at least two LNA nucleosides, the remaining nucleosides of region F and F' are independently selected from the group consisting of MOE and LNA. In some mixed wing embodiments, one or both of region F and F' may further comprise one or more DNA nucleosides.

Mixed wing gapmer designs are disclosed in WO 2008/049085 and WO 2012/109395 .

### Alternating Flank Gapmers

Flanking regions may comprise both LNA and DNA nucleoside and are referred to as "alternating flanks" as they comprise an alternating motif of LNA-DNA-LNA nucleosides. Gapmers comprising such alternating flanks are referred to as "alternating flank gapmers". "Alternative flank gapmers" are thus LNA gapmer oligonucleotides where at least one of the flanks (F or F') comprises DNA in addition to the LNA nucleoside(s). In some embodiments at least one of region F or F', or both region F and F', comprise both LNA nucleosides and DNA nucleosides. In such embodiments, the flanking region F or F', or both F and F' comprise at least three nucleosides, wherein the 5' and 3' most nucleosides of the F and/or F' region are LNA nucleosides.

Alternating flank LNA gapmers are disclosed in WO 2016/127002.

An alternating flank region may comprise up to 3 contiguous DNA nucleosides, such as 1 to 2 or 1 or 2 or 3 contiguous DNA nucleosides.

The alternating flak can be annotated as a series of integers, representing a number of LNA nucleosides (L) followed by a number of DNA nucleosides (D), for example
[L]₁₋₃-[D]₁₋₄-[L]₁₋₃
[L]₁₋₂-[D]₁₋₂-[L]₁₋₂-[D]₁₋₂-[L]₁₋₂

In oligonucleotide designs these will often be represented as numbers such that 2-2-1 represents 5' [L]₂-[D]₂-[L] 3', and 1-1-1-1-1 represents 5' [L]-[D]-[L]-[D]-[L] 3'. The length of the flank (region F and F') in oligonucleotides with alternating flanks may independently be 3 to 10 nucleosides, such as 4 to 8, such as 5 to 6 nucleosides, such as 4, 5, 6 or 7 modified nucleosides. In some embodiments only one of the flanks in the gapmer oligonucleotide is alternating while the other is constituted of LNA nucleotides. It may be advantageous to have at least two LNA nucleosides at the 3' end of the 3' flank (F'), to confer additional exonuclease resistance. Some examples of oligonucleotides with alternating flanks are:
[L]₁₋₅-[D]₁₋₄-[L]₁₋₃-[G]₅₋₁₆-[L]₂₋₆
[L]₁₋₂-[D]₁₋₂-[L]₁₋₂-[D]₁₋₂-[L]₁₋₂-[G]₅₋₁₆-[L]₁₋₂-[D]₁₋₃-[L]₂₋₄
[L]₁₋₅-[G]₅₋₁₆-[L]-[D]-[L]-[D]-[L]₂
with the proviso that the overall length of the gapmer is at least 12, such as at least 14 nucleotides in length.

### Region D' or D" in an oligonucleotide

The oligonucleotide of the invention may in some embodiments comprise or consist of the contiguous nucleotide sequence of the oligonucleotide which is complementary to the target nucleic acid, such as the gapmer F-G-F', and further 5' and/or 3' nucleosides. The further 5' and/or 3' nucleosides may or may not be fully complementary to the target nucleic acid. Such further 5' and/or 3' nucleosides may be referred to as region D' and D" herein.

The addition of region D' or D" may be used for the purpose of joining the contiguous nucleotide sequence, such as the gapmer, to a conjugate moiety or another functional group. When used for joining the contiguous nucleotide sequence with a conjugate moiety is can serve as a biocleavable linker. Alternatively it may be used to provide exonucleoase protection or for ease of synthesis or manufacture.

Region D' and D" can be attached to the 5' end of region F or the 3' end of region F', respectively to generate designs of the following formulas D'-F-G-F', F-G-F'-D" or
D'-F-G-F'-D". In this instance the F-G-F' is the gapmer portion of the oligonucleotide and region D' or D" constitute a separate part of the oligonucleotide.

Region D' or D" may independently comprise or consist of 1, 2, 3, 4 or 5 additional nucleotides, which may be complementary or non-complementary to the target nucleic acid. The nucleotide adjacent to the F or F' region is not a sugar-modified nucleotide, such as a DNA or RNA or base modified versions of these. The D' or D' region may serve as a nuclease susceptible biocleavable linker (see definition of linkers). In some embodiments the additional 5' and/or 3' end nucleotides
are DNA or RNA. Nucleotide based biocleavable linkers suitable for use as region D' or D" are disclosed in WO 2014/076195, which include by way of example a phosphodiester linked DNA dinucleotide. The use of biocleavable linkers in poly-oligonucleotide constructs is disclosed in WO 2015/113922, where they are used to link multiple antisense constructs (e.g. gapmer regions) within a single oligonucleotide.

In one embodiment the oligonucleotide of the invention comprises a region D' and/or D" in addition to the contiguous nucleotide sequence which constitutes the gapmer.

In some embodiments, the oligonucleotide of the present invention can be represented by the following formulae:
F-G-F'; in particular F₁₋₈-G₅₋₁₆-F'₂₋₈
D'-F-G-F', in particular D'₁₋₃-F₁₋₈-G₅₋₁₆-F'₂₋₈
F-G-F'-D", in particular F₁₋₈-G₅₋₁₆-F'₂₋₈-D"₁₋₃
D'-F-G-F'-D", in particular D'₁₋₃- F₁₋₈-G₅₋₁₆-F'₂₋₈-D"₁₋₃

### Totalmers

In some embodiments, all of the nucleosides of the oligonucleotide, or contiguous nucleotide sequence thereof, are sugar modified nucleosides. Such oligonucleotides are referred to as a totalmers herein.

In some embodiments all of the sugar modified nucleosides of a totalmer comprise the same sugar modification, for example they may all be LNA nucleosides, or may also comprise 2'O-MOE nucleosides. In some embodiments the sugar modified nucleosides of a totalmer may be independently selected from LNA nucleosides and 2' substituted nucleosides, such as 2' substituted nucleoside selected from the group consisting of 2'-O-alkyl-RNA, 2'-O-methyl-RNA, 2'-alkoxy-RNA, 2'-O-methoxyethyl-RNA (MOE), 2'-amino-DNA, 2'-Fluoro-RNA, and 2'-F-ANA nucleosides. In some embodiments the oligonucleotide comprises both LNA nucleosides and 2' substituted nucleosides, such as 2' substituted nucleoside selected from the group consisting of 2'-O-alkyl-RNA, 2'-O-methyl-RNA, 2'-alkoxy-RNA, 2'-O-methoxyethyl-RNA (MOE), 2'-amino-DNA, 2'-Fluoro-RNA, and 2'-F-ANA nucleosides. In some embodiments, the oligonucleotide comprises LNA nucleosides and 2'-O-MOE nucleosides. In some embodiments, the oligonucleotide comprises (S)cET LNA nucleosides and 2'-O-MOE nucleosides. In some embodiments, each nucleoside unit of the oligonucleotide is a 2'substituted nucleoside.

In some embodiments, all of the nucleosides of the oligonucleotide or contiguous nucleotide sequence thereof are LNA nucleosides, such as beta-D-oxy-LNA nucleosides and/or (S)cET nucleosides. In some embodiments such LNA totalmer oligonucleotides are between 7 - 12 nucleosides in length (see for example, WO 2009/043353). Such short fully LNA oligonucelotides are particularly effective in inhibiting microRNAs.

Various totalmer compounds are highly effective as therapeutic oligomers, particularly when targeting microRNA (antimiRs) or as splice switching oligomers (SSOs).

In some embodiments, the totalmer comprises or consists of at least one XYX or YXY sequence motif, such as a repeated sequence XYX or YXY, wherein X is LNA and Y is an alternative (i.e. non LNA) nucleotide analogue, such as a 2'-OMe RNA unit and 2'-fluoro DNA unit. The above sequence motif may, in some embodiments, be XXY, XYX, YXY or YYX for example.

In some embodiments, the totalmer may comprise or consist of a contiguous nucleotide sequence of between 7 and 24 nucleotides, such as 7, 9, 10, 11, 12, 13, 14, 15, 16, 17, 18, 19, 20, 21, 22 or 23 nucleotides.

In some embodiments, the contiguous nucleotide sequence of the totolmer comprises of at least 30%, such as at least 40%, such as at least 50%, such as at least 60%, such as at least 70%, such as at least 80%, such as at least 90%, such as 95%, such as 100% LNA units. For full LNA compounds, it is advantageous that they are less than 12 nucleotides in length, such as 7 - 10.

The remaining units may be selected from the non-LNA nucleotide analogues referred to herein in, such those selected from the group consisting of 2'-O-alkyl-RNA unit, 2'-OMe-RNA unit, 2'-amino-DNA unit, 2'-fluoro-DNA unit, LNA unit, PNA unit, HNA unit, INA unit, and a 2'MOE RNA unit, or the group 2'-OMe RNA unit and 2'-fluoro DNA unit.

### Mixmers

The term 'mixmer' refers to oligomers which comprise both DNA nucleosides and sugar modified nucleosides, wherein there are insufficient length of contiguous DNA nucleosides to recruit RNaseH. Suitable mixmers may comprise up to 3 or up to 4 contiguous DNA nucleosides. In some embodiments the mixmers, or contiguous nucleotide sequence thereof, comprise alternating regions of sugar modified nucleosides, and DNA nucleosides. By alternating regions of sugar modified nucleosides which form a RNA like (3'endo) conformation when incorporated into the oligonucleotide, with short regions of DNA nucleosides, non-RNaseH recruiting oligonucleotides may be made. Advantageously, the sugar modified nucleosides are affinity enhancing sugar modified nucleosides.

Oligonucleotide mixmers are often used to provide occupation based modulation of target genes, such as splice modulators or microRNA inhibitors.

In some embodiments the sugar modified nucleosides in the mixmer, or contiguous nucleotide sequence thereof, comprise or are all LNA nucleosides, such as (S)cET or beta-D-oxy LNA nucleosides.

In some embodiments all of the sugar modified nucleosides of a mixmer comprise the same sugar modification, for example they may all be LNA nucleosides, or may also comprise 2'O-MOE nucleosides. In some embodiments the sugar modified nucleosides of a mixmer may be independently selected from LNA nucleosides and 2' substituted nucleosides, such as 2' substituted nucleoside selected from the group consisting of 2'-O-alkyl-RNA, 2'-O-methyl-RNA, 2'-alkoxy-RNA, 2'-O-methoxyethyl-RNA (MOE), 2'-amino-DNA, 2'-Fluoro-RNA, and 2'-F-ANA nucleosides. In some embodiments the oligonucleotide comprises both LNA nucleosides and 2' substituted nucleosides, such as 2' substituted nucleoside selected from the group consisting of 2'-O-alkyl-RNA, 2'-O-methyl-RNA, 2'-alkoxy-RNA, 2'-O-methoxyethyl-RNA (MOE), 2'-amino-DNA, 2'-Fluoro-RNA, and 2'-F-ANA nucleosides. In some embodiments, the oligonucleoitide comprises LNA nucleosides and 2'-O-MOE nucleosides. In some embodiments, the oligonucleotide comprises (S)cET LNA nucleosides and 2'-O-MOE nucleosides.

In some embodiments the mixmer, or continguous nucleotide sequence thereof, comprises only LNA and DNA nucleosides, such LNA mixmer oligonucleotides which may for example be between 8 - 24 nucleosides in length (see for example, WO2007112754, which discloses LNA antmiR inhibitors of microRNAs).

Various mixmer compounds are highly effective as therapeutic oligomers, particularly when targeting microRNA (antimiRs) or as splice switching oligomers (SSOs).

In some embodiments, the mixmer comprises a motif
...[L]m[D]n[L]m[D]n[L]m... or
...[L]m[D]n[L]m[D]n[L]m[D]n[L]m ...or
...[L]m[D]n[L]m[D]n[L]m[D]n[L]m[D]n[L]m ... or
... [L]m[D]n[L]m[D]n[L]m[D]n[L]m[D]n[L]m[D]n[L]m ...
... [L]m[D]n[L]m[D]n[L]m[D]n[L]m[D]n[L]m[D]n[L]m[D]n[L]m... or
... [L]m[D]n[L]m[D]n[L]m[D]n[L]m[D]n[L]m[D]n[L]m[D]n[L]m[D]n[L]m ... or
... [L]m[D]n[L]m[D]n[L]m[D]n[L]m[D]n[L]m[D]n[L]m[D]n[L]m[D]n[L]m[D]n[L]m ... or
[L]m[D]n[L]m[D]n[L]m[D]n[L]m[D]n[L]m[D]n[L]m[D]n[L]m[D]n[L]m[D]n[L]m[D]n[L ]m ...

Wherein L represents sugar modified nucleoside such as a LNA or 2' substituted nucleoside (e.g. 2'-O-MOE), D represents DNA nucleoside, and wherein each m is independently selected from 1 - 6, and each n is independently selected from 1, 2, 3 and 4, such as 1- 3. In some embodiments each L is a LNA nucleoside. In some embodiments, at least one L is a LNA nucleoside and at least one L is a 2'-O-MOE nucleoside. In some embodiments, each L is independently selected from LNA and 2'-O-MOE nucleoside.

In some embodiments, the mixmer may comprise or consist of a contiguous nucleotide sequence of between 10 and 24 nucleotides, such as 11, 12, 13, 14, 15, 16, 17, 18, 19, 20, 21, 22 or 23 nucleotides.

In some embodiments, the contiguous nucleotide sequence of the mixmer comprises of at least 30%, such as at least 40%, such as at least 50% LNA units.

In some embodiments, the mixmer comprises or consists of a contiguous nucleotide sequence of repeating pattern of nucleotide analogues and naturally occurring nucleotides, or one type of nucleotide analogue and a second type of nucleotide analogue. The repeating pattern, may, for instance be: every second or every third nucleotide is a nucleotide analogue, such as LNA, and the remaining nucleotides are naturally occurring nucleotides, such as DNA, or are a 2' substituted nucleotide analogue such as 2'MOE of 2'fluoro analogues as referred to herein, or, in some embodiments selected form the groups of nucleotide analogues referred to herein. It is recognised that the repeating pattern of nucleotide analogues, such as LNA units, may be combined with nucleotide analogues at fixed positions - e.g. at the 5' or 3' termini.

In some embodiments the first nucleotide of the oligomer, counting from the 3' end, is a nucleotide analogue, such as a LNA nucleotide or a 2'-O-MOE nucleoside.

In some embodiments, which maybe the same or different, the second nucleotide of the oligomer, counting from the 3' end, is a nucleotide analogue, such as a LNA nucleotide or a 2'-O-MOE nucleoside.

In some embodiments, which maybe the same or different, the 5' terminal of the oligomer is a nucleotide analogue, such as a LNA nucleotide or a 2'-O-MOE nucleoside.

In some embodiments, the mixmer comprises at least a region comprising at least two consecutive nucleotide analogue units, such as at least two consecutive LNA units.

In some embodiments, the mixmer comprises at least a region comprising at least three consecutive nucleotide analogue units, such as at least three consecutive LNA units.

### Exosomes

Exosomes are natural biological nanovesicles, typically in the range of 30 to 500 nm, that are involved in cell-cell communication via the functionally-active cargo (such as miRNA, mRNA, DNA and proteins).

Exosomes are secreted by all types of cells and are also found abundantly in the body fluids such as: saliva, blood, urine and milk. The major role of exosomes is to carry the information by delivering various effectors or signaling molecules between specific cells (Acta Pol Pharm. 2014 Jul-Aug;71(4):537-43.). Such effectors or signaling molecules can for example be proteins, miRNAs or mRNAs. Exosomes are currently being explored as a delivery vehicle for various drug molecules including RNA therapeutic molecules, to expand the therapeutic and diagnostic applications of such molecules. There are disclosures in the art of exosomes loaded with synthetic molecules such as siRNA, antisense oligonucleotides and small molecules which suggest or show advantages in terms of delivery and efficacy of such molecules compared to the free drug molecules (see for example Andaloussi et al 2013 Advanced Drug Delivery Reviews 65: 391-397, WO2014/168548, WO2016/172598, WO2017/173034 and WO 2018/102397).

Exosomes may be isolated from biological sources, such as milk (milk exosomes), in particular bovine milk is an abundant source for isolating bovine milk exosomes. See for example Manca et al., Scientific Reports (2018) 8:11321.

In some embodiments of the invention, the single stranded oligonucleotide is encapsulated in an exosome (exosome formulation), examples of loading an exosome with a single stranded antisense oligonucleotide are described in EP application No. 18192614.8. The antisense oligonucleotide may be administered to a cell or to a subject in the form of an exosome formulation, in particular oral administration of the exosome formulations are envisioned.

In some embodiments, the antisense oligonucleotide may be conjugated, e.g. with a lipophilic conjugate such as cholesterol, which may be covalently attached to the antisense oligonucleotide via a biocleavable linker (e.g. a region of phosphodiester linked DNA nucleotides). Such lipophilic conjugates can facilitate formulation of antisense oligonucleotides into exosomes and may further enhance the delivery to the target cell.

### Conjugate

The term conjugate as used herein refers to an oligonucleotide which is covalently linked to a non-nucleotide moiety (conjugate moiety or region C or third region).

Conjugation of the oligonucleotide of the invention to one or more non-nucleotide moieties may improve the pharmacology of the oligonucleotide, e.g. by affecting the activity, cellular distribution, cellular uptake or stability of the oligonucleotide. In some embodiments the conjugate moiety modifies or enhance the pharmacokinetic properties of the oligonucleotide by improving cellular distribution, bioavailability, metabolism, excretion, permeability, and/or cellular uptake of the oligonucleotide. In particular, the conjugate may target the oligonucleotide to a specific organ, tissue or cell type and thereby enhance the effectiveness of the oligonucleotide in that organ, tissue or cell type. At the same time the conjugate may serve to reduce activity of the oligonucleotide in non-target cell types, tissues or organs, e.g. off target activity or activity in non-target cell types, tissues or organs.

WO 93/07883 and WO 2013/033230 provides suitable conjugate moieties.

Further suitable conjugate moieties are those capable of binding to the asialoglycoprotein receptor (ASGPR). In particular, tri-valent N-acetylgalactosamine conjugate moieties are suitable for binding to the ASGPR, see for example WO 2014/076196, WO 2014/207232 and WO 2014/179620.

Such conjugates serve to enhance uptake of the oligonucleotide to the liver while reducing its presence in the kidney, thereby increasing the liver/kidney ratio of a conjugated oligonucleotide compared to the unconjugated version of the same oligonucleotide.

Oligonucleotide conjugates and their synthesis has also been reported in comprehensive reviews by Manoharan in Antisense Drug Technology, Principles, Strategies, and Applications, S.T. Crooke, ed., Ch. 16, Marcel Dekker, Inc., 2001 and Manoharan, Antisense and Nucleic Acid Drug Development, 2002, 12, 103

In an embodiment, the non-nucleotide moiety (conjugate moiety) is selected from the group consisting of carbohydrates, cell surface receptor ligands, drug substances, hormones, lipophilic substances, polymers, proteins, peptides, toxins (e.g. bacterial toxins), vitamins, viral proteins (e.g. capsids) or combinations thereof.

### Linkers

A linkage or linker is a connection between two atoms that links one chemical group or segment of interest to another chemical group or segment of interest via one or more covalent bonds. Conjugate moieties can be attached to the oligonucleotide directly or through a linking moiety (e.g. linker or tether). Linkers serve to covalently connect a third region, e.g. a conjugate moiety (Region C), to a first region, e.g. an oligonucleotide or contiguous nucleotide sequence complementary to the target nucleic acid (region A).

In some embodiments of the invention the conjugate or oligonucleotide conjugate of the invention may optionally, comprise a linker region (second region or region B and/or region Y) which is positioned between the oligonucleotide or contiguous nucleotide sequence complementary to the target nucleic acid (region A or first region) and the conjugate moiety (region C or third region).

Region B refers to biocleavable linkers comprising or consisting of a physiologically labile bond that is cleavable under conditions normally encountered or analogous to those encountered within a mammalian body. Conditions under which physiologically labile linkers undergo chemical transformation (e.g., cleavage) include chemical conditions such as pH, temperature, oxidative or reductive conditions or agents, and salt concentration found in or analogous to those encountered in mammalian cells. Mammalian intracellular conditions also include the presence of enzymatic activity normally present in a mammalian cell such as from proteolytic enzymes or hydrolytic enzymes or nucleases. In one embodiment the biocleavable linker is susceptible to S1 nuclease cleavage. In a preferred embodiment the nuclease susceptible linker comprises between 1 and 10 nucleosides, such as 1, 2, 3, 4, 5, 6, 7, 8, 9 or 10 nucleosides, more preferably between 2 and 6 nucleosides and most preferably between 2 and 4 linked nucleosides comprising at least two consecutive phosphodiester linkages, such as at least 3 or 4 or 5 consecutive phosphodiester linkages. Preferably the nucleosides are DNA or RNA. Phosphodiester containing biocleavable linkers are described in more detail in WO 2014/076195

Region Y refers to linkers that are not necessarily biocleavable but primarily serve to covalently connect a conjugate moiety (region C or third region), to an oligonucleotide (region A or first region). The region Y linkers may comprise a chain structure or an oligomer of repeating units such as ethylene glycol, amino acid units or amino alkyl groups The oligonucleotide conjugates of the present invention can be constructed of the following regional elements A-C, A-B-C, A-B-Y-C, A-Y-B-C or A-Y-C. In some embodiments the linker (region Y) is an amino alkyl, such as a C2 - C36 amino alkyl group, including, for example C6 to C12 amino alkyl groups. In a preferred embodiment the linker (region Y) is a C6 amino alkyl group.

### Administration

The oligonucleotides or pharmaceutical compositions of the present invention may be administered topical (such as, to the skin, inhalation, ophthalmic or otic) or enteral (such as, orally or through the gastrointestinal tract) or parenteral (such as, intravenous, subcutaneous, intra-muscular, intracerebral, intracerebroventricular or intrathecal).

In some embodiments the oligonucleotide or pharmaceutical compositions of the present invention are administered by a parenteral route including intravenous, intraarterial, subcutaneous, intraperitoneal or intramuscular injection or infusion, intrathecal or intracranial, e.g. intracerebral or intraventricular, intravitreal administration. In one embodiment the active oligonucleotide or oligonucleotide conjugate is administered intravenously. In another embodiment the active oligonucleotide or oligonucleotide conjugate is administered subcutaneously.

In some embodiments, the oligonucleotide, oligonucleotide conjugate or pharmaceutical composition of the invention is administered at a dose of 0.1 - 15 mg/kg, such as from 0.2 - 10 mg/kg, such as from 0.25 - 5 mg/kg. The administration can be once a week, every 2nd week, every third week or once a month or bi monthly.

The oligonucleotide or oligonucleotide conjugate of the invention as described may be for use for the manufacture of a medicament wherein the medicament is in a dosage form for ophthalmic such as intravitreal injection.

The oligonucleotide may be for opthalmic targets such as Htra-1.

The oligonucleotide or oligonucleotide conjugate of the invention as described may be for use for the manufacture of a medicament wherein the medicament is in a dosage form for intravenous, subcutaneous, intra-muscular, intracerebral, intracerebroventricular or intrathecal administration (e.g. injection).

### Illustrative Advantages

As illustrated herein the achiral phosphorodithioate internucleoside linkage used in the compounds of invention allows for the reduction of the complexity of a non-stereodefined phosphorothioate oligonucleotide, whilst maintaining the activity, efficacy or potency of the oligonucleotide.

Indeed, as illustrated herein, the used in the compounds of invention provides unique benefits in combination with stereodefined phosphorothioates, providing the opportunity to further reduce the complexity of phosphorothioate oligonucleotides, whilst retaining or improving the activity, efficacy or potency of the oligonucleotide.

As illustrated herein the achiral phosphorodithioate internucleoside linkage used in the compounds of invention allows for improvement in cellular uptake in vitro or in vivo.

As illustrated herein the achiral phosphorodithioate internucleoside linkage used in the compounds of invention allows for alteration or improvement in biodistribution in vitro (measured either as tissue or cellula content, or activity/potency in target tissues). Notably we have seen improvement of tissue uptake, content and /or potency in skeletal muscle, heart, spleen, liver, kidney, fibroblasts, epithelial cells.

In the context of a mixmer oligonucleotides, the inventors have identified incorporating a phosphorodithioate linkages (as shown in IA or IB), between or adjacent to one or more DNA nucleosides, provides improvements, such as enhanced stability and/or improved potency. In the context of gapmer oligonucleotides the inventors has seen that incorporation of phosphorodithioate linkages (as shown in IA or IB) between the nucleosides of the flank region (such as between 2'sugar modified nucleosides) also provides improvements, such as enhanced stability and/or improved potency.

As illustrated herein the achiral phosphorodithioate internucleoside linkage used in the compounds of invention allows for improvement in oligonucleotide stability. The incorportation of the achiral phosphorodithioate internucleoside in the compounds of the invention provides enhanced resistance to serum and cellular exonucleases, particularly 3' exonucleoases, but also 5'exonucleoases, and the remarkable sstability of the compounds of the invention further indicate a resistance to endonucleases for compounds which incorporate the achiral phosphorodithioate linkages. The stabilization of oligonucleotides is of particular importance in reducing or preventing the accumulation of toxic degradation products, and prolonging the duration of action of the antisense oligonucleotide. As illustrated in the examples rat serum stability may be used to assay for improved stability. For evaluation of cellular stability, tissue (e.g. liver) homogenate extract may be used - for example see WO2014076195 which provided such methods). Other assays for meansuring oligonucleotide stability include snake venom phosphodiesterase stability assays and S1 nuclease stability).

Reduced toxicity risk of the claimed oligonucleotides is tested in vitro hepatotoxicity assays (e.g. as disclosed in WO 2017/067970) or in vitro nephrotoxicity assays (e.g. as disclosed in WO 2017/216340)., or in vitro neurotoxicity assays (e.g. as disclosed in WO2016127000). Alternatively, toxicity may be assayed in vivo, for example in mouse or rat.

Enhanced stability can provide benefits to the duration of action of the oligonucleotides of the invention, which is of particular benefit for when the administration route is invasive, e.g. parenteral administration, such as, intravenous, subcutaneous, intra-muscular, intracerebral, intraocular, intracerebroventricular or intrathecal administration.

### ANTISENSE OLIGONUCLEOTIDE EMBODIMENTS

The invention relates to an oligonucleotide comprising at least one phosphorodithioate internucleoside linkage of formula (IA) or (IB) 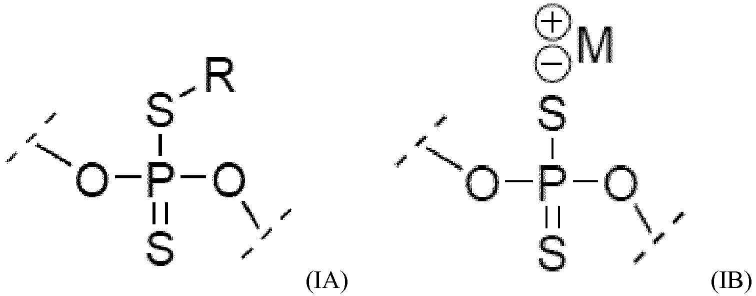 wherein one of the two oxygen atoms is linked to the 3'carbon atom of an adjacent nucleoside (A¹) and the other one is linked to the 5'carbon atom of another adjacent nucleoside (A²), and wherein in (IA) R is hydrogen or a phosphate protecting group, and in (IB) M+ is a cation, such as a metal cation, such as an alkali metal cation, such as a Na+ or K+ cation; or M+ is an ammonium cation , wherein at least one of the two nucleosides (A¹) and (A²) is a LNA nucleoside, and wherein the oligonucleotide comprises between 1 and 5 internucleoside linkages of formula (IA) or (IB) and wherein the further internucleoside linkages are all phosphorothioate internucleoside linkages.

Alternatively stated M is a metal, such as an alkali metal, such as Na or K; or M is NH₄.

The oligonucleotide may, for example, be a single stranded antisense oligonucleotide, which is capable of modulating the expression of a target nucleic acid, such as a target microRNA, or is capable of modulating the splicing of a target pre-mRNA.which comprises a contiguous nucleotide sequence. The antisense oligonucleotide of the invention comprises a contiguous nucleotide sequence which is complementary to the target nucleic acid, and is capable of hybridizing to and modulating the expression of the target nucleic acid. In a preferred embodiment, the antisense oligonucleotide, or the conitguous nucleotide sequence thereof, is a mixmer oligonucleotide wherein either (A¹) or (A²) is a DNA nucleoside.

In the context of the present invention an antisense oligonucleotide is a single stranded oligonucleotide which is complementary to a nucleic acid target, such as a target RNA, and is capable of modulating (e.g. splice modulating of a pre-mRNA target) or inhibiting the expression of the nucleic acid target (e.g. a mRNA target, a premRNA taget, a viral RNA target, or a long non coding RNA target). Depending on the target the length of the oligonucleotide or the length of the region therof which is complementary to (i.e. antisense - preferably the complementary region is fully complementary to the target) may be 7 - 30 nucleotides (the region which is referred to as the contiguous nucleotide sequence). For example LNA nucleotide inhibitors of microRNAs may be as short as 7 contiguous complementary nucleotides (and may be as long as 30 nucleotides), RNaseH recruiting oligonucleotides are typically at least 12 contiguous complementary nucleotides in length, such as 12- 26 nucleotides in length. Splice modulating antisense oligonucleotides typically has a contiguous nucleotide region of 10 - 30 complementary nucleotides.

Splice modulating oligonucleotides, also known as splice-switching oligonucleotides (SSOs) are short, synthetic, antisense, modified nucleic acids that base-pair with a pre-mRNA and disrupt the normal splicing repertoire of the transcript by blocking the RNA-RNA base-pairing or protein-RNA binding interactions that occur between components of the splicing machinery and the pre-mRNA. Splicing of pre-mRNA is required for the proper expression of the vast majority of protein-coding genes, and thus, targeting the process offers a means to manipulate protein production from a gene. Splicing modulation is particularly valuable in cases of disease caused by mutations that lead to disruption of normal splicing or when interfering with the normal splicing process of a gene transcript may be therapeutic. SSOs offer an effective and specific way to target and alter splicing in a therapeutic manner. See Haven's and Hasting NAR (2016) 44, 6549-6563. SSOs may be complementary to an Exon/Intron boundary in the target pre-mRNA or may target splicing enhanced or silencer elements (collectively refered to as cis-acting splice elements) within the pre-mRNA that regulates spicing of the pre-mRNA. Splice modulation may result in exon skipping, or exon inclusion and thereby modulates alternive splicing of a pre-mRNA. SSOs function by non nuclease mediated modulation of the target pre-mRNA, and therefore are not capable of recruiting RNaseH, they are often either fully modified oligonucleotides, i.e. each nucleoside comprises a modified sugar moiety, such as a 2'sugar substituted sugar moiety (for example fully 2'-O-MOE oligonucleotides of e.g. 15 - 25 nucleotides in length, often 18 - 22 or 20 nucleotides in length, based on a phosphorothioate back bone), or LNA mixmer oligonucleotides (oligonucleotides 10-30 nucleotides in length which comprises DNA and LNA nucleosides, and optinally other 2' sugar modified nucleosides, such as 2'-O-MOE. Also envisaged are LNA oligonucleotides which do not comprises DNA nucleosides, but comprise of LNA and other 2'sugar modified nucleosides, such as 2'-O-MOE nucleosides. Table 1 of Haven's and Hasting NAR (2016) 44, 6549-6563, illustrates a range of SSO targets and the chemistry of the oligonucleotides used which have reported activity in vivo, and is reproduced below in Table A:

**TABLE A**

| **Condition** | **Target gene** | **Stage/Mode l** | **SSO** | **Target (Action)** | **Route** | **Ref** (see Haven' s and Hastin g |
|---|---|---|---|---|---|---|
| **Condition** | **Target gene** | **Stage/Mode l** | **SSO** | **Target (Action)** | **Route** | **Ref** (see Haven' s and Hastin g |
| Block cryptic/Aberrant splicing caused by mutations | | | | | | |
| β-Thalassemia | *HBB* | mouse | PPMO | intron 2 aberrant 5'ss (correct splicing) | IV | (144) |
| Fukuyama congenital muscular dystrophy | *FKTN* | mouse | VPMO | exon 10 aberrant 3'ss; alternative 5'ss; ESE (correct splicing) | IM | (145) |
| Hutchinson-Gilford progeria | *LMNA* | mouse | VPMO; 2'-MOE /PS | exon 10 5'ss; exon 11 cryptic 5'ss; exon 11 ESE (block exon 11 splicing) | IV/IP | (146,14 7) |
| Leber congenital amaurosis | *CEP290* | mouse | 2'-OMe /PS; AAV | Intron 26 cryptic exon (correct splicing) | IVI | (56) |
| Myotonic dystrophy | *CLCN1* | mouse | PMO | exon 7a 3'ss (exon 7a skipping) | IM | (53,148) |
| Usher syndrome | *USH1C* | mouse | 2'-MOE /PS | exon 3 cryptic 5'ss (correct | IP | (40) |
| | | | | splicing) | | |
| X-linked agammaglobulinemia | *BTK* | mouse | PPMO | pseudoexon 4A ESS (pseudoexon skipping) | IV/SC | (149) |

| Switch alternative splicing | | | | | | |
|---|---|---|---|---|---|---|
| Alzheimer's disease | *LRP8* | mouse | 2'-MOE /PS | intron 19 ISS (exon 19 inclusion) | ICV | (42) |
| Autoimmune diabetes susceptibility | *CTLA4* | mouse | PPMO | exon 2 3'ss (exon skipping) | IP | (150) |
| Cancer | *BCL2L1* | mouse | 2'-MOE /PS | exon 2 5'ss (alternative 5'ss) | IV/NP | (151) |
| Cancer | *ERBB4* | mouse | LNA | exon 26 5'ss (exon skipping) | IP | (152) |
| Cancer | *MDM4* | mouse | PMO | exon 6 5'ss (exon skipping) | ITM | (153) |
| Cancer | *STAT3* | mouse | VPMO | exon 23 α 3'ss (β 3'ss use) | ITM | (154) |

| **Condition** | **Target gene** | **Stage/Mode l** | **SSO** | **Target (Action)** | **Route** | **Ref** (see Haven' s and Hastin g |
|---|---|---|---|---|---|---|
| Inflammation | *IL1RAP* | mouse | 2-OMe /PS;LNA | exon 9 ESE (exon skipping) | IV/NP | (155) |
| Inflammation | *TNFRSF1B* | mouse | LNA /PS | exon 7 5'ss (exon skipping) | IP | (156) |
| Neovascularization | *FLT1* | mouse | PMO | exon 13 5'ss (alternative pA site) | IVI / ITM | (157) |
| Neovascularization | *KDR* | mouse | PMO | exon 13 5'ss (alternative pA site) | IVI / SCJ | (158) |
| Spinal muscular atrophy | *SMN2* | clinical trials | 2'-MOE /PS | intron 7 ISS (exon 7 inclusion) | IT | (43,142) |

| Correct open reading frame | | | | | | |
|---|---|---|---|---|---|---|
| cardiomyopathy | *MYBPC3* | mouse | AAV | Exon 5 and 6 ESEs (exon 5, 6 skipping) | IV | (159) |
| Cardiomyopathy | *TTN* | mouse | VPMO | exon 326 ESE (exon skipping) | IP | (160) |
| Duchenne muscular dystrophy (DMD) | *DMD* | clinical trials | 2'-OMe / PMO | exon 51 ESE (exon | IV/SC | (46,98) |

| Condition | **Target gene** | **Stage/Mode l** | **SSO** | **Target (Action)** | **Route** | **Ref (see** Haven' s and Hastin g |
|---|---|---|---|---|---|---|
| | | | | skipping) | | |
| Nijmegen breakage syndrome | *NBN* | mouse | VPMO | exon 6/7 ESEs (exon skipping) | IV | (161) |

| Disrupt open reading frame/Protein function | | | | | | |
|---|---|---|---|---|---|---|
| Ebola | *IL10* | mouse | PPMO | exon 4 3'ss (exon skipping) | IP | (162) |
| Huntington disease | *HTT* | mouse | 2'-OMe /PS | exon 12 skipping | IS | (163) |
| Hypercholesterolemia | *APOB* | mouse | 2'-OMe /PS | exon 27 3'ss (exon skipping) | IV | (164) |
| Muscle-Wasting/DMD | *MSTN* | mouse | PPMO/VPMO/ 2'-OMe | exon 2 ESE (exon skipping) | IV/ IM/ IP | (165,16 6) |
| Pompe disease | *GYS2* | mouse | PPMO | exon 6 5'ss (exon skipping) | IM/IV | (167) |
| Spinocerebellar ataxia type 3 | *ATXN3* | mouse | 2'-OMe /PS | exon 9, 10 skipping | ICV | (168) |

In some embodiments of the invention, the antisense oligonucleotide is a splice modulating oligonucleotide which is complementary to a pre-mRNA selected from the group consisting of a HBB, FKTN, LMNA, CEP290, CLCN1, USH1C, BTK, LRP8, CTLA4, BCL2L1, ERBB4, MDM4, STAT3, IL1RAP, TNFRSF1B, FLT1, KDR, SMN2, MYBPC3, TTN, DMD, NBN, IL10, HTT, APOB, MSTN, GYS2, and ATXN3. Exemplary diseases which may be treated with the SSOs of the invention, on a target by target basis are provided in Table A.

### Htra-1 Targeting Antisense Oligonucelotides of the Invention

In some embodiments, the antisense oligonucleotide of the invention is complementary to the mRNA or pre-mRNA encoding the human high temperature requirement A1 Serine protease (Htral) - see WO 2018/002105 for example. Inhibition of Htral expression using the antisense oligonucleotides of the invention which target Htral mRNa or premRNA are beneficial for a treating a range of medical disorders, such as macular degeneration, e.g. age-related macular degeneration (geographic atrophy). Human Htral pre-mRNA and mRNA target sequences are available as follows:

| **Species** | **Chr** . | **Stran d** | **Genomic coordinates** | | **Assembly** | **NCBI reference sequence* accession number for mRNA** |
|---|---|---|---|---|---|---|
| | | | **Start** | **End** | | |
| Human | 10 | fwd | 12246152 5 | 12251490 8 | GRCh38.p2 release 107 | NM_002775.4 |

Compounds of the invention which target Htra-1 are listed as Htra1#1 - 38 in the examples.

### FURTHER EMBODIMENTS OF THE INVENTION

In some embodiments of the invention, the oligonucleotide is an antisense oligonucleotide capable of modulating the expression of a target RNA in a cell expressing said target RNA .

In some embodiments, the oligonucleotide is an antisense oligonucleotide capable of inhibiting the expression of a target RNA in a cell expressing said target RNA .

In some embodiments, one of (A¹) and (A²) is a LNA nucleoside and the other one is a DNA nucleoside, a RNA nucleoside or a sugar modified nucleoside .

In some embodiments, one of (A¹) and (A²) is a LNA nucleoside and the other one is a DNA nucleoside or a sugar modified nucleoside.

In some embodiments, one of (A¹) and (A²) is a LNA nucleoside and the other one is a DNA nucleoside .

In some embodiments, one of (A¹) and (A²) is a LNA nucleoside and the other one is a sugar modified nucleoside .

In some embodiments, said sugar modified nucleoside is a 2'-sugar modified nucleoside .

In some embodiments, said 2'-sugar modified nucleoside is 2'-alkoxy-RNA, 2'-alkoxyalkoxy-RNA, 2'-amino-DNA, 2'-fluoro-RNA, 2'-fluoro-ANA or a LNA nucleoside .

In some embodiments, said 2'-sugar modified nucleoside is a LNA nucleoside .

In some embodiments, the LNA nucleosides are independently selected from beta-D-oxy LNA, 6'-methyl-beta- D-oxy LNA and ENA.

In some embodiments, the LNA nucleosides are both beta-D-oxy LNA.

In some embodiments, said 2'-sugar modified nucleoside is 2'-alkoxyalkoxy-RNA .

In some embodiments, 2'-alkoxy-RNA is 2'-methoxy-RNA.

In some embodiments, 2'-alkoxyalkoxy-RNA is 2'-methoxyethoxy-RNA.

An oligonucleotide according to the invention comprises
between 1 and 5, more particularly 1, 2, 3, 4 or 5 phosphorodithioate internucleoside linkages of formula (I) as defined above;
In some embodiments, the oligonucleotide is a gapmer, in particular a LNA gapmer, a mixed wing gapmer, an alternating flank gapmer, a splice switching oligomer, a mixmer or a totalmer;
In some embodiments, the oligonucleotide is a gapmer and wherein the at least one phosphorodithioate internucleoside linkage of formula (I) is comprised in the gap region and/or in one or more flanking region of the gapmer;
In some embodiments, the oligonucleotide is of 7 to 30 nucleotides in length.

When the oligonucleotide of the invention is a gapmer, it is advantageously of 12 to 26 nucleotides in length. 16 nucleotides is a particularly advantageous gapmer oligonucleotide length.

When the oligonucleotide is a full LNA oligonucleotide, it is advantageously of 7 to 10 nucleotides in length.

When the oligonucleotide is a mixmer oligonucleotide, it is advantageously of 8 to 30 nucleotides in length.

In some embodiments, one or more nucleoside is a nucleobase modified nucleoside .

In some embodiments, the oligonucleotide is an antisense oligonucleotide, a siRNA, a microRNA mimic or a ribozyme .

The invention also provides a pharmaceutically acceptable salt of an oligonucleotide according to the invention, in particular a sodium or a potassium salt.

The invention also provides a conjugate comprising an oligonucleotide or a pharmaceutically acceptable salt according to the invention and at least one conjugate moiety covalently attached to said oligonucleotide or said pharmaceutically acceptable salt, optionally *via* a linker moiety .

The invention also provides a pharmaceutical composition comprising an oligonucleotide, pharmaceutically acceptable salt or conjugate according to the invention and a therapeutically inert carrier .

The invention also provides an oligonucleotide, pharmaceutically acceptable salt or conjugate according to the invention for use as a therapeutically active substance .

The oligonucleotide, pharmaceutically acceptable salt or conjugate according to the invention may be used as a medicament.

In some embodiments, the oligonucleotide of the invention has a higher activity in modulating its target nucleic acid, as compared to the corresponding fully phosphorothioate linked-oligonucleotide. In some embodiments the invention provides for oligonucleotides with enhanced activity, enhanced potency, enhanced specific activity or enhanced cellular uptake. In some embodiments the invention provides for oligonucleotides which have an altered duration of action *in vitro* or *in vivo,* such as a prolonged duration of action *in vitro* or *in vivo.* In some embodiments the higher activity in modulating the target nucleic acid is determined *in vitro* or *in vivo* in a cell which is expressing the target nucleic acid.

In some embodiments the oligonucleotide of the invention has altered pharmacological properties, such as reduced toxicity, for example reduced nephrotoxicity, reduced hepatotoxicity or reduced immune stimulation. Hepatotoxicity may be determined, for example *in vivo,* or by using the in vitro assays disclosed in WO 2017/067970 . Nephrotoxicity may be determined, for example *in vitro,* or by using the assays disclosed in PCT/EP2017/064770, hereby incorporated by reference. In some embodiments the oligonucleotide of the invention comprises a 5' CG 3' dinucleotide, such as a DNA 5' CG 3' dinucleotide, wherein the internucleoside linkage between C and G is a phosphorodithioate internucleoside linkage of formula (I) as defined above.

In some embodiments, the oligonucleotide of the invention has improved nuclease resistance such as improved biostability in blood serum. In some embodiments, the 3' terminal nucleoside of the oligonucleotide of the invention has an A or G base, such as a 3' terminal LNA-A or LNA-G nucleoside. Suitably, the internucleoside linkage between the two 3' most nucleosides of the oligonucleotide may be a phosphorodithioate internucleoside linkage according to formula (I) as defined above.

In some embodiments the oligonucleotide of the invention has enhanced bioavailability. In some embodiments the oligonucleotide of the invention has a greater blood exposure, such as a longer retention time in blood.

The non-bridging phosphorodithioate modification is introduced into oligonucleotides by means of solid phase synthesis using the phosphoramidite method. Syntheses are performed using controlled pore glass (CPG) equipped with a universal linker as the support. On such a solid support an oligonucleotide is typically built up in a 3' to 5' direction by means of sequential cycles consisting of coupling of 5'O-DMT protected nucleoside phosphoramidite building blocks followed by (thio)oxidation, capping and deprotection of the DMT group. Introduction of non-bridging phosphorodithioates is achieved using appropriate thiophosphoramidite building blocks followed by thiooxidation of the primary intermediate.

While the corresponding DNA thiophosphoramidites are commercially available, the respective LNA building blocks have not been described before. They can be prepared from the 5'-O-DMT-protected nucleoside 3'-alcohols e.g. by the reaction with mono-benzoyl protected ethanedithiol and tripyrrolidin-1-ylphosphane.

The oligonucleotide according to the invention can thus for example be manufactured according to Scheme 2, wherein R¹, R^{2a}, R^{2b}, R^{4a}, R^{4b}, R⁵, R^{x}, R^{y} and V are as defined below.

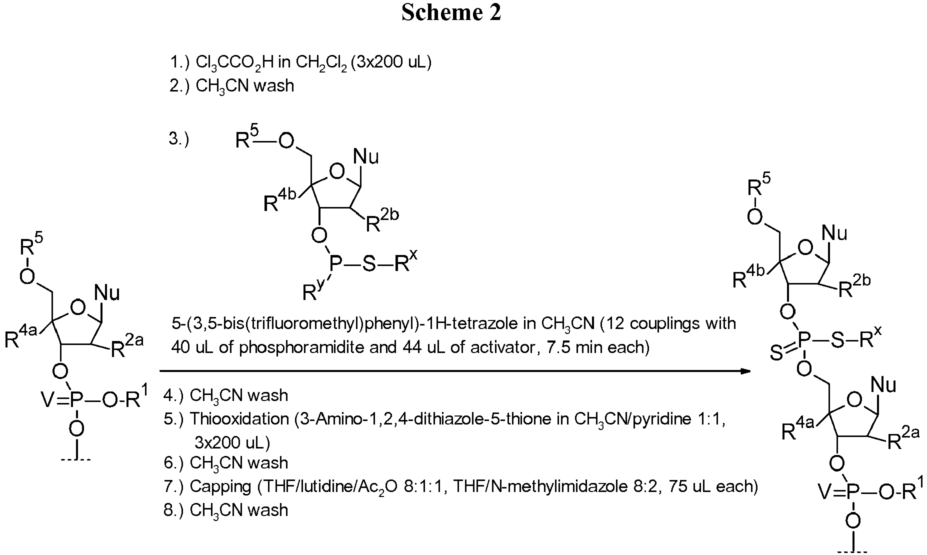

A process for the manufacture of an oligonucleotide according to the invention may comprise the following steps:
(a) Coupling a thiophosphoramidite nucleoside to the terminal 5' oxygen atom of a nucleotide or oligonucleotide to produce a thiophosphite triester intermediate;
(b) Thiooxidizing the thiophosphite triester intermediate obtained in step (a); and
(c) Optionally further elongating the oligonucleotide.

A process for the manufacture of an oligonucleotide according to the invention may comprise the following steps:
(a1) Coupling a compound of formula (A) 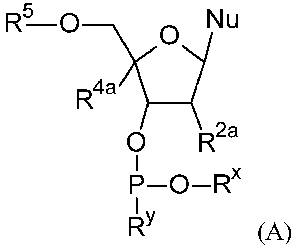 to the 5' oxygen atom of a nucleotide or oligonucleotide of formula (B) 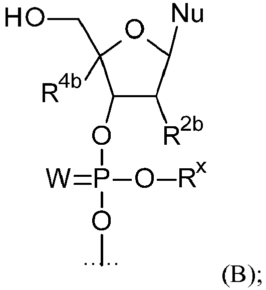
(b1) Thiooxidizing the thiophosphite triester intermediate obtained in step (a1); and
(c1) Optionally further elongating the oligonucleotide;
wherein
R^{2a} and R^{4a} together form -X-Y- as defined above; or
R^{4a} is hydrogen and R^{2a} is selected from alkoxy, in particular methoxy, halogen, in particular fluoro, alkoxyalkoxy, in particular methoxyethoxy, alkenyloxy, in particular allyloxy and aminoalkoxy, in particular aminoethyloxy;
R^{2b} and R^{4b} together form -X-Y- as defined above; or
R^{2b} and R^{4b} are both hydrogen at the same time; or
R^{4b} is hydrogen and R^{2b} is selected from alkoxy, in particular methoxy, halogen, in particular fluoro, alkoxyalkoxy, in particular methoxyethoxy, alkenyloxy, in particular allyloxy and aminoalkoxy, in particular aminoethyloxy;
V is oxygen or sulfur; and
wherein R⁵, R^{x}, R^{y} and Nu are as defined below.

A process for the manufacture of an oligonucleotide according to the invention may comprise the following steps:
(a2) Coupling a compound of formula (II) 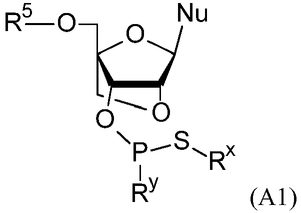 to the 5' oxygen atom of a nucleotide or oligonucleotide of formula (IV) 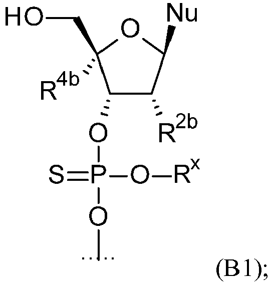
(b2) Thiooxidizing the thiophosphite triester intermediate obtained in step (a2); and
(c2) Optionally further elongating the oligonucleotide;
wherein
R^{2b} and R^{4b} together form -X-Y- as defined above; or
R^{2b} and R^{4b} are both hydrogen at the same time; or
R^{4b} is hydrogen and R^{2b} is selected from alkoxy, in particular methoxy, halogen, in particular fluoro, alkoxyalkoxy, in particular methoxyethoxy, alkenyloxy, in particular allyloxy and aminoalkoxy, in particular aminoethyloxy; and
wherein R⁵, R^{x}, R^{y} and Nu are as defined below.

The oligonucleotide of the invention may be an antisense oligonucleotide capable of modulating the expression of a target RNA in a cell expressing said target RNA.

In some embodiments, the oligonucleotide is an antisense oligonucleotide capable of inhibiting the expression of a target RNA in a cell expressing said target RNA .

In some embodiments, the oligonucleotide is capable of recruiting RNAseH, such as human RN aseH 1 .

In some embodiments, the oligonucleotide is a gapmer oligonucleotide wherein the gapmer oligonucleotide comprises a contiguous nucleotide sequence of formula 5'-F-G-F'-3', wherein G is a region of 5 to 18 nucleosides which is capable of recruiting RnaseH, and said region G is flanked 5' and 3' by flanking regions F and F' respectively, wherein regions F and F' independently comprise or consist of 1 to 7 2'-sugar modified nucleotides, wherein the nucleoside of region F which is adjacent to region G is a 2'-sugar modified nucleoside and wherein the nucleoside of region F' which is adjacent to region G is a 2'-sugar modified nucleoside.

In some embodiments where the oligonucleotide is a gapmer oligonucleotide, the 2'-sugar modified nucleosides are independently selected from 2'-alkoxy-RNA, 2'-alkoxyalkoxy-RNA, 2'-amino-DNA, 2'-fluoro-RNA, 2'-fluoro-ANA and LNA nucleosides.

In some embodiments where the oligonucleotide is a gapmer oligonucleotide, 2'-alkoxyalkoxy-RNA is a 2'-methoxyethoxy-RNA (2'-O-MOE) .

In some embodiments where the oligonucleotide is a gapmer oligonucleotide, region F and region F' comprise or consist of 2'-methoxyethoxy-RNA nucleotides .

In some embodiments where the oligonucleotide is a gapmer oligonucleotide, both regions F and F' consist of 2'-methoxyethoxy-RNA nucleotides

In some embodiments where the oligonucleotide is a gapmer oligonucleotide, at least one or all of the 2'-sugar modified nucleosides in region F or region F', or in both regions F and F', are LNA nucleosides .

In some embodiments where the oligonucleotide is a gapmer oligonucleotide, region F or region F', or both regions F and F', comprise at least one LNA nucleoside and at least one DNA nucleoside .

In some embodiments where the oligonucleotide is a gapmer oligonucleotide, region F or region F', or both region F and F' comprise at least one LNA nucleoside and at least one non-LNA 2'-sugar modified nucleoside, such as at least one 2'-methoxyethoxy-RNA nucleoside .

In some embodiments where the oligonucleotide is a gapmer oligonucleotide, the gap region comprises 5 to 16, in particular 8 to 16, more particularly 8, 9, 10, 11, 12, 13 or 14 contiguous DNA nucleosides .

In some embodiments where the oligonucleotide is a gapmer oligonucleotide, region F and region F' are independently 1, 2, 3, 4, 5, 6, 7 or 8 nucleosides in length.

In some embodiments where the oligonucleotide is a gapmer oligonucleotide, region F and region F' each indendently comprise 1, 2, 3 or 4 LNA nucleosides .

In some embodiments where the oligonucleotide is a gapmer oligonucleotide, the LNA nucleosides are independently selected from beta-D-oxy LNA, 6'-methyl-beta-D-oxy LNA and ENA;
In some embodiments where the oligonucleotide is a gapmer oligonucleotide, the LNA nucleosides are beta-D-oxy LNA.

In some embodiments where the oligonucleotide is a gapmer oligonucleotide, the oligonucleotide, or contiguous nucleotide sequence thereof (F-G-F'), is of 10 to 30 nucleotides in length, in particular 12 to 22, more particularly of 14 to 20 oligonucleotides in length .

In some embodiments, the gapmer oligonucleotide comprises a contiguous nucleotide sequence of formula 5'-D'-F-G-F'-D"-3', wherein F, G and F' are as defined in any one of claims 4 to 17 and wherein region D' and D" each independently consist of 0 to 5 nucleotides, in particular 2, 3 or 4 nucleotides, in particular DNA nucleotides

In some embodiments, the gapmer oligonucleotide is capable of recruiting human RNaseH1.

In some embodiments where the oligonucleotide is a gapmer oligonucleotide, said at least one phosphorodithioate internucleoside linkage of formula (I) as defined above is positioned between adjacent nucleosides in region F or region F', between region F and region G or between region G and region F' .

In some embodiments where the oligonucleotide is a gapmer oligonucleotide, the internucleoside linkages between the nucleosides of region G are independently selected from phosphorothioate internucleoside linkages and phosphorodithioate internucleoside linkages of formula (I) as defined above.

In some embodiments where the oligonucleotide is a gapmer oligonucleotide, the internucleoside linkages between the nucleosides of region G comprise 0, 1, 2 or 3 phosphorodithioate internucleoside linkages of formula (I) as defined above .

In some embodiments where the oligonucleotide is a gapmer oligonucleotide, the flanking regions F and F' together comprise 1, 2, 3 or 4 phosphorodithioate internucleoside linkages of formula (I) as defined above .

In some embodiments where the oligonucleotide is a gapmer oligonucleotide, flanking regions F and F' each comprise 2 phosphorodithioate internucleoside linkages of formula (I) as defined above .

In some embodiments where the oligonucleotide is a gapmer oligonucleotide, all the internucleoside linkages of flanking regions F and/or F' are phosphordithioate internucleoside linkages of formula (I) as defined above .

A gapmer oligonucleotide according to the invention may comprise as at least one stereo defined phosphorothioate internucleoside linkage .

The gap region may comprises- 1, 2, 3, 4 or 5 stereodefined phosphorothioate internucleoside linkages.

In some embodiments, all the internucleoside linkages between the nucleosides of the gap region are stereodefined phosphorothioate internucleoside linkages .

A gapmer oligonucleotide according to the invention may comprise at least one phosphorodithioate internucleoside linkage of formula (I) as defined above positioned between at least two adjacent nucleosides of region F, or between at least two adjacent nucleosides of region F', or between region F and region G, or between region G and region F', and the remaining internucleoside linkages between the nucleotides of region F and F' are independently selected from phosphorothioate internucleoside linkages, and phosphorodithioate internucleoside linkages of formula (I). The phosphorothioate internucleoside linkages of region F and F' may be either stereorandom or stereo defined, or may be independently selected from stereorandom and stereo defined .

A gapmer oligonucleotide according to the invention may comprise at least one phosphorodithioate internucleoside linkage of formula (I) as defined above positioned between at least two adjacent nucleosides of region F, or between at least two adjacent nucleosides of region F', or between region F and region G, or between region G and region F', and the remaining internucleoside linkages between the nucleotides of region F and F', between region F and region G and between region G and region F', are independently selected from phosphorothioate internucleoside linkages and phosphorodithioate internucleoside linkage of formula (I); The phosphorothioate internucleoside linkages of region F and F' may be either stereorandom or stereo defined, or may be independently selected from stereorandom and stereo defined .

A gapmer oligonucleotide according to the invention may comprise at least one phosphorodithioate internucleoside linkage of formula (I) as defined above positioned between at least two adjacent nucleosides of region F, or between at least two adjacent nucleosides of region F', or between region F and region G, or between region G and region F', and the remaining internucleoside linkages between the nucleotides of region F and F' and between region F and region G and between region G and region F', are independently selected from stereodefined phosphorothioate internucleoside linkages and phosphorodithioate internucleoside linkage of formula (I) .

A gapmer oligonucleotide according to the invention may comprise at least one phosphorodithioate internucleoside linkage of formula (I) as defined above positioned between at least two adjacent nucleosides of region F, or between at least two adjacent nucleosides of region F', or between region F and region G, or between region G and region F', and the remaining internucleoside linkages within region F and F', between region F and region G and between region G and region F', are phosphorothioate internucleoside linkages, which may be all stereorandom phosphorothioate internucleoside linkages, all stereodefined phosphorothioate internucleoside linkages, or may be independently selected from stereorandom and stereodefined phosphorothioate internucleoside linkages .

In some embodiments, the remaining internucleoside linkages within region F, within region F' or within both region F and region F' are all phosphorodithioate internucleoside linkages of formula (I) as defined above.

In some embodiments, the internucleoside linkages between the nucleosides of region G comprise 0, 1, 2 or 3 phosphorodithioate internucleoside linkages of formula (I) as defined above and the remaining internucleoside linkages within region G are independently selected from stereo defined phosphorothioate internucleoside linkages and stereorandom phosphorothioate internucleoside linkages .

In some embodiments, the internucleoside linkages between the nucleosides of region G comprise 0, 1, 2 or 3 phosphorodithioate internucleoside linkages of formula (I) as defined above and at least one of the remaining internucleoside linkages within region G, or all of the remaining internucleoside linkages within region G are stereo defined phosphorothioate internucleoside linkages .

In some embodiments, the internucleoside linkages between the nucleosides of region G comprise 0, 1, 2 or 3 phosphorodithioate internucleoside linkages of formula (I) as defined above and the remaining internucleoside linkages within region G are phosphorothioate internucleoside linkages, such as stereorandom phosphorothioate internucleoside linkages .

In some embodiments, at least one of region F or F' comprise the at least one phosphorodithioate internucleoside linkages of formula (I) as defined above and all the internucleoside linkages within region G are phosphorothioate internucleoside linkages, such as stereorandom phosphorothioate internucleoside linkages.

In some embodiments, at least one of region F or F' comprise the at least one phosphorodithioate internucleoside linkages of formula (I) as defined above and all the internucleoside linkages within region G are phosphorothioate internucleoside linkages, wherein at least one of the phosphorothioate internucleoside linkages within region G is a stereodefined phosphorothioate internucleoside linkage .

In some embodiments, at least one of region F or F' comprise the at least one phosphorodithioate internucleoside linkages of formula (I) as defined above and all the internucleoside linkages within region G are stereodefined phosphorothioate internucleoside linkages .

In some embodiments, the internucleoside linkage between region F and G, or the internucleoside linkage between region G and F', or both the internucleoside linkages between region F and G and between region G and F', are phosphorodithioate internucleoside linkages of formula (I) as defined above, and wherein, in the event that only one of the internucleoside linkages between region F and G and between region G and F' is a phosphorodithioate internucleoside linkages of formula (I) as defined above, the other internucleoside linkage between region F and G or between region G and F' is a phosphorothioate internucleoside linkage .

In some embodiments, at least one of region F or F' comprise the at least one phosphorodithioate internucleoside linkage of formula (I) as defined above, wherein the internucleoside linkage between region F and G, or the internucleoside linkage between region G and F', or both the internucleoside linkages between region F and G and between region G and F', are phosphorodithioate internucleoside linkages of formula (I) as defined above and wherein in the event that only one of the internucleoside linkages between region F and G and between region G and F' is a phosphorodithioate internucleoside linkages of formula (I) as defined above, the other internucleoside linkage between region F and G or between region G and F' is a phosphorothioate internucleoside linkage.

In some embodiments, the internucleoside linkages between the nucleosides of region G comprise 0, 1, 2 or 3 phosphorodithioate internucleoside linkages of formula (I) as defined above and the remaining internucleoside linkages within region G are phosphorothioate internucleoside linkages, wherein the internucleoside linkage between region F and G, or the internucleoside linkage between region G and F', or both the internucleoside linkages between region F and G and between region G and F', are phosphorodithioate internucleoside linkages of formula (I) as defined above and wherein in the event that only one of the internucleoside linkages between region F and G and between region G and F' is a phosphorodithioate internucleoside linkages of formula (I) as defined above, the other internucleoside linkage between region F and G or between region G and F' is a phosphorothioate internucleoside linkage .

In some embodiments, at least one of region F or F' comprise the at least one phosphorodithioate internucleoside linkages of formula (I) as defined above, wherein the internucleoside linkages between the nucleosides of region G comprise 0, 1, 2 or 3 phosphorodithioate internucleoside linkages of formula (I) as defined above and the remaining internucleoside linkages within region G are phosphorothioate internucleoside linkages, wherein the internucleoside linkage between region F and G, or the internucleoside linkage between region G and F', or both the internucleoside linkages between region F and G and between region G and F', are phosphorodithioate internucleoside linkages of formula (I) as defined above, and wherein, in the event that only one of the internucleoside linkages between region F and G and between region G and F' is a phosphorodithioate internucleoside linkage of formula (I) as defined above, the other internucleoside linkage between region F and G or between region G and F' is a phosphorothioate internucleoside linkage .

In some embodiments, region F or region F' comprise at least one phosphorodithioate internucleoside linkages of formula (I) as defined above, or wherein the internucleoside linkage between region F and region G, or between region G and region F' comprise at least one phosphorodithioate internucleoside linkage of formula (I) as defined above, region G comprises 1, 2 or 3 phosphorodithioate internucleoside linkages of formula (I) as defined above, and the remaining internucleoside linkages within region G are phosphorothioate internucleoside linkages .

In some embodiments, region F or region F' comprise at least one phosphorodithioate internucleoside linkages of formula (I) as defined above, or wherein the internucleoside linkage between region F and region G, or between region G and region F' comprise at least one phosphorodithioate internucleoside linkages of formula (I) as defined above, all of the internucleoside linkage within region G are phosphorothioate internucleoside linkages and wherein at least one of the phosphorothioate internucleoside linkages within region G is a stereodefined phosphorothioate internucleoside linkage.

In some embodiments, region F or region F' comprise at least one phosphorodithioate internucleoside linkages of formula (I) as defined above, or wherein the internucleoside linkage between region F and region G, or between region G and region F' comprise at least one phosphorodithioate internucleoside linkages of formula (I) as defined above, all of the internucleoside linkages within region G are phosphorothioate internucleoside linkages and wherein all of the phosphorothioate internucleoside linkages within region G are stereo defined phosphorothioate internucleoside linkages .

In some embodiments, other than the at least one phosphorodithioate internucleoside linkages of formula (I) as defined above, all the remaining internucleoside linkages within the gapmer region F-G-F' are phosphorothioate internucleoside linkages.

In some embodiments, at least one of region F or F' comprise the at least one phosphorodithioate internucleoside linkages of formula (I) as defined above and all the internucleoside linkages within region G are stereodefined phosphorothioate internucleoside linkages .

In some embodiments, other than the at least one phosphorodithioate internucleoside linkages of formula (I) all the remaining internucleoside linkages within the gapmer region F-G-F' are stereo defined phosphorothioate internucleoside linkages .

A gapmer oligonucleotide according to the invention may be a LNA gapmer, a mixed wing gapmer, an alternating flank gapmer or a gap-breaker gapmer.

The invention also provides a pharmaceutically acceptable salt of a gapmer oligonucleotide according to the invention, in particular a sodium or a potassium salt.

The invention also provides a conjugate comprising a gapmer oligonucleotide or a pharmaceutically acceptable salt according to the invention and at least one conjugate moiety covalently attached to said oligonucleotide or said pharmaceutically acceptable salt, optionally *via* a linker moiety, in particular *via* a a bioclieavable linker, particularly via 2 to 4 phosphodiester linked DNA nucleosides (e.g. region D' or D") .

The invention also provides a pharmaceutical composition comprising a gapmer oligonucleotide, pharmaceutically acceptable salt or conjugate according to the invention and a therapeutically inert carrier .

The invention also provides a gapmer oligonucleotide, pharmaceutically acceptable salt or conjugate according to the invention for use as a therapeutically active substance .

The invention will now be illustrated by the following examples which have no limiting character.

### Examples

### Example 1: Monomer synthesis

### 1.1: S-(2-sulfanylethyl) benzenecarbothioate

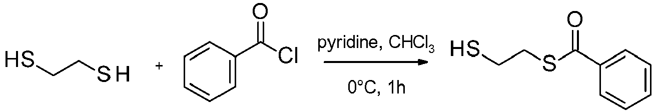

To a solution of 1,2-ethanedithiol (133.57 mL, 1592 mmol, 1 eq) and pyridine (64.4 mL, 796 mmol, 0.5 eq) in chloroform (200 mL) was added benzoyl chloride (92.4 mL, 796 mmol, 0.5 eq) in chloroform (200 mL) dropwise for 1 hr, and the reaction was stirred at 0 °C for 1 hr. The mixture was washed with water (300 mL) and brine (300 mL). The organic phase was dried over Na₂SO₄ and concentrated to a yellow oil. The oil was distilled (135~145°C) to afford S-(2-sulfanylethyl) benzenecarbothioate (40 g, 202mmol, 13% yield) as a colorless oil. ¹H NMR (400 MHz, CDCl₃) δ 7.97 (d, *J*=7.34 Hz, 2H), 7.53-7.64 (m, 1H), 7.47 (t, *J*=7.58 Hz, 2H), 3.31 (t, *J*=7.34 Hz, 2H), 2.77-2.86 (m, 2H), 1.70 (t, *J*=8.56 Hz, 1H).

### 1.2: S-[2-[[(1R,3R,4R,7S)-1-[[bis(4-methoxyphenyl)-phenyl-methoxy]methyl]-3-(5-methyl-2,4-dioxo-pyrimidin-1-yl)-2,5-dioxabicyclo[2.2.1]heptan-7-yl]oxy-pyrrolidin-1-yl-phosphanyl] sulfanylethyl] benzenecarbothioate

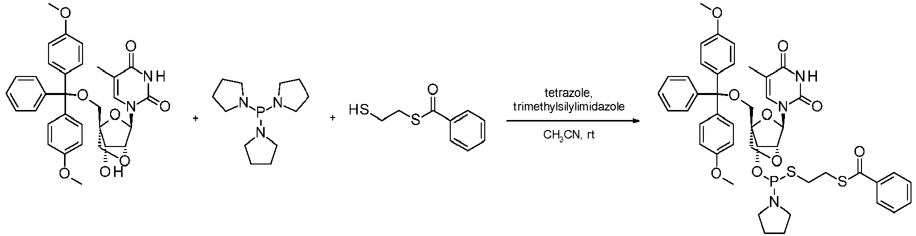

1-[(1R,4R,6R,7S)-4-[[bis(4-methoxyphenyl)-phenyl-methoxy]methyl]-7-hydroxy-2,5-dioxabicyclo[2.2.1]heptan-6-yl]-5-methyl-pyrimidine-2,4-dione (2.29 g, 4.00 mmol, 1.0 eq) was dissolved in 60 mL of anhydrous dichloromethane to which a spatula of 3 Å molecular sieves was added. Tripyrrolidin-1-ylphosphane (960 mg, 3.98 mmol, 0.99 eq) was added via syringe followed by seven 0.1 mmol aliquots of tetrazole (7 * 0.4 mL of a 0.5 M solution in anhydrous acetonitrile stored over 3 Å molecular sieves) at 2 min intervals. N-trimethylsilylimidazole (56.0 mg, 0.400 mmol, 0.1 eq) was then added to the reaction. After 5 min, tetrazole (21.6 mL of a 0.5 M solution in anhydrous acetonitrile) was added, immediately followed by the addition of S-(2-sulfanylethyl) benzenecarbothioate (1.04 g, 5.24 mmol, 1.31 eq). The reaction was allowed to proceed for 120 sec. Four identical batches of the reaction were united and quenched by pouring the solution into 600 mL of dichloromethane containing 40 mL of triethylamine. The mixture was immediately washed with saturated sodium bicarbonate (800 mL) followed by 10% sodium carbonate (2 * 800 mL) and brine (800 mL). The organic layer was dried over Na₂SO₄. After 10-15 min the drying agent was removed by filtration. Triethylamine (40 mL) was added to the solution which was concentrated using a rotary evaporator to a syrup. The syrup was dissolved in toluene (200 mL) and triethylamine (40 mL), and this solution was pipetted into 4500 mL of vigorously stirred heptane to precipitate the fluffy white product. After most of the heptane was decanted, the white precipitate was collected by filtration through a medium sintered glass funnel and subsequently dried under vacuum to give a white solid. The solid was purified by prep-HPLC (Phenomenex Gemini C18, 250x50mm, 10 mm column, 0.05% ammonium hydroxide in water / CH₃CN), and freeze-dried to afford 4.58 g of target compound as a white solid. ³¹P NMR (162 MHz, CD₃CN) δ 167.6, 164.2. ¹H NMR (400 MHz, CD₃CN) δ 9.16 (br s, 1H), 7.93 (t, J=7.41 Hz, 2H), 7.60-7.71 (m, 1H), 7.45-7.57 (m, 4H), 7.24-7.45 (m, 7H), 6.90 (d, J=8.93 Hz, 4H), 5.53-5.63 (m, 1H), 4.41-4.64 (m, 2H), 3.74-3.88 (m, 8H), 3.39-3.63 (m, 2H), 3.03-3.32 (m, 5H), 2.77-2.94 (m, 2H), 1.66-1.84 (m, 4H), 1.54-1.66 (m, 3H).

### 1.3: S-[2-[[(1R,3R,4R,7S)-3-(6-benzamidopurin-9-yl)-1-[[bis(4-methoxyphenyl)-phenyl-methoxy] methyl]-2,5-dioxabicyclo [2.2.1] heptan-7-yl] oxy-pyrrolidin-1-yl-phosphanyl]sulfanylethyl] benzenecarbothioate

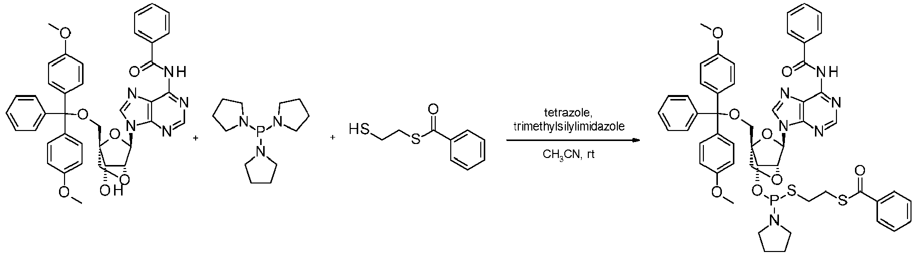

N-[9-[(1R,4R,6R,7S)-4-[[bis(4-methoxyphenyl)-phenyl-methoxy]methyl]-7-hydroxy-2,5-dioxabicyclo[2.2.1]heptan-6-yl]purin-6-yl]benzamide (2.74g, 4.00 mmol, 1.0 eq) was dissolved in 60 mL of anhydrous dichloromethane to which a spatula of 3 Å molecular sieves was added. Tripyrrolidin-1-ylphosphane (960 mg, 3.98 mmol, 0.99 eq) was added via syringe followed by seven 0.1 mmol aliquots of tetrazole (7 * 0.4 mL of a 0.5 M solution in anhydrous acetonitrile stored over 3 Å molecular sieves) at 2 min intervals. 1-(trimethylsilyl)-1H-imidazole (56.0 mg, 0.400 mmol, 0.1 eq) was then added to the reaction. After 5 min, tetrazole (21.6 mL of a 0.5 M solution in anhydrous acetonitrile) was added, immediately followed by the addition of S-(2-sulfanylethyl) benzenecarbothioate (1.04 g, 5.24 mmol, 1.31 eq). The reaction was allowed to proceed for 120 s.

Four identical batches of the reaction were united and quenched by pouring the solution into 600 mL of dichloromethane containing 40 mL of triethylamine. The mixture was immediately washed with saturated sodium bicarbonate (800 mL) followed by 10% sodium carbonate (2 * 800 mL) and brine (800 mL). The organic layer was dried over Na₂SO₄. After 10-15 min the drying agent was removed by filtration. Triethylamine (10 mL) was added to the solution which was concentrated using a rotary evaporator to a syrup. The syrup was dissolved in toluene (100 mL) and triethylamine (20 mL), and this solution was pipetted into 4500 mL of vigorously stirred heptane to precipitate the fluffy white product. After most of the heptane was decanted, the white precipitate was collected by filtration through a medium sintered glass funnel and subsequently dried under vacuum to give a white solid. The solid was purified by prep-HPLC (Phenomenex Gemini C18, 250x50mm, 10 mm column, 0.05% ammonium hydroxide in water / CH₃CN), and freeze-dried to afford 5.26 g of target compound as a white solid. ³¹P NMR (162 MHz, CD₃CN) δ 165.6, 164.7. ¹H NMR (400 MHz, CD₃CN) δ 8.56 (d, *J*=10.76 Hz, 1H), 8.24 (d, *J*=10.27 Hz, 1H), 7.82-7.93 (m, 2H), 7.71-7.80 (m, 2H), 6.92-7.54 (m, 14H), 6.68-6.83 (m, 4H), 6.03 (d, *J*=6.48 Hz, 1H), 4.70-4.90 (m, 2H), 3.81-3.98 (m, 2H), 3.59-3.68 (m, 7H), 3.25-3.47 (m, 2H), 2.81-3.02 (m, 6H), 2.56-2.81 (m, 2H), 1.44-1.72 (m, 4H).

### 1.4: S-[2-[[(1R,3R,4R,7S)-3-(4-benzamido-5-methyl-2-oxo-pyrimidin-1-yl)-1-[[bis(4-methoxyphenyl)-phenyl-methoxy] methyl] -2,5-dioxabicyclo [2.2.1] heptan-7-yl] oxy-pyrrolidin-1-yl-phosphanyl]sulfanylethyl] benzenecarbothioate

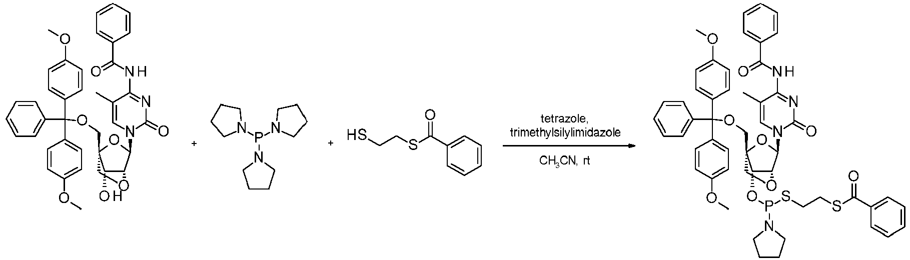

N-[1-[(1R,4R,6R,7S)-4-[[bis(4-methoxyphenyl)-phenyl-methoxy]methyl]-7-hydroxy-2,5-dioxabicyclo[2.2.1]heptan-6-yl]-5-methyl-2-oxo-pyrimidin-4-yl]benzamide (2.70 g, 4.00 mmol, 1.0 eq) was dissolved in 60 mL of anhydrous dichloromethane to which a spatula of 3 Å molecular sieves was added. Tripyrrolidin-1-ylphosphane (965 mg, 4.00 mmol, 1.0 eq) was added via syringe followed by seven 0.1 mmol aliquots of tetrazole (7 * 0.4 mL of a 0.5 M solution in anhydrous acetonitrile stored over 3 Å molecular sieves) at 2 min intervals. 1-(trimethylsilyl)-1H-imidazole (56.0 mg, 0.400 mmol, 0.1 eq) was then added to the reaction. After 5 min, tetrazole (21.6 mL of a 0.5 M solution in anhydrous acetonitrile) was added, immediately followed by the addition of S-(2-sulfanylethyl) benzenecarbothioate (1.04 g, 5.24 mmol, 1.31 eq). The reaction was allowed to proceed for 120 sec. Four identical batches of the reaction were quenched and united by pouring the solution into 600 mL of dichloromethane containing 40 mL of triethylamine. The mixture was immediately washed with saturated sodium bicarbonate (800 mL) followed by 10% sodium carbonate (2 * 800 mL) and brine (800 mL). The organic layer was dried over Na₂SO₄. After 10-15 min the drying agent was removed by filtration. Triethylamine (40 mL) was added to the solution which was concentrated using a rotary evaporator to a syrup. The syrup was dissolved in toluene (100 mL) and triethylamine (30 mL), and this solution was pipetted into 4500 mL of vigorously stirred heptane to precipitate the fluffy white product. After most of the heptane was decanted, the white precipitate was collected by filtration through a medium sintered glass funnel and subsequently dried under vacuum to give a white solid. The solid was purified by prep-HPLC (Phenomenex Gemini C18, 250x50mm, 10 mm column, 0.05% ammonium hydroxide in water / CH₃CN) and freeze-dried to afford 2.05 g of target compound as a white solid. ³¹P NMR (162 MHz, CD₃CN) δ 171.2, 167.4. ¹H NMR (400 MHz, CD₃CN) δ 8.18-8.32 (m, 2H), 7.81-7.93 (m, 3H), 7.35-7.60 (m, 14H), 7.17-7.35 (m, 2H), 6.93 (d, *J*=8.93 Hz, 4H), 5.65 (d, *J*=15.04 Hz, 1H), 4.56-4.72 (m, 2H), 3.69-3.90 (m, 8H), 3.45-3.61 (m, 2H), 3.03-3.26 (m, 6H), 2.76-3.02 (m, 2H), 1.65-1.93 (m, 7H).

### 1.5: S-[2-[[(1R,3R,4R,7S)-1-[[bis(4-methoxyphenyl)-phenyl-methoxy]methyl]-3-[2-[(E)-dimethylaminomethyleneamino]-6-oxo-1H-purin-9-yl]-2,5-dioxabicyclo[2.2.1]heptan-7-yl]oxy-pyrrolidin-1-yl-phosphanyl]sulfanylethyl] benzenecarbothioate

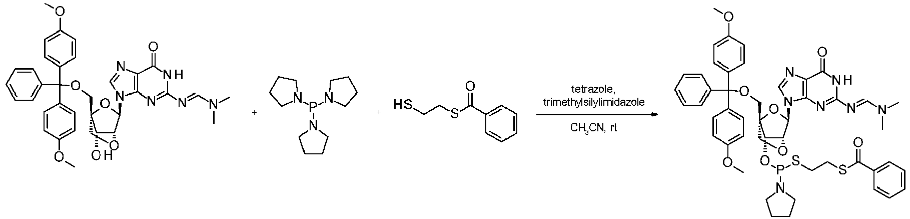

N'-[9-[(1R,4R,6R,7S)-4-[[bis(4-methoxyphenyl)-phenyl-methoxy]methyl]-7-hydroxy-2,5-dioxabicyclo[2.2.1]heptan-6-yl]-6-oxo-1H-purin-2-yl]-N,N-dimethyl-formamidine (2.62 mg, 4.00 mmol, 1.0 eq) was dissolved in 200 mL of anhydrous dichloromethane to which a spatula of 3 Å molecular sieves was added. Tripyrrolidin-1-ylphosphane (965 mg, 4.00 mmol, 1.0 eq) was added via syringe followed by seven 0.1 mmol aliquots of tetrazole (7 * 0.4 mL of a 0.5 M solution in anhydrous acetonitrile stored over 3 Å molecular sieves) at 2 min intervals. 1-(trimethylsilyl)-1H-imidazole (56.0 mg, 0.400 mmol, 0.1 eq) was then added to the reaction. After 5 min, tetrazole (21.6 mL of a 0.5 M solution in anhydrous acetonitrile) was added, immediately followed by the addition of S-(2-sulfanylethyl) benzenecarbothioate (1.04 g, 5.24 mmol, 1.31 eq). The reaction was allowed to proceed for 180 s.

Four identical batches were combined and quenched by pouring the solutions into 600 mL of dichloromethane containing 40 mL of triethylamine. The mixture was immediately washed with saturated sodium bicarbonate (800 mL) followed by 10% sodium carbonate (2 * 800 mL) and brine (800 mL). The organic layer was dried over Na₂SO₄. After 10-15 min the drying agent was removed by filtration. Triethylamine (40 mL) was added to the solution which was concentrated using a rotary evaporator to a syrup. The syrup was dissolved in toluene (100 mL) and triethylamine (30 mL), and this solution was pipetted into 4500 mL of vigorously stirred heptane to precipitate the fluffy white product. After most of the heptane was decanted, the white precipitate was collected by filtration through a medium sintered glass funnel and subsequently dried under vacuum to give a white solid. The solid was purified by prep-HPLC (Phenomenex Gemini C18, 250x50mm, 10 mm column, 0.05% ammonium hydroxide in water / CH₃CN) and freeze-dried to afford 3.82 g of target compound as a yellow solid. ³¹P NMR (162 MHz, CD₃CN) δ 167.1, 162.2. ¹H NMR (400 MHz, CD₃CN) δ 9.36 (br s, 1H), 8.63 (d, *J*=16.51 Hz, 1H), 7.78-8.00 (m, 3H), 7.66 (t, *J*=7.62 Hz, 1H), 7.42-7.57 (m, 4H), 7.24-7.40 (m, 7H), 6.89 (d, *J*=8.68 Hz, 4H), 5.92-5.98 (m, 1H), 4.72-4.97 (m, 2H), 3.86-4.05 (m, 2H), 3.78 (2s, 6H), 3.27-3.70 (m, 3H), 2.87-3.20 (m, 12H), 2.67-2.82 (m, 2H), 1.54-1.79 (m, 4H).

### Example 2: Oligonucleotide synthesis

Oligonucleotides were synthesized using a MerMade 12 automated DNA synthesizer by Bioautomation. Syntheses were conducted on a 1 µmol scale using a controlled pore glass support (500Å) bearing a universal linker.

In standard cycle procedures for the coupling of DNA and LNA phosphoramidites DMT deprotection was performed with 3% (w/v) trichloroacetic acid in CH₂Cl₂ in three applications of 200 µL for 30 sec. The respective phosphoramidites were coupled three times with 100 µL of 0.1 M solutions in acetonitrile (or acetonitrile/CH₂Cl₂ 1:1 for the LNA-^{Me}C building block) and 110 µL of a 0.1 M solution of 5-(3,5-bis(trifluoromethylphenyl))-1H-tetrazole in acetonitrile as an activator and a coupling time of 180 sec. For thiooxidation a 0.1m solution of 3-amino-1,2,4-dithiazole-5-thione in acetonitrile/pyridine 1:1 was used (3x190 µL, 55 sec). Capping was performed using THF/lutidine/Ac₂O 8:1:1 (CapA, 75 µmol) and THF/N-methylimidazole 8:2 (CapB, 75 µmol) for 55 sec.

Synthesis cycles for the introduction of thiophosphoramidites included DMT deprotection using 3% (w/v) trichloroacetic acid in in CH₂Cl₂ in three applications of 200 µL for 30 sec. Commercially available DNA thiophosphoramidites or freshly prepared LNA thiophosphoramidites were coupled three times with 100 µL of 0.15 M solutions in 10% (v/v) CH₂Cl₂ in acetonitrile and 110 µL of a 0.1 M solution of 5-(3,5-bis(trifluoromethylphenyl))-1H-tetrazole in acetonitrile as an activator and a coupling time of 600 sec each. Thiooxidation was performed using a 0.1 M solution of 3-amino-1,2,4-dithiazole-5-thione in acetonitrile/pyridine in three applications for 55 sec. Capping was performed using THF/lutidine/Ac₂O 8:1:1 (CapA, 75 µmol) and THF/*N*-methylimidazole 8:2 (CapB, 75 µmol) for 55 sec.

Upon completion of the automated synthesis, removal of the nucleobase protecting groups and cleavage from the solid support is carried out using an ammonia (32%):ethanol (3:1, v:v) mixture containing 20 mM DTT at 55°C for 15-16 h.

Crude DMT-on oligonucleotides were purified either using a solid phase extraction cartridge and repurification with ion exchange chromatography or by RP-HPLC purification using a C18 column followed by DMT removal with 80% aqueous acetic acid and ethanol precipitation.

In the following examples we have used the following thio linkage chemistries

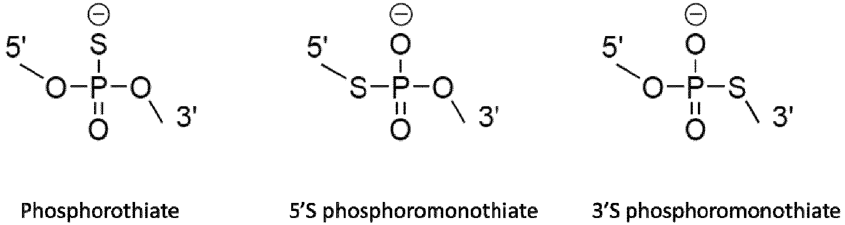

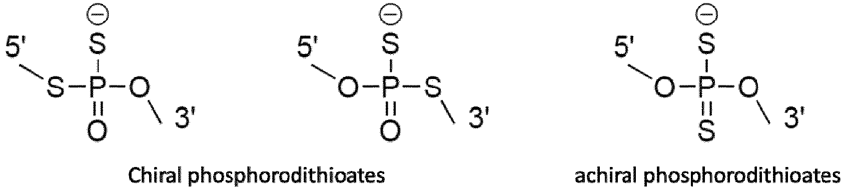

In the following examples, unless otherwise indicated, the achiral phosphorodithioate linkages (also referred to as P2S) are non-bridging dithioates (as illustrated in formula (IA) or (IB)), and are labelled as *. The compounds used in the example include compounds with the following sequence of nucleobases:
SEQ ID NO 1: GCATTGGTATTCA
SEQ ID NO 2: TCTCCCAGCGTGCGCCAT
SEQ ID NO 3: GAGTTACTTGCCAACT
SEQ ID NO 4: TATTTACCTGGTTGTT
SEQ ID NO 5: CAATCAGTCCTAG

The following molecules have been prepared following the above procedure.

| Compound ID No. | Compound (SEQ ID NO) | Calculated mass | Found mass |
|---|---|---|---|
| #1 | G^{m}Ca*ttggtatT^{m}CA | 4341.6 | 4341.6 |
| #2 | G^{m}Cat*tggtatT^{m}CA | 4341.6 | 4341.6 |
| #3 | G^{m}Catt*ggtatT^{m}CA | 4341.6 | 4341.6 |
| #4 | G^{m}Cattg*gtatT^{m}CA | 4341.6 | 4341.6 |
| #5 | G^{m}Cattgg*tatT^{m}CA | 4341.6 | 4341.6 |
| #6 | G^{m}Cattggt*atT^{m}CA | 4341.6 | 4341.6 |
| #7 | G^{m}Cattggta*tT^{m}CA | 4341.6 | 4341.6 |
| #8 | G^{m}Cattggtat*T^{m}CA | 4341.6 | 4341.6 |
| #9 | G^{m}Cat*t*ggtatT^{m}CA | 4357.6 | 4356.8 |
| #10 | G^{m}Cattggt*at*T^{m}CA | 4357.6 | 4356.8 |
| #11 | G^{m}Cat*tggt*atT^{m}CA | 4357.6 | 4357.2 |
| #12 | G^{m}Catt*ggtat*T^{m}CA | 4357.6 | 4356.8 |
| #13 | G^{m}Cat*tggtat*T^{m}CA | 4357.6 | 4356.9 |
| #14 | G^{m}Cat*t*ggtat*T^{m}CA | 4373.7 | 4373.5 |
| #15 | G^{m}Catt*ggt*at*T^{m}CA | 4373.7 | 4373.1 |
| #16 | G^{m}Cat*t*ggt*atT^{m}CA | 4373.7 | 4373.0 |
| #17 | G^{m}Cat*t*ggt*at*T^{m}CA | 4389.8 | 4389.1 |
| #18 | G^{m}Ca*ttg*gta*tT^{m}CA | 4373.7 | 4373.0 |
| #19 | G^{m}Ca*tt*gg*ta*tT^{m}CA | 4389.8 | 4389.0 |
| #20 | G^{m}Ca*ttggta*tT^{m}CA | 4357.6 | 4356.9 |
| #21 | G^{m}Ca*ttggtat*T^{m}CA | 4357.6 | 4357.1 |
| #22 | G^{m}Cat*tggta*tT^{m}CA | 4357.6 | 4356.9 |
| #23 | G^{m}Cattg*gt*atT^{m}CA | 4357.6 | 4357.6 |
| #24 | G^{m}Cattg*g*t*atT^{m}CA | 4373.7 | 4373.7 |
| #25 | G^{m}Cattg*g*t*at*T^{m}CA | 4389.8 | 4389.8 |
| #26 | G^{m}Cattg*g*t*a*t*T^{m}CA | 4405.8 | 4405.8 |
| #27 | G^{m}CattggtatT^{m}C*A | 4341.6 | 4342.0 |
| #28 | G*^{m}CattggtatT^{m}CA | 4341.6 | 4342.5 |
| #29 | G*^{m}CattggtatT^{m}C*A | 4357.6 | 4359.0 |
| #30 | G*^{m}CattggtatT*^{m}C*A | 4373.7 | 4368.5 |
| #31 | G*^{m}C*attggtatT^{m}C*A | 4373.7 | 4369.2 |
| #32 | G*^{m}C*attggtatT*^{m}C*A | 4389.8 | 4390.6 |

| | | | |
|---|---|---|---|
| * Dithioate modification between adjacent nucleotides A, G, ^{m}C, T represent LNA nucleotides a, g, c, t represent DNA nucleotides all other linkages were prepared as phosphorothioates | | | |

### Example 3: In vitro efficacy and cellular uptake experiments

Primary rat Hepatocytes were plated in 96-well plates and treated in Williams Medium E containing 10% FCS without antibiotics. Cells were treated with LNA solutions in the indicated concentrations in full cell culture medium. After an incubation time of 24 and 72 hrs, respectively, the cells were washed 3 times with PBS containing Ca²⁺ and Mg²⁺ and lysed with 165 uL PureLink Pro lysis buffer. Total RNA was isolated using the PureLink PRO 96 RNA Kit from Thermo Fisher according to the manufacturers instructions and RT-qPCR was performed using the LightCycler Multiplex RNA Virus Master (Roche) with Primer Probe Sets for RnApoB (Invitrogen). The obtained data was normalized to Ribogreen.

Intracellular concentrations of the LNA oligonucleotides were determined using an hybridization based ELISA assay for a variety of compounds. All data points were performed in triplicates and data is given as the average thereof.

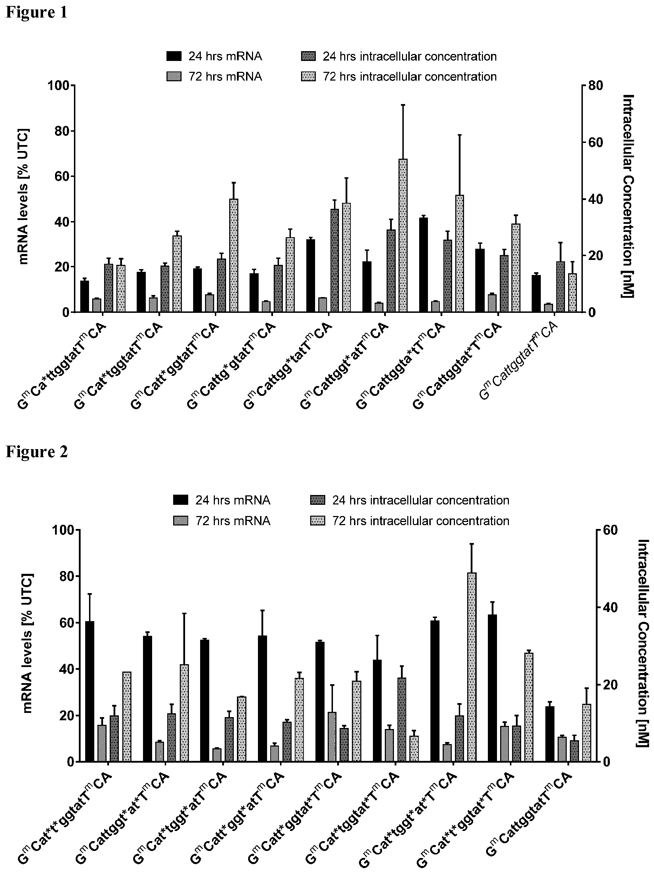

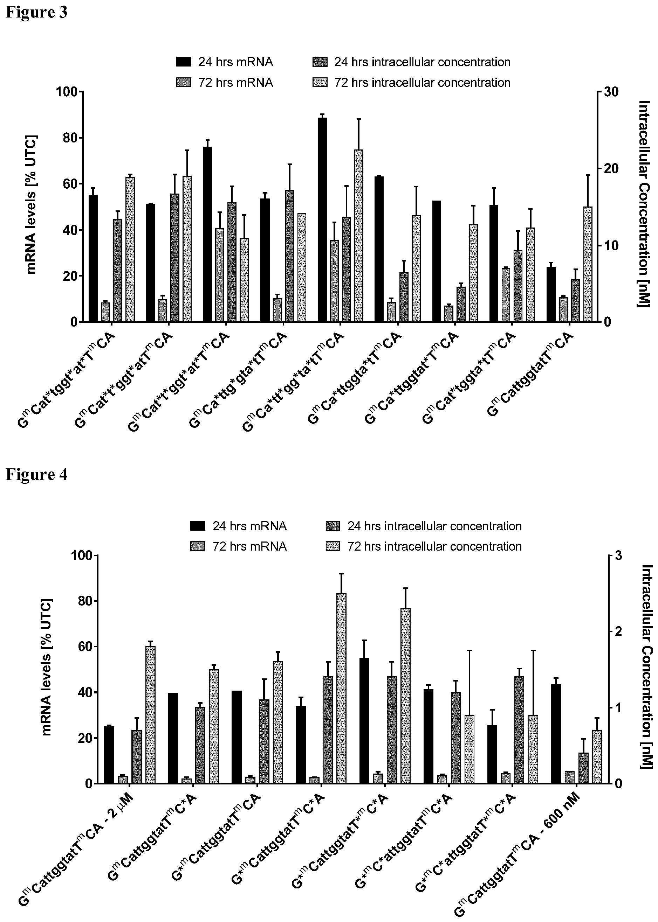

The results are shown in Figures 1 to 4.

### Example 4: Thermal melting (Tm) of oligonucleotides containing a phophorodithioate internucleoside linkage hybridized to RNA and DNA

The following oligonucleotides have been prepared. Phosphorothoiate linkages are designated by the S subscript; Phosphorodithioate linkages according to the invention are designated by the PS2 subscript.

| Compound | |
|---|---|
| 1 | 5'-G_{S} ^{m}C_{S} a_{S} t_{S} t_{S} g_{S} g_{S} t_{S} a_{S} t_{S} T_{S} ^{m}C_{PS2} A -3' |
| 2 | 5'-G_{PS2} ^{m}C_{S} a_{S} t_{S} t_{S} g_{S} g_{S} t_{S} a_{S} t_{S} T_{S} ^{m}C_{S} A -3' |
| 3 | 5'-G_{PS2} ^{m}C_{S} a_{S} t_{S} t_{S} g_{S} g_{S} t_{S} a_{S} t_{S} T_{S} ^{m}C_{PS2} A -3' |
| 4 | 5'-G_{PS2} ^{m}C_{S} a_{S} t_{S} t_{S} g_{S} g_{S} t_{S} a_{S} t_{S} T_{PS2} ^{m}C_{PS2} A -3' |
| 5 | 5'-G_{PS2} ^{m}C_{PS2} a_{S} t_{S} t_{S} g_{S} g_{S} t_{S} a_{S} t_{S} T_{S} ^{m}C_{PS2} A -3' |
| 6 | 5'-G_{PS2} ^{m}C_{PS2} a_{S} t_{S} t_{S} g_{S} g_{S} t_{S} a_{S} t_{S} T_{PS2} ^{m}C_{PS2} A -3' |
| Control | 5'-Gs ^{m}C_{S} a_{S} t_{S} t_{S} g_{S} g_{S} t_{S} a_{S} t_{S} T_{S} ^{m}C_{S} A -3' |
| DNA Control SEQ ID NO 2 | 5'-t_{S} c_{S} t_{S} c_{S} c_{S} c_{S} a_{S} g_{S} c_{S} g_{S} t_{S} g_{S} c_{S} g_{S} c_{S} c_{S} a_{S} t -3' |

Compounds 1-6 have the sequence motif SEQ ID NO 1.

The thermal melting (Tm) of compounds **1-6** hybridized to RNA and DNA was measured according to the following procedure.

A solution of equimolar amount of RNA or DNA and LNA oligonucleotide (1.5 µM) in buffer (100 mM NaCl, 0.1 mM EDTA, 10 mM Na₂HPO₄, pH 7) was heated to 90 °C for 1 min and then allowed to cool to room temperature. The UV absorbance at 260 nm was recorded using a Cary Series UV-Vis spectrophotometer (heating rate 1 °C per minute; reading rate one per min). The absorbance was plotted against temperature and the Tm values were calculated by taking the first derivative of each curve.

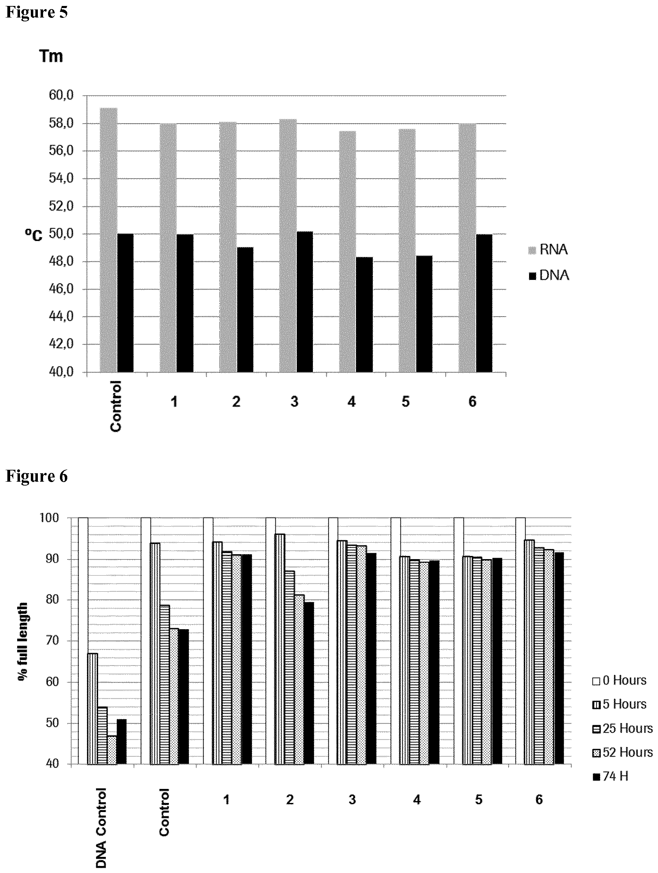

The results are summarized in the Table below and in Figure 5.

| **RNA** | | | | **DNA** | | |
|---|---|---|---|---|---|---|
| | Td | Ta | ΔTm | Td | Ta | ΔTm |
| **Control** | 59,1 | 57,7 | 1,5 | 50,1 | 47,7 | 2,4 |
| **1** | 58,0 | 54,8 | 3,2 | 50,0 | 46,9 | 3,1 |
| **2** | 58,1 | 55,7 | 2,5 | 49,1 | 46,7 | 2,4 |
| **3** | 58, 3 | 55,5 | 2,8 | 50,2 | 47,6 | 2,6 |
| **4** | 57,5 | 54,4 | 3,1 | 48,4 | 46,5 | 1,8 |
| **5** | 57,6 | 56,3 | 1,3 | 48,5 | 47,4 | 1,1 |
| **6** | 58,0 | 55,8 | 2,2 | 50,0 | 46,9 | 3,1 |

| | | | | | | |
|---|---|---|---|---|---|---|
| Td: Temperature of dissociation (denaturation); Ta: Temperature of association (renaturation) | | | | | | |

The compounds according to the invention retain the high affinity for RNA and DNA of the control.

### Example 5: Serum stability of oligonucleotides containing a phophorodithioate internucleoside linkage

Stability of oligonucleotides **1-6** in serum from male Sprague-Dawling rats was measured according to the following procedure.

A 25 µM oligonucleotide solution in rat serum mixed with Nuclease buffer (30 mM sodium acetate, 1mM zinc sulfate, 300 mM NaCl, pH 4,6) 3:1 were incubated at 37 °C for 0, 5, 25, 52 or 74 hours. Samples 2µL were injected for UPLC-MS analysis on a Water Acquity UPLC equipped with a Water Acquity BEH C₁₈, 1,7 µm column. The analogue peak areas measured at 260 nm compensated with the extention constants of the different degradation lengths were used to establish the % of uncleaved oligonucleotide.

UPLC eluents: A: 2,5% MeOH, 0,2 M HEP, 16,3 mM TEA B: 60% MeOH, 0,2 M HEP, 16,3 mM TEA

| TIME MIN. | FLOW ML/ | % A BUFFER | % B BUFFER |
|---|---|---|---|
| | MIN | | |
| 0 | 0.5 | 90 | 10 |
| 0.5 | 0.5 | 90 | 10 |
| 5 | 0.5 | 70 | 30 |
| 6 | 0.5 | 70 | 30 |
| 7 | 0.5 | 0 | 100 |
| 8 | 0.5 | 0 | 100 |
| 9 | 0.5 | 90 | 10 |
| 14.9 | 0.5 | 90 | 10 |
| 15 | 0.5 | 90 | 10 |

The results are summarized in Figure 6.

The compounds having at least one phosphorodithioate internucleoside linkage according to the invention have a superior nuclease resistance than the compounds having only phosphorothioate internucleoside linkages.

The initial oligonucleotide degradation seen after 5 hours in compounds **1-6** was found to be caused by the presence of a monothioate impurity.

### Example 7: Dithioate Modified Gapmers: Exploring the dithioates in the gap region of LNA gapmers.

### Compounds Tested

| *single modification in the gap* | | | |
|---|---|---|---|
| #1 | GCa*ttggtatTCA | #5 | GCattgg*tatTCA |
| #2 | GCat*tggtatTCA | #6 | GCattggt*atTCA |
| #3 | GCatt*ggtatTCA | #7 | GCattggta*tTCA |
| #4 | GCattg*gtatTCA | #8 | GCattggtatTCA |

| *cumulation in gap region* | | | *dithioate in LNA flanks* |
|---|---|---|---|
| #9 | GCattg*gt*atTCA | #13 | GC*attggtatTCA |
| #10 | GCattg*g*t*atTCA | #14 | GCattggtatT*CA |
| #11 | GCattg*g*t*at*TCA | #15 | GCattggtatTC*A |
| #12 | GCattg*g*t*a*t*TCA | #16 | GC*attggtatT*C*A |
| Ref. | GCattggtatTCA | | |
| Compounds #1 - #16 and Ref. have the sequence motif shown in SEQ ID NO 1. | | | |
| Upper case letter: beta-D-oxy LNA nucleoside; lower case letter DNA nucleoside; | | | |
| * = achiral phosphorodithioatc modified linkages; all other linkages phosphorothioate | | | |

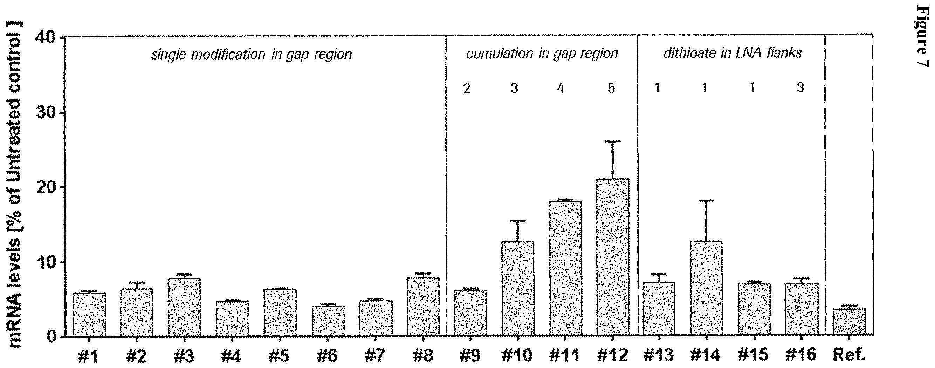

**Experimental:** The above compounds targeting ApoB mRNA, were tested in primary rat hepatocytes using gymnotic uptake, with incubation for 72 hrs with a compound concentration of 2 µM. The target mRNA levels were then measured using RT-PCR. Results are shown in Figure 7.

The results shown in figure 7 illustrate that both single and multiple achiral phosphorodithioates are accommodated in the gap and flank regions. The use of more than 3 or 4 achiral phosphorodithioates in the gap may tend to reduce potency as compared to the use of multiple achiral phosphorodithioates in the flank region.

### Example 8: Positional dependency on activity - design optimisation

### Compounds Tested

| *2 modifications* | | | |
|---|---|---|---|
| #1 | GCat*t*ggtatTCA | #6 | GCat*tggtat*TCA |
| #2 | GCattggt*at*TCA | #7 | GCa*ttggta*tTCA |
| #3 | GCat*tggt*atTCA | #8 | GCa*ttggtat*TCA |
| #4 | GCatt*ggt*atTCA | #9 | GCat*tggta*tTCA |
| #5 | GCatt*ggtat*TCA | | |

| *3 modifications* | | | *4 modifications* |
|---|---|---|---|
| #10 | GCat*tggt*at*TCA | #15 | GCat*t*ggt*at*TCA |
| #11 | GCat*t*ggtat*TCA | #16 | GCa*tt*gg*ta*tTCA |
| #12 | GCatt*ggta*t*TCA | | |
| #13 | GCat*t*ggt*atTCA | | |
| #14 | GCa*ttg*gta*tTCA | | |
| Ref. | GCattggtatTCA | | |
| Compounds #1 - #16 and Ref. have the sequence motif shown in SEQ ID NO 1. | | | |
| Upper case letter: beta-D-oxy LNA nucleoside; lower case letter DNA nucleoside; | | | |
| * = achiral phosphorodithioate modified linkages; all other linkages phosphorothioate | | | |

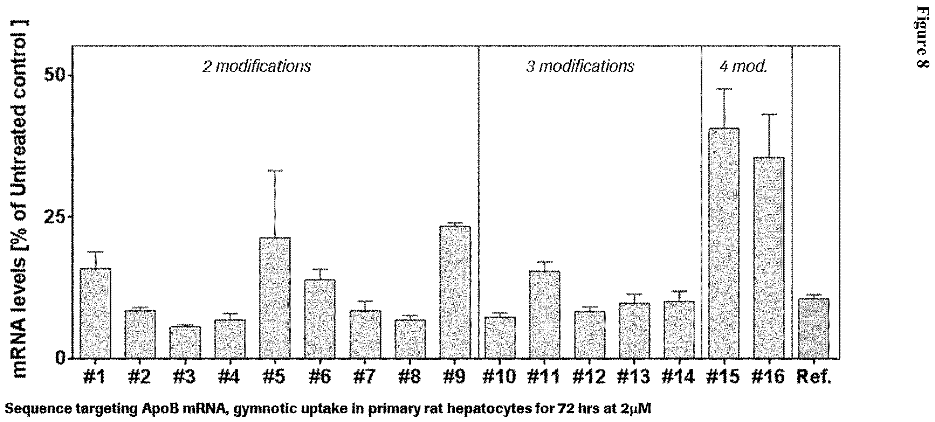

**Experimental:** The above compounds targeting ApoB mRNA, were tested in primary rat hepatocytes using gymnotic uptake, with incubation for 72 hrs with a compound concentration of 2µM. The target mRNA levels were then measured using RT-PCR. Results are shown in Figure 8.

### Example 9: Cellular Uptake of achiral phosphorodithioate gapmers

### Compounds Tested

| *single modification in the gap* | | | |
|---|---|---|---|
| #1 | GCa*ttggtatTCA | #5 | GCattgg*tatTCA |
| #2 | GCat*tggtatTCA | #6 | GCattggt*atTCA |
| #3 | GCatt*ggtatTCA | #7 | GCattggta*tTCA |
| #4 | GCattg*gtatTCA | #8 | GCattggtat*TCA |
| Ref. | GCattggtatTCA | | |

| | | | |
|---|---|---|---|
| Compounds #1 - #16 and Ref. have the sequence motif shown in SEQ ID NO 1. Upper case letter: beta-D-oxy LNA nucleoside; lower case letter DNA nucleoside; ∗= achiral phosphorodithioate modified linkages; all other linkages phosphorothioate | | | |

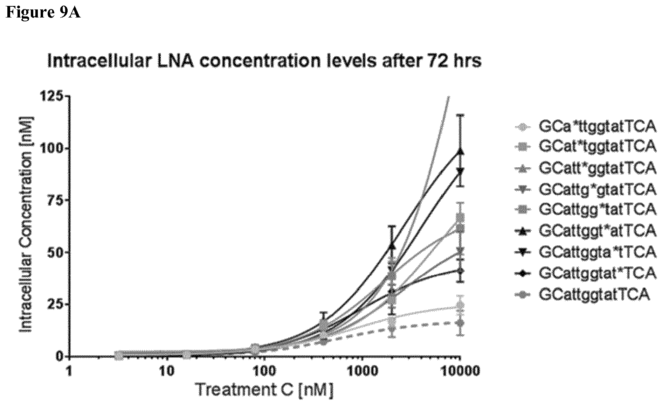

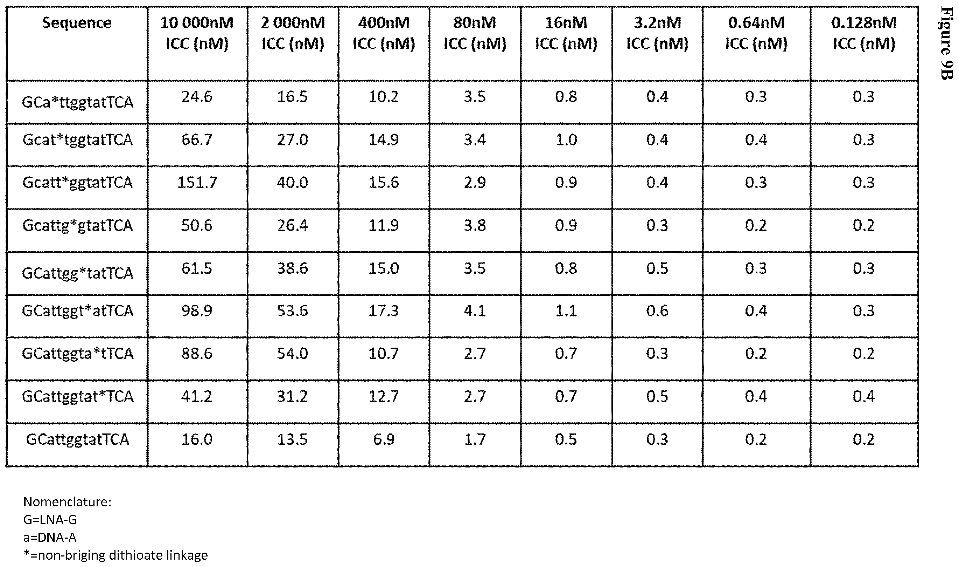

**Experimental:** The above compounds targeting ApoB mRNA, were tested in primary rat hepatocytes using gymnotic uptake, with incubation for 72 hrs with a compound concentration of 2µM. Oligonucleotide content was determined using a hybridization based ELISA assay. The results are shown in Figures 9A and 9B.

Without exception, the inclusion of an achiral phosphorodithioate provided enhanced cellular uptake. There was however a diversity in the uptake improvement depending upon the position of the achiral phosphorodithioate linkage.

### Example 10: Increasing the achiral phosphorodithioate load in the flank region of a gapmer

### Compounds Tested (Sequence motif= SEQ ID NO 1)

| *modifications in the flanks* | | | | | |
|---|---|---|---|---|---|
| | | IC50 [nM] | | | IC50 [nM] |
| ● | GCattggtatTC*A | 7.3 | ▼ | G*CattggtatT*C*A | 9.2 |
| ■ | G*CattggtatTCA | 10.4 | ◆ | G*C*attggtatTC*A | 8.9 |
| ▲ | G*CattggtatTC*A | 6.8 | ○ | G*C*attggtatT*C*A | 4.9 |
| Ref. | GCattggtatTCA | 33.3 | | | |

| | | | | | |
|---|---|---|---|---|---|
| Upper case letter: beta-D-oxy LNA nucleoside; lower case letter DNA nucleoside; ∗ = achiral phosphorodithioate modified linkages; all other linkages phosphorothioate | | | | | |

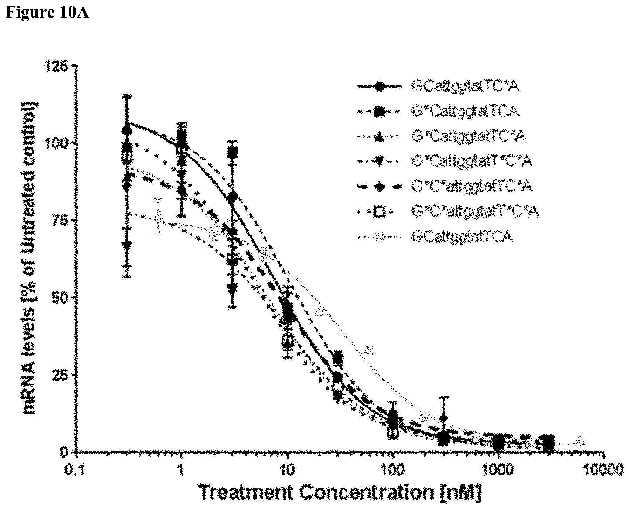

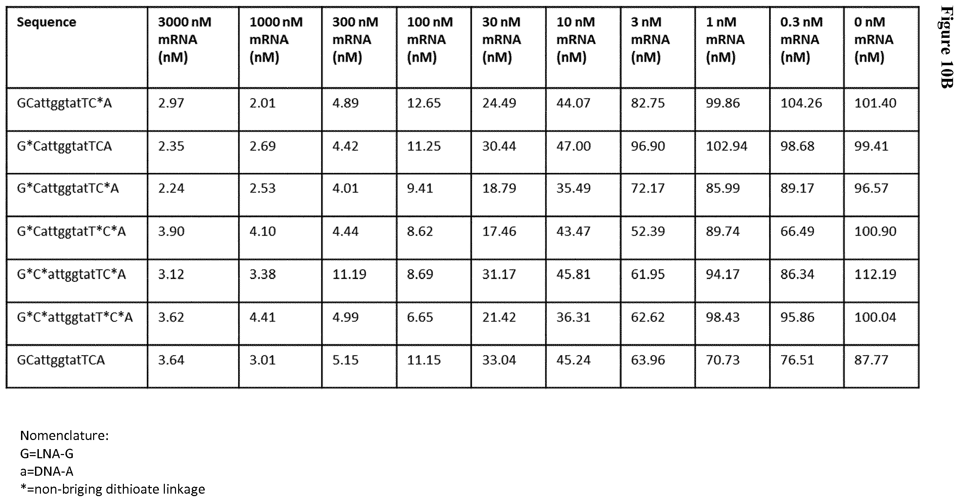

**Experimental:** The above compounds targeting ApoB mRNA, were tested in primary rat hepatocytes using gymnotic uptake, with incubation for 72 hrs with a compound concentration of 2µM. The target mRNA levels were then measured using RT-PCR. The results are shown in Figures 10A and 10B.

The introduction of achiral phosphorodithioate modifications in the flank regions of gapmers provided without exception a pronounced increase in potency, with a reduction in IC50 of 3 - 7 x. Interestingly, an increase in the number of chiral phosphorodithioate modifications in the flanks results in a lower IC50.

### Example 11: Effect of achiral phosphorodithioate linkages in different cell types, in vitro.

### Compounds Tested (Sequence motif = SEQ ID NO 3)

| *modification in the flanks* | | | |
|---|---|---|---|
| #1 | GAGttacttgccaAC*T | #5 | G*A*GttacttgccaAC*T |
| #2 | G*AGttacttgccaACT | #6 | G*A*GttacttgccaA*C*T |
| #3 | G*AGttacttgccaAC*T | #7 | G*A*G*ttacttgccaA*C*T |
| #4 | G*AGttacttgccaA*C*T | | |
| Ref. | GAGttacttgccaACT | | |

| | | | |
|---|---|---|---|
| Upper case letter: beta-D-oxy LNA nucleoside; lower case letter DNA nucleoside; * = achiral phosphorodithioate modified linkages; all other linkages phosphorothioate | | | |

The above compounds which traget Malat-1 were tested in three in vitro cell systems: human primary skeletal muscles, human primary bronchial epithelial cells and mouse fibroblasts (LTK cells) using gymnotic uptake for 72 hours, at a range of concentrations to determine the compound potency (IC50).

Concentration range for LTK cells: 50µM, ½log dilution, 8 concentrations.

RNA levels of Malat1 were quantified using qPCR (Normalised to GAPDH level) and IC50 values were determined.

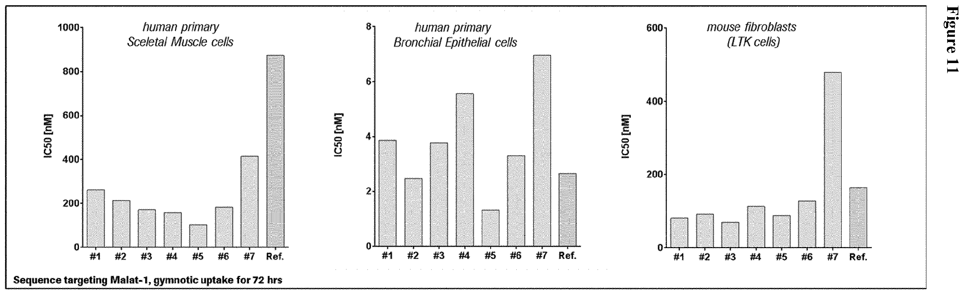

The IC50 results are shown in figure 11. The introduction of achiral phosphorodithioate provided a reliable enhanced potency in skeletal muscle cells, and in general gave an improved potency into mouse fibroblasts. The effect in human bronchial epithelial cells was more compound specific, however in some compounds (#5) were markedly more potent than the reference compound.

### Example 12: In vitro rat serum stability of 5' and 3' end protected LNA oligonucleotides.

### Compounds Tested (Sequence motif = SEQ ID NO 1)

| | |
|---|---|
| #1 | PS/DNA oligonucleotide |
| #2 | GCattggtatTCA |
| #3 | G*CattggtatTCA |
| #4 | GCattggtatTC*A |
| #5 | G*CattggtatTC*A |
| #6 | G*CattggtatT*C*A |
| #7 | G*C*attggtatTC*A |
| #8 | G*C*attggtatT*C*A |

| | |
|---|---|
| Upper case letter: beta-D-oxy LNA nucleoside; lower case letter DNA nucleoside; * = achiral phosphorodithioate modified linkages; all other linkages phosphorothioate | |

### Experimental - see example 5.

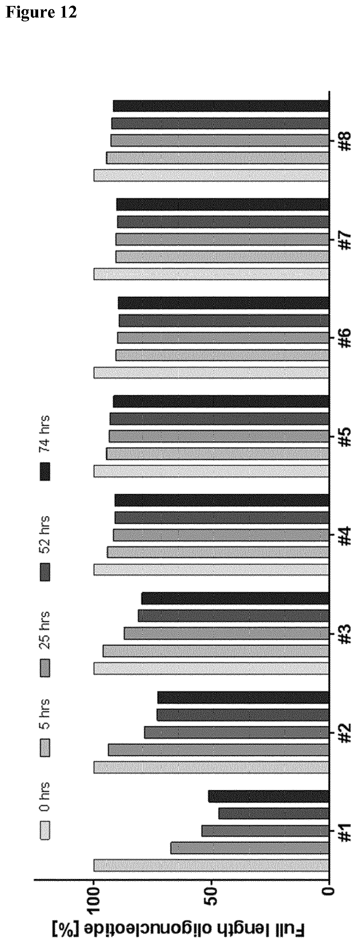

The results are shown in figure 12. We have identified that the 3' end of LNA phosphorothioate oligonucleotides are more susceptible to serum nucleases than previously thought and this appears to be related to the chirality of the phosphorothioate linkage(s) at the 3' end of the oligonucleotide - as illustrated by the rapid cleavage of 50% of the parent oligonucleotide #1. The 5' end protection with an achiral phosphorodithioate provided an improved protection. The 3' end protection with an achiral phosphorodithioate provided complete protection to rat serum exonucleases - the slight reduction seen for compound #4 - #8 was correlated to a monothioate impurity.

The 5' and/or 3' end protection of antisense oligonucleotides with the achiral phosphorothioate linkages is therefore considered to provide a solution to a major instability problem with stereorandom and stereodefined phosphorothioates.

### Example 13: In vivo assessment of gapmers with achiral phosphorodithioate linkages in the flanks.

### Compounds Tested (Sequence motif = SEQ ID NO 1)

| | |
|---|---|
| #1 | GCattggtatTC*A |
| #2 | G*CattggtatTCA |
| #3 | G*CattggtatTC*A |
| #4 | G*CattggtatT*C*A |
| #5 | G*C*attggtatTC*A |
| #6 | G*C*attggtatT*C*A |
| #7 | G*C***attggtat**T*C*A |
| #8 | GCat*tggt*at*TCA |
| #9 | **GCa**t***tgg**t***a**t***TC**A |
| Ref. | GCattggtatTCA |

| | |
|---|---|
| Upper case letter: beta-D-oxy LNA nucleoside; lower case letter DNA nucleoside; * = achiral phosphorodithioate modified linkages; all other linkages phosphorothioate. Note the underlined bold nucleosides are linked at the 3' position by stereodefined phosphorothioate internucleoside linkages. Compound #7 has a stereodefined motif in the gap region of SSRSSRSR (S = Sp, R = Rp). The backbone motif of compound #9 = RRSPRSSPSPSS, wherein S = Sp, R = Rp, and P = achiral PS2 linkage (*). | |

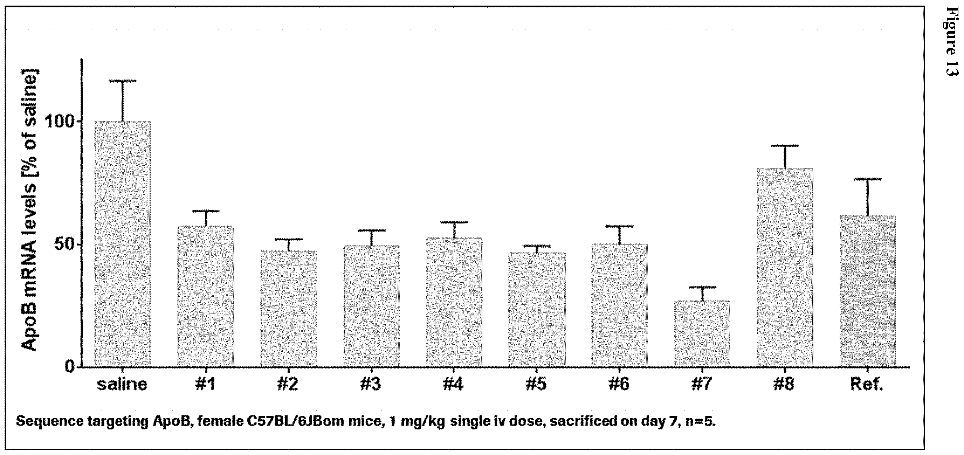

Experimental: The above compounds targeting ApoB were administered to female C57BL/6JBom mice, using a1 mg/kg single iv dose, and were sacrificed on day 7, n=5. The mRNA reduction in the liver was measured using RT-PCR and the results are shown in Figure 13.

The results show that in general the introduction of the achiral phosphorodithioate internucleoside linkages provides an improved potency, notably all the compounds with achiral phosphorodithioate linkages in the flank regions show improved potency. As illustarted in the in vitro experiment, the use of multiple phosphorodithioate linkages in the agap region (#8) was accomodated without a notable loss of potency. Of particular interest is the combined effect of gapmer designs with stereodefined phosphorothioate linkages in the gap region, with achiral phosphorodithioate linkages in the flanks, illustrating a synergy in combining these linkages technologies with an antisense oligonucelotide.

### Example 14: In vivo tissue content in liver of gapmers with achiral phosphorodithioates with modified flanks and gap region.

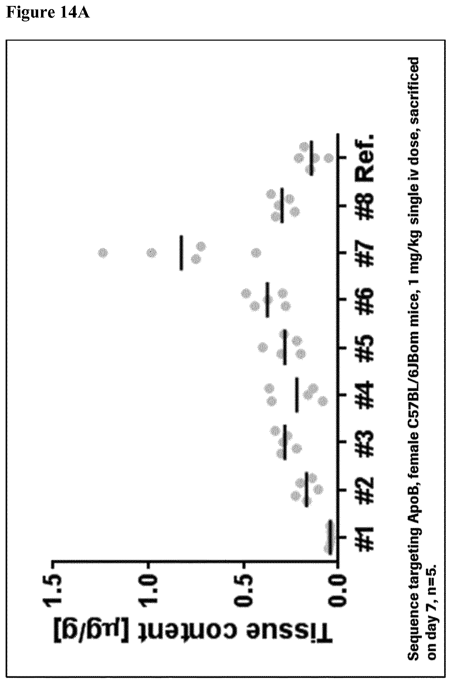

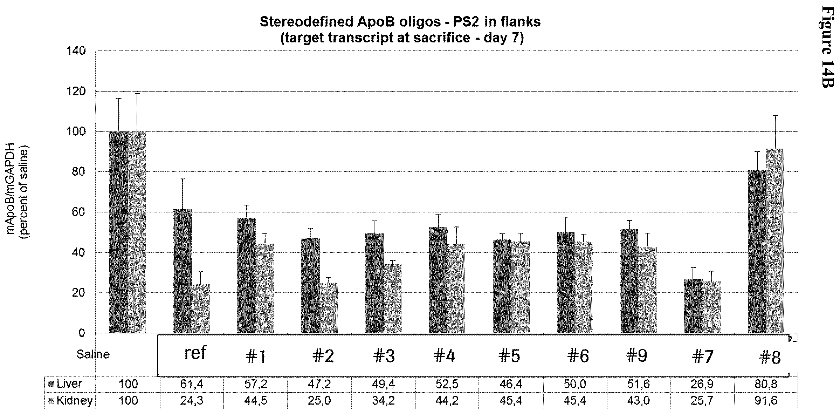

Compounds and experimental - see example 13. The results of the tissue content (determined by hybridsation based ELISA to measure content in liver and kidney samples from the sacrificed animals) is shown in Figure 14A & B. Note that there was an experimental error for compound #1 - see Figure 14B data.

Results: Figure 14A. All the antisense oligonucleotides which contained the achiral phosphorodithioate linkages had a higher tissue uptake/content as compared to the reference compound. Figure 14B shows that the introduction of the achiral phosphorodithioate linkage enhanced the biodistribution (as determined by the liver/kidney ratio) of all the compounds tested.

### Example 15: Metabolite anaysis from in vivo experiment

Compounds and experimental - see example 13. Metabolite analysis was performed using the methods disclosed in C. Husser et al., Anal. Chem. 2017, 89, 6821.

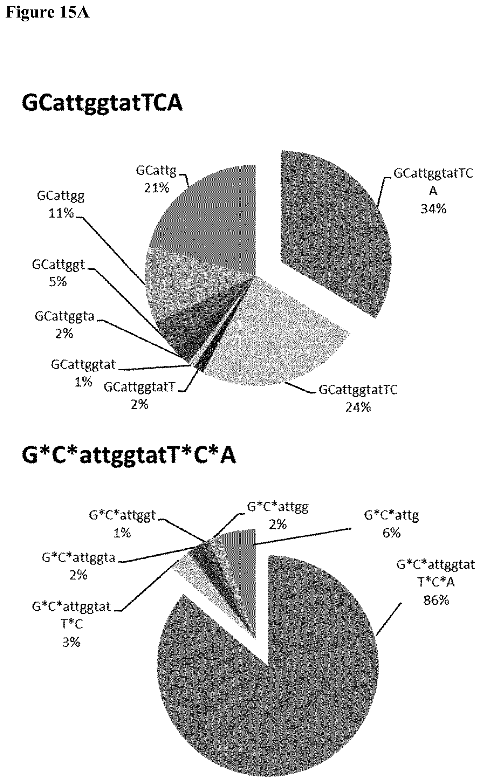

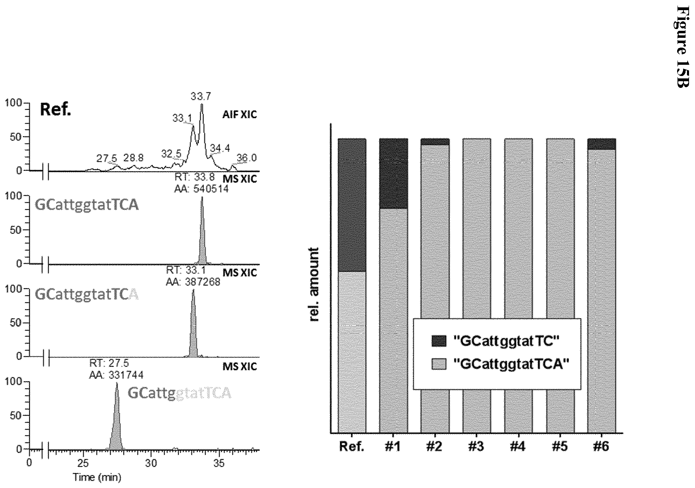

The results are shown in Figures 15A and 15B. The phosphorodithioate modification efficiently prevents 3'-exonucleolytic degradation in vivo. There remains some endonuclease cleavage (note compounds #1-6 tested all have DNA phosphorothioate gap regions so this was expected). Given the remarkable exonuclease protection it is considered that the use of achiral phosphorodithioate linkages within antisense oligonucleotides may be used to prevent or limit endonuclease cleavage. The enhanced nuclease resistance of achiral phosphordithioates is expected to provide notable pharmacological benefits, such as enhanced activity and prolonged duration of action, and possibly avoidance of toxic degradation products.

### Example 16: In vivo - Long term liver activity (ApoB)

### Compounds tested (Sequence motif = SEQ ID NO 1):

| Ref. | GCattggtatTCA |
|---|---|
| #1 | G*C*attggtatT*C*A |
| #2 | **GCattggtatTCA** |
| #3 | G*C***attggtat**T*C*A |

| | |
|---|---|
| Upper case letter: beta-D-oxy LNA nucleoside; lower case letter DNA nucleoside; * = achiral phosphorodithioate modified linkages; all other linkages phosphorothioate. Note the underlined bold nucleosides are linked at the 3' position by stereodefined phosphorothioate internucleoside linkages. Compound #3 has a stereodefined motif in the gap region of SSRSSRSR (S = Sp, R = Rp). The backbone motif of compound #2 = RRSSRSSRSRSS, wherein S = Sp, R = Rp, and P = achiral PS2 linkage (*). | |

### Experimental: As in example 13, however sacrifice was performed at day 7 or 21.

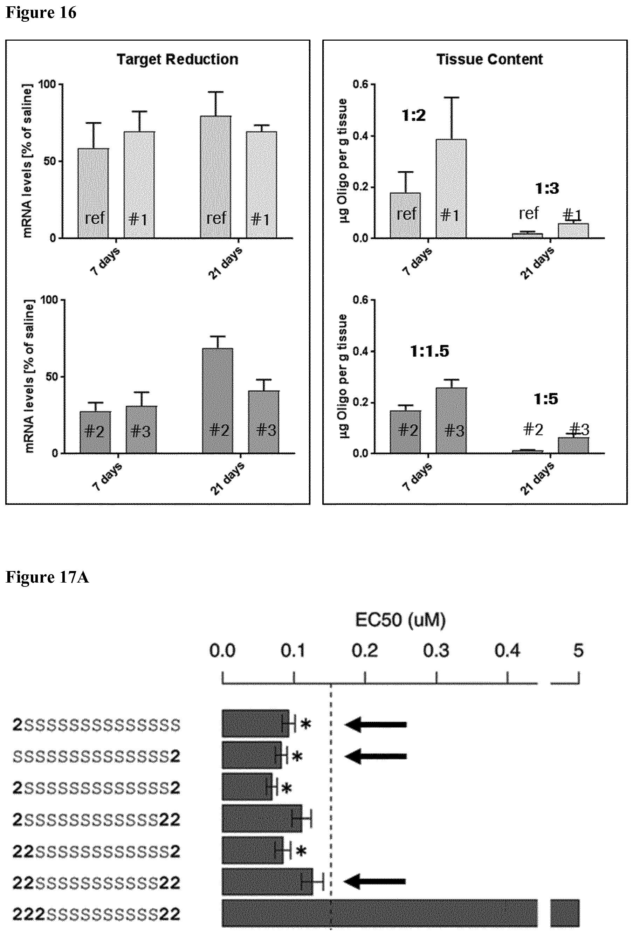

The results are shown in Figure 16. Compared to the phosphorothioate reference compound, the introduction of the achiral phosphorodithioate provided a prolonged duration of action in the liver and this was correlated with a higher tissue content at 21 days. Notably, the combination of phosphorodithioate linked flank regions with stereodefined phosphorothioate linkages in the gap region provided further benefit with regards to prolonged potency and duration of action, again emphasising the remarkable synergy in combining achiral phosphorodithioate internucleoside linkages with stereodefined phosphorothioate linkages in antisense oligonucleotides.

### Example 17: In vivo study using Malat-1 targeting achiral phosphorodithioates modified gapmers.

### Compounds Tested (Sequence motif = SEQ ID NO 3)

| Ref. | GAGttacttgccaACT |
|---|---|
| Increasing P2S load in flanks | |
| #1 | G*AGttacttgccaACT |
| #2 | GAGttacttgccaAC*T |
| #3 | G*AGttacttgccaAC*T |
| #4 | G*AGttacttgccaA*C*T |
| #5 | G*A*GttacttgccaAC*T |
| #6 | G*A*GttacttgccaA*C*T |
| #7 | G*A*G*ttacttgccaA*C*T |

| | |
|---|---|
| Upper case letter: beta-D-oxy LNA nucleoside; lower case letter DNA nucleoside; * = achiral phosphorodithioate modified linkages; all other linkages phosphorothioate. | |

### Experimental:

In vitro: Mouse LTK cells were used to determined the in vitro concentration dose response curve - measuring the MALAT-1 mRNA inhibition.

**In vivo:** Mice (C57/BL6) were administered 15mg/kg dose subcutaneously of the oligonucleotide in three doses on day 1, 2 and 3 (n=5). The mice were sacrificed on day 8, and MALAT-1 RNA reduction and tissue content was measured for liver, heart, kidney, spleen and lung. The parent compounds was administered in two doses 3*15mg/kg and 3*30mg/kg.

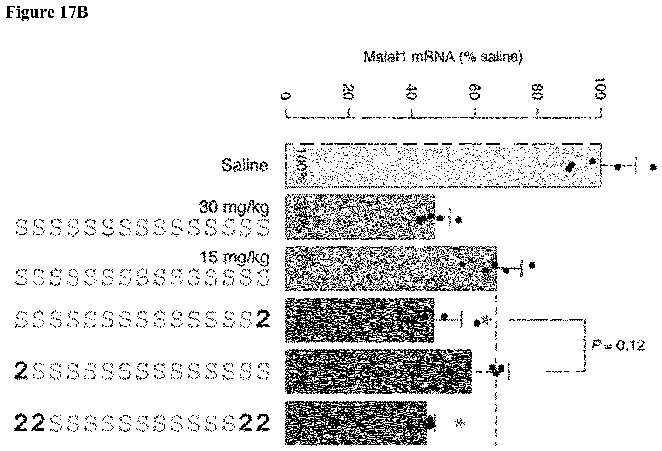

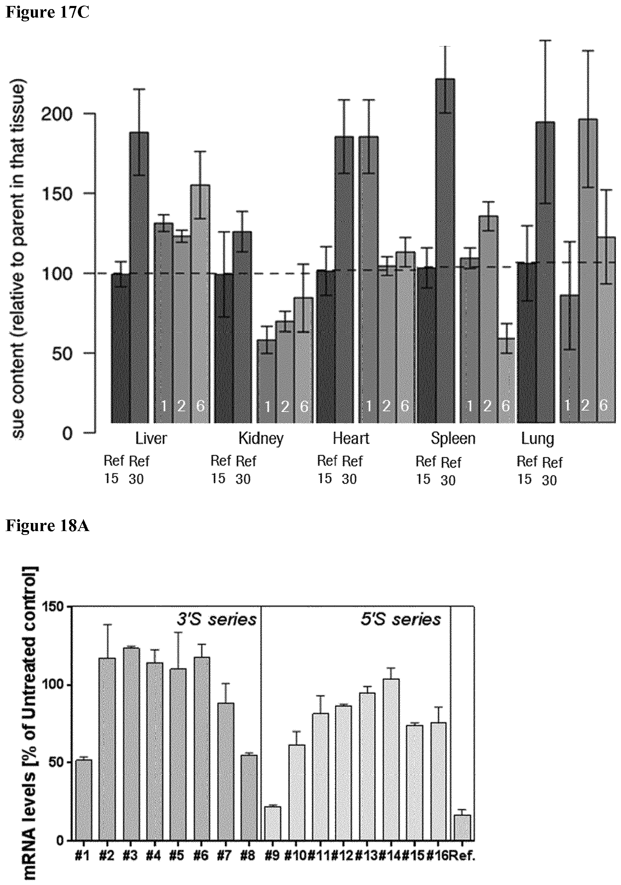

**Results:** The in vitro results are shown in Figure 17 - compounds with 1, 2, 3 and 4 achiral phosphorodithioates in the flanks were found to be highly potent in vitro. The compound #7 with 5 achiral phosphorodithioates inthe flanks was found to have a lower potency than those with 1 - 4 achiral phosphorodithioates in the flanks. The most potent compounds #1, #2 and #6 were selected for the in vivo study. The in vivo results are shown in Figure 17B (heart) - which illustrates that the achiral phosphorodithioate compounds were about twice as potent in knocking down MALAT-1 in the heart as the reference compound. Notably the use of the achiral phosphorodithioate internucleoside linkage between the two 3' terminal nucleosides of the antisense oligonucleotides provided a marked improvement over the equivalent 5' end protected oligonucelotide.

Figure 17C shows the results of the tissue content analysis from the in vivo study. All three oligonucleotide containing the achrial phosphorodithioate internucleoside linkages had higher tissue content in liver. The di-thioates results in similar or higher content in heart and liver, and lower content in kidney, again illustrating superiority over PS-modified antisense oligonucleotides. Notably the tissue content in heart was only higher for compound 1, indicating that the enhanced in vivo potency may not be a consequence of the tissue content, but a higher specific activity.

### Example 18: Achiral monophosphothioate modifications tested do not provide the portable benefits seen with achiral phosphorodithioate linkages.

### Compounds Tested (Sequence motif = SEQ ID NO 1)

| | | | |
|---|---|---|---|
| #1 | GCa^{▪}ttggtatTCA | #9 | GC^{†}attggtatTCA |
| #2 | GCat^{▪}tggtatTCA | #10 | GCa^{†}ttggtatTCA |
| #3 | GCatt^{▪}ggtatTCA | #11 | GCa^{†}ttggtatTCA |
| #4 | GCattg^{▪}gtatTCA | #12 | GCa^{†}ttggtatTCA |
| #5 | GCattgg^{▪}tatTCA | #13 | GCattg^{†}gtatTCA |
| #6 | GCattggt^{▪}atTCA | #14 | GCattgg^{†}tatTCA |
| #7 | GCattggta^{▪}tTCA | #15 | GCattggt^{†}atTCA |
| #8 | GCattggtat^{▪}TCA | #16 | GCattggta^{†}tTCA |
| Ref. | GCattggtatTCA | | |

| | | | |
|---|---|---|---|
| Upper case letter: beta-D-oxy LNA nucleoside; lower case letter DNA nucleoside; ^{▪} = 3'-S phosphorothioate linkage, all other linkages are phosphorothioate. ^{†} = 5'-S phosphorothioate linkage, all other linkages are phosphorothioate. | | | |

In this study we synthesised a series of 3' or 5' S modified phosphorothioates oligonucelotide gapmers targeting ApoB - the positioning of the sulfur in the backbone linkages results in an achiral internucleoside linkage. For synthesis methods see WO2018/019799.

The compounds were tested in vitro as previously described - e.g. see example 8.

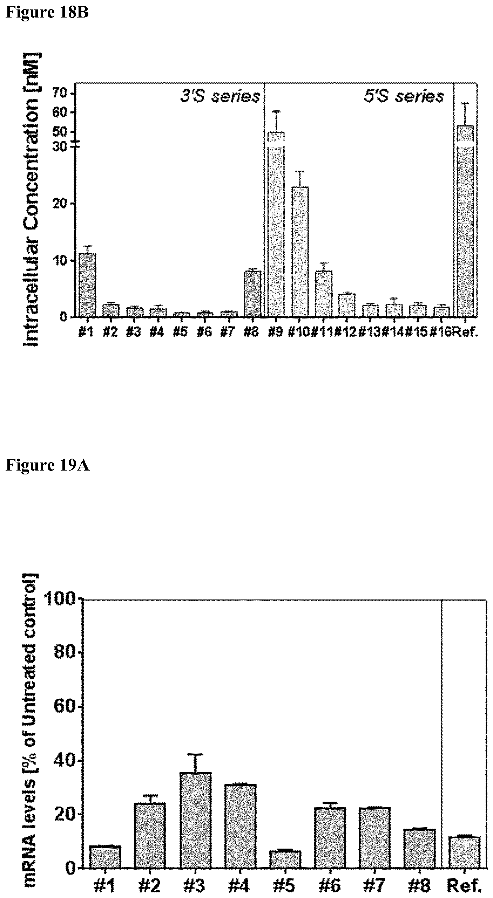

The results are shown in figure 18A: The results show that in general the achiral monophosphorothioates were detremental to potency oft he compounds, although in some instances the compounds retained potency. This appears to correlate with the cellular content (figure 18B).

### Reference Example 19: Chiral phosphorodithioate modications can provide benefits to antisense oligonucelotide gapmers.

### Compounds Tested (Sequence motif = SEQ ID NO 1)

| | |
|---|---|
| #1 | GCa^{◆}ttggtatTCA |
| #2 | GCat^{◆}tggtatTCA |
| #3 | GCatt^{◆}ggtatTCA |
| #4 | GCattg^{◆}gtatTCA |
| #5 | GCattgg^{◆}tatTCA |
| #6 | GCattggt^{◆}atTCA |
| #7 | GCattggta^{◆}tTCA |
| #8 | GCattggtat^{◆}TCA |
| Ref. | GCattggtatTCA |

| | |
|---|---|
| Upper case letter: beta-D-oxy LNA nucleoside; lower case letter DNA nucleoside; ^{◆} = chiral phosphorodithioate linkage, all other linkages are phosphorothioate. | |

In this study we synthesised a series of stereorandom chiral phosphorodithioates oligonucelotide gapmers targeting ApoB - the positioning of the sulfur in the backbone linkages results in an chiral internucleoside linkage.

### The compounds were tested in vitro as previously described - e.g. see example 8.

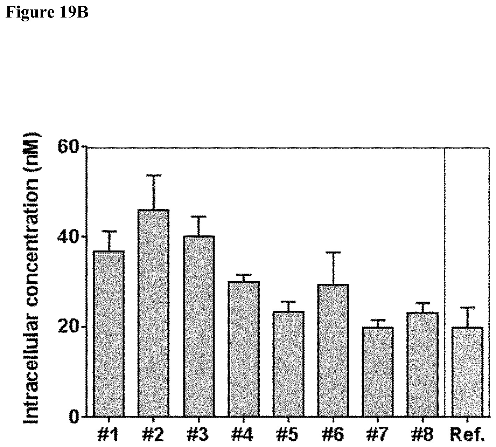

The results are shown in figure 19A: The results show that in some positions the chiral phosphorodithioate compounds were as potent as the reference compound, indicating the chiral phosphorodithioate was not incompatible with antisense functionality - however the benefit was compound specific (i.e. does not appear portable). A similar picture is seen with regards to cellular uptake (Figure 19B), although there does not appear to be a correlation between antisense activity and cellular uptake.

### Example 20: In vivo study using Htra-1 targeting achiral phosphorodithioates modified gapmers.

### Compounds Tested

All compounds have the sequence: TATttacctggtTGTT (SEQ ID NO 4), wherein capital letters are beta-D-oxy LNA nucleosides, lowercase letters are DNA nucleosides. In the following table, the backbone motif represents the pattern of backbone modifications for each internucleoside linkage starting at the linkage between the 5' dinucleotide, and finishing with the internucleoside linkage between the 3' dinucleotide (left to right). X = stereorandom phosphorothioate internucleoside linkage, P = achiral phosphorodithioate (*), S = Sp stereodefined phosphorothioate internucleoside linkage, R = Rp stereodefined phosphorothioate internucleoside linkage.

| | | |
|---|---|---|
| Htra1#Parent | TATttacctggtTGTT | XXXXXXXXXXXXXXX |
| Htra1#1 | TATttacctggtTGTT | XXXPXXXXXXXXXXX |
| Htra1#2 | TATttacctggtTGTT | XXXXPXXXXXXXXXX |
| Htra1#3 | TATttacctggtTGTT | XXXXXPXXXXXXXXX |
| Htra1#4 | TATttacctggtTGTT | XXXXXXPXXXXXXXX |
| Htra1#5 | TATttacctggtTGTT | XXXXXXXPXXXXXXX |
| Htra1#6 | TATttacctggtTGTT | XXXXXXXXPXXXXXX |
| Htra1#7 | TATttacctggtTGTT | XXXXXXXXXPXXXXX |
| Htra1#8 | TATttacctggtTGTT | XXXXXXXXXXPXXXX |
| Htra1#9 | TATttacctggtTGTT | XXXXXXXXXXXPXXX |
| Htra1#10 | TATttacctggtTGTT | XXXXPPPPXXXXXXX |
| Htra1#11 | TATttacctggtTGTT | XXXXXXPPPPXXXXX |
| Htra1#12 | TATttacctggtTGTT | XXXXXXXXPPPPXXX |
| Htra1#13 | TATttacctggtTGTT | PSRRRSSSRRRRRRP |
| Htra1#14 | TATttacctggtTGTT | PSRRRSSSRRRRPXP |
| Htra1#15 | TATttacctggtTGTT | PRRRRSSSSRRRRSP |
| Htra1#16 | TATttacctggtTGTT | PRRRRSSSSRRRRSS |
| Htra1#17 | TATttacctggtTGTT | RRRRRSSSSRRRRSP |
| Htra1#18 | TATttacctggtTGTT | PXPRRSSSSRRRPXP |
| Htra1#19 | TATttacctggtTGTT | PXXRRSSSSRRRXXP |
| Htra1#20 | TATttacctggtTGTT | PXXXXXXXXXXXXXX |
| Htra1#21 | TATttacctggtTGTT | XXPXXXXXXXXXXXX |
| Htra1#22 | TATttacctggtTGTT | XXXXXXXXXXXXPXX |
| Htra1#23 | TATttacctggtTGTT | XXXXXXXXXXXXXXP |
| Htra1#24 | TATttacctggtTGTT | PXXXXXXXXXXXXXP |
| Htra1#25 | TATttacctggtTGTT | PXPXXXXXXXXXPXP |
| Htra1#26 | TATttacctggtTGTT | XPXXXXXXXXXXXXX |
| Htra1#27 | TATttacctggtTGTT | PPPXXXXXXXXXPXP |
| Htra1#28 | TATttacctggtTGTT | PXXXXXXXXXXXXPP |
| Htra1#29 | TATttacctggtTGTT | PPXXXXXXXXXXXXP |
| Htra1#30 | TATttacctggtTGTT | PPXXXXXXXXXXXPP |
| Htra1#31 | TATttacctggtTGTT | PPXXXXXXXXXXPPP |
| Htra1#32 | TATttacctggtTGTT | PPPXXXXXXXXXXPP |
| Htra1#33 | TATttacctggtTGTT | PPPXXXXXXXXXPPP |
| Htra1#34 | TATttacctggtTGTT | PSPRRSSSSRRRPRP |
| Htra1#35 | TATttacctggtTGTT | PSPRRSSSSRRRPSP |
| Htra1#36 | TATttacctggtTGTT | PRPRRSSSSRRRPSP |
| Htra1#37 | TATttacctggtTGTT | PRPRRSSSSRRRPRP |
| Htra1#38 | TATttacctggtTGTT | PPPRRSSSSRRRPPP |

### Experimental:

Human glioblastoma U251 cell line was purchased from ECACC and maintained as recommended by the supplier in a humidified incubator at 37°C with 5% CO₂. For assays, 15000 U251 cells/well were seeded in a 96 multi well plate in starvation media (media recommended by the supplier with the exception of 1% FBS instead of 10%). Cells were incubated for 24 hours before addition of oligonucleotides dissolved in PBS.

Concentration of oligonucleotides: 5, 1 and 0.2 µM. 4 days after addition of oligonucleotides, the cells were harvested. RNA was extracted using the PureLink Pro 96 RNA Purification kit (Ambion, according to the manufacturer's instructions). cDNA was then synthesized using M-MLT Reverse Transcriptase, random decamers RETROscript, RNase inhibitor (Ambion, according the manufacturer's instruction) with 100mM dNTP set PCR Grade (Invitrogen) and DNase/RNase free Water (Gibco). For gene expressions analysis, qPCR was performed using TagMan Fast Advanced Master Mix (2X) (Ambion) in a doublex set up. Following TaqMan primer assays were used for qPCR: HTRA1, Hs01016151_ m1 (FAM-MGB) and house keeping gene, TBP, Hs4326322E (VIC-MGB) from Life Technologies. EC50 determinations were performed in Graph Pad Prism6.The relative HTRA1 mRNA expression level in the table is shown as % of control (PBS-treated cells).

### Results:

| | mRNA level remaining at varions doses | | | | Potency | Max Efficacy |
|---|---|---|---|---|---|---|
| | 5 µM | 1 µM | 0.2 µM | 1 µM | EC50 [µM] | mRNA level [% of ctrl] |
| Htra1#Parent | 18 | 38 | 116 | 58 | 1,16 | 5,9 |
| Htra1#1 | 34 | | | | | |
| Htra1#2 | 50 | | | | | |
| Htra1#3 | 28 | | | | | |
| Htra1#4 | 44 | | | | | |
| Htra1#5 | 39 | | | | | |
| Htra1#6 | 47 | | | | | |
| Htra1#7 | 41 | | | | | |
| Htra1#8 | 44 | | | | | |
| Htra1#9 | 47 | | | | | |
| Htra1#10 | 36 | | | | | |
| Htra1#11 | 53 | | | | | |
| Htra1#12 | 36 | | | | | |
| Htra1#13 | 11 | 57 | 97 | | | |
| Htra1#14 | 3 | 18 | 83 | | | |
| Htra1#15 | 3 | 18 | 85 | | | |
| Htra1#16 | 4 | 24 | 85 | | | |
| Htra1#17 | 3 | 19 | 108 | | | |
| Htra1#18 | 2 | 10 | 76 | | 0,15 | 4,0 |
| Htra1#19 | 4 | 35 | 90 | | 0,44 | 3,7 |
| Htra1#20 | 25 | 87 | 96 | 57 | | |
| Htra1#21 | 22 | 73 | 78 | | | |
| Htra1#22 | 24 | | 100 | | | |
| Htra1#23 | 20 | 50 | 117 | 53 | 0,91 | 9,0 |
| Htra1#24 | 5 | 51 | 136 | 44 | | |
| Htra1#25 | 3 | 27 | 69 | | | |
| Htra1#26 | 27 | 72 | 93 | | | |
| Htra1#27 | 7 | 30 | 99 | | 0,35 | 4,7 |
| Htra1#28 | | | | 67 | | |
| Htra1#29 | | | | 55 | | |
| Htra1#30 | | | | 56 | | |
| Htra1#31 | | | | 54 | | |
| Htra1#32 | | | | 61 | | |
| Htra1#33 | | | | 54 | | |
| Htra1#34 | | | | 54 | 0,78 | 5,1 |
| Htra1#35 | | | | 20 | 0,17 | 3,6 |
| Htra1#36 | | | | 15 | 0,13 | 3,1 |
| Htra1#37 | | | | 42 | 0,69 | 3,9 |
| Htra1#38 | | | | 24 | 0,23 | 4,2 |

### Example 21: A PS2 walk on a LNA Mixmer targeting TNFRSF1B exon 7 skipping

We have previously identified that the skipping of *TNFRSF1B* exon 7 using a mixmer (13'mer) SSO#26 - is highly effective in targeting the 3' splice site of intron 6 - exon 7 of TNFRSF1B (see WO2008131807 & WO2007058894 for background information).

This experiment was established to determine whether the presence of phosphorodithioate linkages of formula (IA) or (IB) (PS2) can be useful in further enhancing the splice modulation activity of splice switching oligonucleotides. To determine the effect, we introduced phosphorodithioates linkages of formula (IA) or (IB) in different positions of the parent oligonucleotide SSO#26 and synthesized the following compounds (Table below).

Compounds tested: Dithioate modified oligonucleotides of the parent oligonucleotide (SSO#26). Phosphorodithioate internucleoside linkages of formula ((IA) or (IB)) were introduced in positions marked with a *, all other internucleoside linkages are phosphorothioate internucleoside linkages (stereorandom), capital letters represent beta-D-oxy LNA nucleosides, and LNA C are 5-methyl-cytosine, lower case letters represent DNA nucleosides.

| | Compounds (Sequence motif = SEQ ID NO 5) | | Compounds |
|---|---|---|---|
| SSO#1 | CAaT*cAG*tcCtA*G | SSO#14 | CAaTcAGtcCt*AG |
| SSO#2 | CAaTcAG*tcCtA*G | SSO#15 | CAaTcAGtcCtA*G |
| SSO#3 | C *AaTcAGtcC *tAG | SSO#16 | C*A*aT*cA*G*tcC*tA*G |
| SSO#4 | C*AaTcAGtcCtAG | SSO#17 | C*AaT*cAG*tcC*tA*G |
| SSO#5 | CA*aTcAGtcCtAG | SSO#18 | C*A*aTc*A*GtcCt*A*G |
| SSO#6 | CAa*TcAGtcCtAG | SSO#19 | C*A*aTcAG*t*cCt*A*G |
| SSO#7 | CAaT*cAGtcCtAG | SSO#20 | C*A*a*T*cA*G*t*cCtAG |
| SSO#8 | CAaTc*AGtcCtAG | SSO#21 | CAaTcA*G*t*cCt*A*G |
| SSO#9 | CAaTcA*GtcCtAG | SSO#22 | CAa*Tc*AGt*c*Ct*AG |
| SSO#10 | CAaTcAG*tcCtAG | SSO#23 | CAa*Tc*AGt*cCt*AG |
| SSO#11 | CAaTcAGt*cCtAG | SSO#24 | CAa*TcAGt*c*Ct*AG |
| SSO#12 | CAaTcAGtc*CtAG | SSO#25 | CAa*Tc*AGtc*CtAG |
| SSO#13 | CAaTcAGtcC*tAG | SSO#26 | CAaTcAGtcCtAG |

### Experimental:

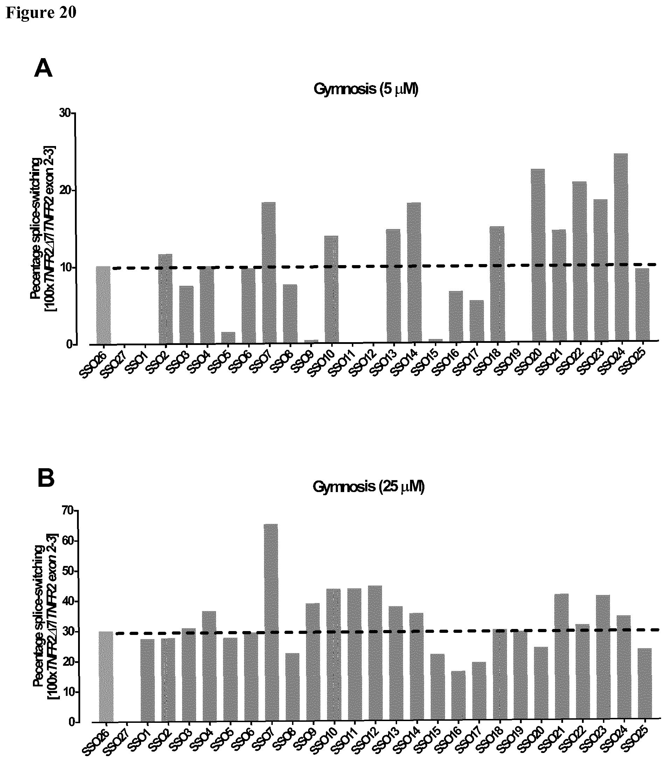

Oligonucleotide uptake and exon skipping in Colo 205 cells (human colorectal adenocarcinoma) was analyzed by gymnotic uptake at two different concentrations (5 µM and 25 µM). Cells were seeded in 96 well plates (25,000 cells per well) and the oligonucleotide added. Three days after addition of oligonucleotides, total RNA was isolated from 96 well plates using Qiagen setup. The percentage of splice-switching was analyzed by droplet digital PCR (BioRad) with a FAM-labelled probe spanning the exon 6-8 junction (exon 7 skipping) and the total amount of *TNFRSF1B* (wild type and exon 7 skipped) was analyzed with a HEX-labelled probe and primers from IDT spanning exon 2-3. The presence of a phosphorodithioate linkage has an effect on the ability of an oligonucleotide to introduce exon skipping (figure 20). At 5 µM, the most potent PS2 oligonucleotide increases the exon skipping by more than two fold, where the parent (SSO#26) shows approximately 10% exon skipping, SSO#25 shows more than 20% exon 7 skipping. At 25 µM, the most potent oligonucleotide reaches more than 60% exon skipping (SSO#7), again more than 2 fold better than the parent. Oligonucleotide SSO#22, in which all DNA nucleotides have a dithioate modification (PS2) instead of the phosphorothioate modification (PS) shows increased activity, compared to the parent, and is the third most potent oligonucleotide at 5 µM, and second most potent splice switching oligonucleotide at 25 µM (figure 20). Exchanging all linkages between LNA nucleosides with a PS2 linkage (SSO#16) however reduced the potency in splice switching compared to the parent oligonucleotide (figure 20). Furthermore, it is clear that introducing a PS2 at certain positions, may not be beneficial for the exon skipping activity and at 5 µM, SSO#1, SSO#9, SSO#11, SSO#12 and SSO#14 do not show significant splice switching activity at the lower concentration, but all were effective at the higher concentration (Figure 20). This examples illustrate that the PS2 linkage is compatible with splice modulating oligonucleotides and further emphasizes a clear benefit in introducing PS2 linkages adjacent to DNA nucleosides, or between adjacent DNA nucleosides, within the mixmer oligonucleotide, such as LNA mixmers - these designs were notably more effective in modulating splicing.

### Materials and methods

Assay to detect *TNFRSF1B* exon 7 skipping by droplet digital PCR
**Forward sequence:** CAACTCCAGAACCCAGCACT (SEQ ID NO 6)
**Reverse sequence:** CTTATCGGCAGGCAAGTGAG (SEQ ID NO 7)
**Probe Sequence:** GCACAAGGGCTTCTCAACTGGAAGAG (SEQ ID NO 8)
**Fluorophore:** FAM

Assay to detect total amount of *TNFRSF1B*
IDT assay **Hs.PT.58.40638488 spanning exon 2-3**

### Example 22: The stability of mixmer oligos containing phosphorodithioates modifications

Three dithioate modified oligonucleotides of the parent **(SSO#26)** were selected for stability assay using S1 nuclease (table 2). The selected oligonucleotides were incubated at 37°C at 25 µM for either 30 min or 2h in 100 µL reaction buffer containing 1× S1 Nuclease buffer, and 10U of S1 nuclease according to manufacturer's instruction (Invitrogen, Catalogue no. 18001-016). The S1 nuclease reaction was stopped by adding 2 µL of 500 mM EDTA solution to the 100 µL reaction mixture. 2.5 µL of the reaction mixture was diluted in Novex ^{™} TBE-Urea 2x sample buffer (LC6876 Invitrogen) and loaded onto Novex ^{™} 15% TBE-Urea gels (EC6885BOX, Invitrogen). The gels were run for approximately 1 hour at 180 V, afterwards gel images were acquired with SYBR gold staining (S11494, Invitrogen) and the ChemiDoc^{™} Touch Imaging System (BIO-RAD).

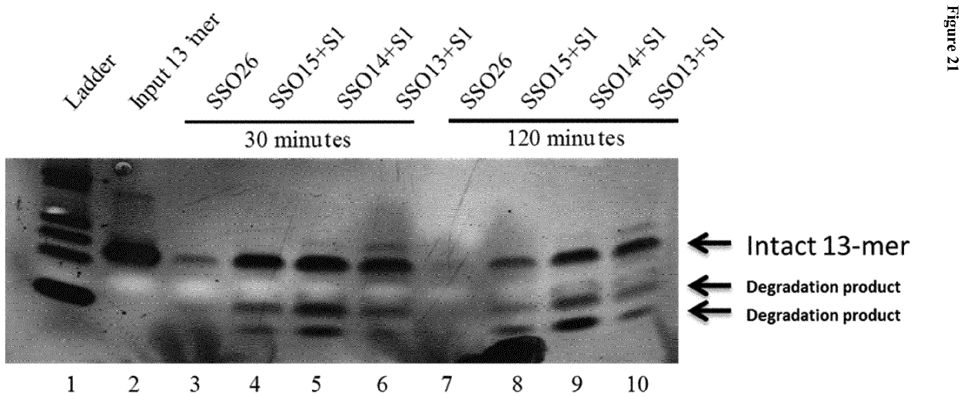

The stability of the PS2 containing oligonucleotides was tested with 30 and 120 minutes incubation of the S1 nuclease. The position of the PS2 linkage is influencing the stability, and the presence of a PS2 3'to a DNA nucleotide (SSO#14) has the greatest impact (figure 21). After 30 minutes of incubation with S1 nuclease, the parent oligonucleotide is almost degraded, whereas the PS2 modified oligos shows a strong band representing the 13'mer. In addition, SSO#14 shows stronger bands representing degradation products indicating a stabilization of the remaining oligo, even after the initial cleavage by S1 nuclease (figure 21, lane 5+9).

These data illustrate that the presence of a phosphorodithioates when introduced into oligonucelotides, such as mixmer oligonucleotides, provides protection against endonucleoase activity - and surprisingly this is achieved whilst maintaining efficacy of the oligonucelotides, indeed as shown in the present experiments, the splice modulating activity may be notably improved. It is considered that PS2 linkages adjacent to DNA nucleosides, or between DNA nucleosides, in a mixmer oligonucelotides, herein illustrated by mixmers comprising LNA and DNA nucleosides enhances endonuclease stability. For use in antisense oligonucleotides, such as mixmers (SSOs or antimiRs for example), it is therefore considered that using PS2 linkages between contiguous DNA nucleosides is beneficial. Such benefits can also be provided by using a 5' or 3' PS2 linkage adjacent to a DNA nucleoside which is flanked 5' or 3' (respectively) by a 2'sugar modified nucleoside, such as LNA or MOE.

The invention therefore further provides improved antisense oligonucelotides for use in occupation based mechanisms, such as in splice modulating or for microRNA inhibition.

## Claims

1. An oligonucleotide comprising at least one phosphorodithioate internucleoside linkage of formula (IA) or (IB) 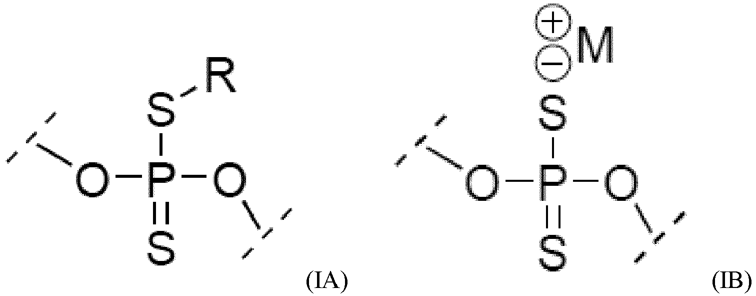
wherein one of the two oxygen atoms is linked to the 3'carbon atom of an adjacent nucleoside (A¹) and the other one is linked to the 5'carbon atom of another adjacent nucleoside (A²), wherein at least one of the two nucleosides (A¹) and (A²) is a LNA nucleoside and wherein in (IA) R is hydrogen or a phosphate protecting group, and in (IB) M+ is a cation, such as a metal cation, such as an alkali metal cation, such as a Na+ or K+ cation; or M+ is an ammonium cation;
wherein the oligonucleotide comprises between 1 and 5 internucleoside linkages of formula (IA) or (IB) and wherein the further internucleoside linkages are all phosphorothioate internucleoside linkages.

2. An oligonucleotide according to claim 1, comprising 1 to 4 internucleoside linkages of formula (IA) or (IB).

3. An oligonucleotide according to claim 1 or 2, wherein one of (A¹) and (A²) is a LNA nucleoside and the other one is a DNA nucleoside, a RNA nucleoside or a sugar modified nucleoside.

4. An oligonucleotide according to any one of claims 1 to 3, wherein one of (A¹) and (A²) is a LNA nucleoside and the other one is a DNA nucleoside or a sugar modified nucleoside.

5. An oligonucleotide according to any one of claims 1 to 4, wherein one of (A¹) and (A²) is a LNA nucleoside and the other one is a DNA nucleoside.

6. An oligonucleotide according to any one of claims 1 to 4, wherein one of (A¹) and (A²) is a LNA nucleoside and the other one is a sugar modified nucleoside.

7. An oligonucleotide according to any one of claims 3 to 6, wherein said sugar modified nucleoside is a 2'-sugar modified nucleoside.

8. An oligonucleotide according to claim 7, wherein said 2'-sugar modified nucleoside is 2'-alkoxy-RNA, 2'-alkoxyalkoxy-RNA, 2'-amino-DNA, 2'-fluoro-RNA, 2'-fluoro-ANA or a LNA nucleoside.

9. An oligonucleotide according to claim 7 or 8, wherein said 2'-sugar modified nucleoside is a LNA nucleoside.

10. An oligonucleotide according to any one of claims 1 to 9, wherein the LNA nucleosides are independently selected from beta-D-oxy LNA, 6'-methyl-beta- D-oxy LNA and ENA.

11. An oligonucleotide according to claim 9 or 10, wherein the LNA nucleosides are both beta-D-oxy LNA.

12. An oligonucleotide according to claim 7 or 8, wherein said 2'-sugar modified nucleoside is 2'-alkoxyalkoxy-RNA.

13. An oligonucleotide according to claim 10, wherein 2'-alkoxy-RNA is 2'-methoxy-RNA.

14. An oligonucleotide according to any one of claims 1 to 12, wherein 2'-alkoxyalkoxy-RNA is 2'-methoxyethoxy-RNA.

15. An oligonucleotide according to any one of claims 1 to 14, wherein the oligonucleotide is of 7 to 30 nucleotides in length.

16. An oligonucleotide according to any one of claims 1 to 15, wherein one or more nucleoside is a nucleobase modified nucleoside.

17. An oligonucleotide according to any one of claims 1 to 16, wherein the oligonucleotide is an antisense oligonucleotide, a siRNA, a microRNA mimic or a ribozyme.

18. A pharmaceutically acceptable salt of an oligonucleotide according to any one of claims 1 to 17, in particular a sodium or a potassium salt or ammonium salt.

19. A conjugate comprising an oligonucleotide or a pharmaceutically acceptable salt according to any one of claims 1 to 18 and at least one conjugate moiety covalently attached to said oligonucleotide or said pharmaceutically acceptable salt, optionally *via* a linker moiety.

20. A pharmaceutical composition comprising an oligonucleotide, pharmaceutically acceptable salt or conjugate according to any one of claims 1 to 19 and a therapeutically inert carrier.

21. An oligonucleotide, pharmaceutically acceptable salt or conjugate according to any one of claims 1 to 19 for use as a therapeutically active substance.

## Patentansprüche

1. Oligonukleotid, umfassend mindestens eine Phosphordithioat-Intemukleosid-Verknüpfung der Formel (IA) oder (IB) 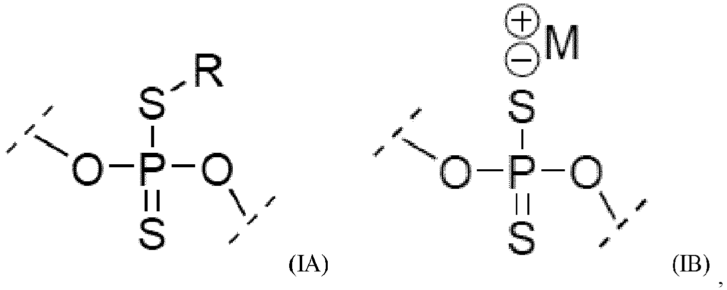
wobei eines von den zwei Sauerstoffatomen mit dem 3'-Kohlenstoffatom eines nachbarständigen Nukleosids (A¹) verknüpft ist und das andere mit dem 5'-Kohlenstoffatom eines anderen nachbarständigen Nukleosids (A²) verknüpft ist, wobei mindestens eines von den zwei Nukleosiden (A¹) und (A²) ein LNA-Nukleosid ist und wobei in (IA) R Wasserstoff oder eine Phosphatschutzgruppe ist und in (IB) M+ ein Kation, wie ein Metallkation, wie ein Alkalimetallkation, wie ein Na+- oder K+-Kation ist; oder M+ ein Ammoniumkation ist;
wobei das Oligonukleotid zwischen 1 und 5 Internukleosid-Verknüpfungen der Formel (IA) oder (IB) umfasst und wobei die weiteren Internukleosid-Verknüpfungen alle Phosphorthioat-Internukleosid-Verknüpfungen sind.

2. Oligonukleotid nach Anspruch 1, umfassend 1 bis 4 Internukleosid-Verknüpfungen der Formel (IA) oder (IB).

3. Oligonukleotid nach Anspruch 1 oder 2, wobei eines von (A¹) und (A²) ein LNA-Nukleosid ist und das andere ein DNA-Nukleosid, ein RNA-Nukleosid oder ein zuckermodifiziertes Nukleosid ist.

4. Oligonukleotid nach einem der Ansprüche 1 bis 3, wobei eines von (A¹) und (A²) ein LNA-Nukleosid ist und das andere ein DNA-Nukleosid oder ein zuckermodifiziertes Nukleosid ist.

5. Oligonukleotid nach einem der Ansprüche 1 bis 4, wobei eines von (A¹) und (A²) ein LNA-Nukleosid ist und das andere ein DNA-Nukleosid ist.

6. Oligonukleotid nach einem der Ansprüche 1 bis 4, wobei eines von (A¹) und (A²) ein LNA-Nukleosid ist und das andere ein zuckermodifiziertes Nukleosid ist.

7. Oligonukleotid nach einem der Ansprüche 3 bis 6, wobei das zuckermodifizierte Nukleosid ein 2'-zuckermodifiziertes Nukleosid ist.

8. Oligonukleotid nach Anspruch 7, wobei das 2'-zuckermodifizierte Nukleosid 2'-Alkoxy-RNA, 2'-Alkoxyalkoxy-RNA, 2'-Amino-DNA, 2`-Fluor-RNA, 2'-Fluor-ANA oder ein LNA-Nukleosid ist.

9. Oligonukleotid nach Anspruch 7 oder 8, wobei das 2`-zuckermodifizierte Nukleosid ein LNA-Nukleosid ist.

10. Oligonukleotid nach einem der Ansprüche 1 bis 9, wobei die LNA-Nukleoside unabhängig voneinander aus beta-D-Oxy-LNA, 6'-Methyl-beta-D-oxy-LNA und ENA ausgewählt sind.

11. Oligonukleotid nach Anspruch 9 oder 10, wobei die LNA-Nukleoside beide beta-D-Oxy-LNA sind.

12. Oligonukleotid nach Anspruch 7 oder 8, wobei das 2'-zuckermodifizierte Nukleosid 2'-Alkoxyalkoxy-RNA ist.

13. Oligonukleotid nach Anspruch 10, wobei 2'-Alkoxy-RNA 2'-Methoxy-RNA ist.

14. Oligonukleotid nach einem der Ansprüche 1 bis 12, wobei 2'-Alkoxyalkoxy-RNA 2'-Methoxyethoxy-RNA ist.

15. Oligonukleotid nach einem der Ansprüche 1 bis 14, wobei das Oligonukleotid eine Länge von 7 bis 30 Nukleotiden hat.

16. Oligonukleotid nach einem der Ansprüche 1 bis 15, wobei ein oder mehrere Nukleosid(e) ein nukleobasenmodifiziertes Nukleosid ist/sind.

17. Oligonukleotid nach einem der Ansprüche 1 bis 16, wobei das Oligonukleotid ein Antisense-Oligonukleotid, eine siRNA, ein microRNA-Nachahmer oder ein Ribozym ist.

18. Pharmazeutisch unbedenkliches Salz eines Oligonukleotids nach einem der Ansprüche 1 bis 17, insbesondere ein Natrium- oder ein Kaliumsalz oder Ammoniumsalz.

19. Konjugat, umfassend ein Oligonukleotid oder ein pharmazeutisch unbedenkliches Salz nach einem der Ansprüche 1 bis 18 und mindestens eine Konjugateinheit, die gegebenenfalls über eine Linker-Einheit kovalent an das Oligonukleotid oder das pharmazeutisch unbedenkliche Salz gebunden ist.

20. Pharmazeutische Zusammensetzung, umfassend ein Oligonukleotid, pharmazeutisch unbedenkliches Salz oder Konjugat nach einem der Ansprüche 1 bis 19 und einen therapeutisch inerten Träger.

21. Oligonukleotid, pharmazeutisch unbedenkliches Salz oder Konjugat nach einem der Ansprüche 1 bis 19 zur Verwendung als eine therapeutisch wirksame Substanz.

## Revendications

1. Oligonucléotide comprenant au moins une liaison internucléosidique phosphorodithioate de formule (IA) ou (IB) 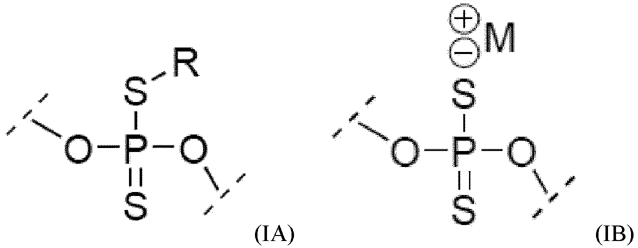
dans lequel l'un des deux atomes d'oxygène est lié à l'atome de carbone en 3' d'un nucléoside (A¹) adjacent et l'autre est lié à l'atome de carbone en 5' d'un autre nucléoside (A²) adjacent, dans lequel au moins l'un des deux nucléosides (A¹) et (A²) est un nucléoside de LNA et dans lequel, dans (IA), R représente un hydrogène ou un groupe protecteur de phosphate, et dans (IB), M+ représente un cation, tel qu'un cation métallique, tel qu'un cation de métal alcalin, tel qu'un cation Na+ ou K+ ; ou M+ représente un cation ammonium ;
dans lequel l'oligonucléotide comprend entre 1 et 5 liaisons intemucléosidiques de formule (IA) ou (IB) et dans lequel les autres liaisons intemucléosidiques sont toutes des liaisons intemucléosidiques phosphorothioate.

2. Oligonucléotide selon la revendication 1, comprenant de 1 à 4 liaisons intemucléosidiques de formule (IA) ou (IB).

3. Oligonucléotide selon la revendication 1 ou 2, dans lequel l'un de (A¹) et (A²) est un nucléoside de LNA et l'autre est un nucléoside d'ADN, un nucléoside d'ARN ou un nucléoside modifié par un sucre.

4. Oligonucléotide selon l'une quelconque des revendications 1 à 3, dans lequel l'un de (A¹) et (A²) est un nucléoside de LNA et l'autre est un nucléoside d'ADN ou un nucléoside modifié par un sucre.

5. Oligonucléotide selon l'une quelconque des revendications 1 à 4, dans lequel l'un de (A¹) et (A²) est un nucléoside de LNA et l'autre est un nucléoside d'ADN.

6. Oligonucléotide selon l'une quelconque des revendications 1 à 4, dans lequel l'un de (A¹) et (A²) est un nucléoside de LNA et l'autre est un nucléoside modifié par un sucre.

7. Oligonucléotide selon l'une quelconque des revendications 3 à 6, dans lequel ledit nucléoside modifié par un sucre est un nucléoside modifié par un sucre en 2'.

8. Oligonucléotide selon la revendication 7, dans lequel ledit nucléoside modifié par un sucre en 2' est un 2'-alcoxy-ARN, un 2'-alcoxyalcoxy-ARN, un 2'-amino-ADN, un 2'-fluoro-ARN, un 2'-fluoro-ANA ou un nucléoside de LNA.

9. Oligonucléotide selon la revendication 7 ou 8, dans lequel ledit nucléoside modifié par un sucre en 2' est un nucléoside de LNA.

10. Oligonucléotide selon l'une quelconque des revendications 1 à 9, dans lequel les nucléosides de LNA sont indépendamment choisis parmi un bêta-D-oxy LNA, un 6'-méthyl-bêta-D-oxy LNA et un ENA.

11. Oligonucléotide selon la revendication 9 ou 10, dans lequel les nucléosides de LNA sont tous deux des bêta-D-oxy LNA.

12. Oligonucléotide selon la revendication 7 ou 8, dans lequel ledit nucléoside modifié par un sucre en 2' est un 2'-alcoxyalcoxy-ARN.

13. Oligonucléotide selon la revendication 10, dans lequel le 2'-alcoxy-ARN est un 2'-méthoxy-ARN.

14. Oligonucléotide selon l'une quelconque des revendications 1 à 12, dans lequel le 2'-alcoxyalcoxy-ARN est un 2'-méthoxyéthoxy-ARN.

15. Oligonucléotide selon l'une quelconque des revendications 1 à 14, dans lequel l'oligonucléotide est de 7 à 30 nucléotides de longueur.

16. Oligonucléotide selon l'une quelconque des revendications 1 à 15, dans lequel un ou plusieurs nucléoside est un nucléoside modifié par une nucléobase.

17. Oligonucléotide selon l'une quelconque des revendications 1 à 16, dans lequel l'oligonucléotide est un oligonucléotide antisens, un pARNi, un mimétique de microARN ou un ribozyme.

18. Sel pharmaceutiquement acceptable d'un oligonucléotide selon l'une quelconque des revendications 1 à 17, en particulier un sel de sodium ou de potassium ou un sel d'ammonium.

19. Conjugué comprenant un oligonucléotide ou un sel pharmaceutiquement acceptable selon l'une quelconque des revendications 1 à 18 et au moins une fraction conjuguée liée de manière covalente audit oligonucléotide ou audit sel pharmaceutiquement acceptable, éventuellement par l'intermédiaire d'une fraction de liaison.

20. Composition pharmaceutique comprenant un oligonucléotide, un sel pharmaceutiquement acceptable ou un conjugué selon l'une quelconque des revendications 1 à 19 et un véhicule thérapeutiquement inerte.

21. Oligonucléotide, sel pharmaceutiquement acceptable ou conjugué selon l'une quelconque des revendications 1 à 19 pour une utilisation comme substance thérapeutiquement active.
